# EUROPEAN PATENT SPECIFICATION

(11) **EP 3 168 847 B1**
(45) Date of publication and mention of the grant of the patent: **16.05.2018**
(21) Application number: 15818813.6
(22) Date of filing: 01.07.2015
(51) Int. Cl.: H01G 9/20, C07D 409/14, C07D 495/04, C07D 495/14, C07D 519/00, C09B 57/10, C09B 67/44

(54) **PHOTOELECTRIC CONVERSION ELEMENT, DYE-SENSITIZED SOLAR CELL, METAL COMPLEX DYE, DYE SOLUTION, AND TERPYRIDINE COMPOUND OR ESTERIFICATION PRODUCT THEREOF**
PHOTOELEKTRISCHES UMWANDLUNGSELEMENT, FARBSTOFFSENSIBILISIERTE SOLARZELLE, METALLKOMPLEXFARBSTOFF, FARBSTOFFLÖSUNG UND TERPYRIDIN-VERBINDUNG ODER VERESTERUNGSPRODUKT DAVON
ÉLÉMENT DE CONVERSION PHOTOÉLECTRIQUE, CELLULE SOLAIRE À PIGMENT PHOTOSENSIBLE, COLORANT À COMPLEXE MÉTALLIQUE, SOLUTION DE COLORANT, ET COMPOSÉ DE TERPYRIDINE OU SON PRODUIT D'ESTÉRIFICATION

(30) Priority: 07.07.2014 JP 2014140079; 27.02.2015 JP 2015039390
(43) Date of publication of application: 17.05.2017
(73) Proprietor: Fujifilm Corporation, Minato-ku Tokyo 106-8620 (JP)
(72) Inventor: TSUNA, Kazuhiro, Kanagawa 258-8577 (JP); WATANABE, Kousuke, Kanagawa 258-8577 (JP); KOBAYASHI, Katsumi, Kanagawa 258-8577 (JP)
(74) Representative: Hoffmann Eitle
(86) International application number: PCT/JP2015/068977
(87) International publication number: WO 2016/006512

(56) References cited:
- WO-A1-2012/017872
- JP-A- 2012 036 237
- JP-A- 2012 146 632

## Description

### CROSS-REFERENCE TO RELATED APPLICATIONS

This application is a Continuation of PCT International Application No. PCT/JP2015/068977 filed on July 1, 2015, which claims priorities under 35 U.S.C. § 119 (a) to Japanese Patent Application No.JP2014-140079 filed on July 7, 2014, and JP2015-039390 filed on February 27, 2015.

### BACKGROUND OF THE INVENTION

### 1. Field of the Invention

The present invention relates to a photoelectric conversion element, a dye-sensitized solar cell, a metal complex dye, a dye solution, and a terpyridine compound or an esterified product thereof.

### 2. Description of the Related Art

Photoelectric conversion elements are used in various photosensors, copying machines, photoelectrochemical cells such as solar cells, and the like. These photoelectric conversion elements have adopted various systems to be put into use, such as systems utilizing metals, systems utilizing semiconductors, systems utilizing organic pigments or dyes, or combinations of these elements. In particular, solar cells utilizing inexhaustible solar energy do not necessitate fuels, and full-fledged practicalization of solar cells as an inexhaustible clean energy is being highly expected. Above all, research and development of silicon-based solar cells has long been in progress, and many countries also support policy-wise considerations, and thus dissemination of silicon-based solar cells is still in progress. However, silicon is an inorganic material, and thus, naturally has limitations in terms of improvement of throughput, cost, and the like.

Therefore, research is being vigorously carried out on photoelectrochemical cells (also referred to as dye-sensitized solar cells), using metal complex dyes. In particular, the research was fueled by the results of research conducted by Graetzel et al. of École Polytechnique Fédérale de Lausanne in Switzerland. They adopted a structure in which a dye formed from a ruthenium complex was fixed on the surface of a porous titanium oxide film, and realized photoelectric conversion efficiency that is equivalent to that of amorphous silicon. Thus, dye-sensitized solar cells that can be produced even without use of expensive vacuum devices have instantly drawn the attention of researchers all over the world.

Hitherto, dyes called N3, N719, N749 (also referred to as Black Dye), Z907, and J2, and the like have generally been developed as metal complex dyes for use in dye-sensitized solar cells. However, all of the photoelectric conversion elements and dye-sensitized solar cells using these dyes are not sufficient in terms of photoelectric conversion efficiency and durability (heat stability).

Therefore, development of metal complex dyes capable of improving the photoelectric conversion efficiency or the durability of photoelectric conversion elements and dye-sensitized solar cells is in progress.

For example, JP2013-67773A describes a metal complex dye having a terpyridine ligand in which a plurality of thiophene ring groups are linked and introduced into the 3-position with respect to the ring-constituting nitrogen atom coordinating to a metal ion of a terminal pyridine ring, and three monodentate ligands. It also describes that a photoelectrochemical cell using the metal complex dye accomplishes high photoelectric conversion efficiency and has excellent durability.

JP2013-229285A describes a metal complex dye having a terpyridine ligand in which one thiophene ring group is bonded at the 3-position with respect to the ring-constituting nitrogen atom coordinating to a metal ion of a terminal pyridine ring, and a doner ligand having a cyclic group substituted with a specific substituent. It also describes that a photoelectrochemical cell using the metal complex dye accomplishes both of reduction in performance irregularity and improvement of photoelectric conversion efficiency and durability.

JP2012-36237A describes a metal complex dye having a terpyridine ligand in which a plurality of thiophene ring group are introduced to at least one of a terminal pyridine ring and a centered pyridine ring by linkage, and three monodentate ligands or bipyridine ligands. It also describes that a photoelectrochemical cell using the metal complex dye has high photoelectric conversion efficiency and excellent durability.

US2012/0247561A describes a tridentate ligand in which one thiophene ring group is bonded at the 2- or 3-position with respect to the ring-constituting nitrogen atom coordinating to a metal ion of a terminal pyridine ring, and a metal complex having such a ligand and three thioisocyanate anions.

### SUMMARY OF THE INVENTION

However, in recent years, studies and development of photoelectric conversion elements and dye-sensitized solar cells have been actively conducted, and thus, increasingly higher performance thereof is required. There is a demand for, in particular, further enhancement and improvements in their photoelectric conversion efficiency and durability.

In photoelectric conversion elements and dye-sensitized solar cells, a layer (also referred to as a semiconductor layer) which is formed of semiconductor fine particles and carries a metal complex dye is usually formed into a layer with a thickness of 10 to several hundred µm. For such photoelectric conversion elements and dye-sensitized solar cells, reduction in thickness (size) and weight has been required. However, the photoelectric conversion efficiency varies depending on the film thickness of a semiconductor layer, and tends to decrease as the film thickness becomes smaller. Accordingly, excellent photoelectric conversion efficiency is required to be exhibited even in a case where the film thickness of the semiconductor layer is small.

The present invention has an object to provide a photoelectric conversion element and a dye-sensitized solar cell, each of which is less affected by the film thickness of a semiconductor layer, exhibits excellent photoelectric conversion efficiency, particularly even when the film thickness is small, and has high durability; and a metal complex dye, a dye solution, and a terpyridine compound or an esterified product thereof, each of which is used in the photoelectric conversion element and the dye-sensitized solar cell.

The present inventors have discovered that when a combination of a tridentate ligand with a terminal portion having a nitrogen-containing aromatic ring in which a plurality of thiophene ring groups are introduced to a ring-constituting atom at the 4-position with respect to a coordinating atom by linkage, with a bidentate or tridentate ligand which coordinates to a metal ion through at least one nitrogen atom and an anion of at least one atom is used in a metal complex dye for use in a photoelectric conversion element and a dye-sensitized solar cell, it is possible to realize further improvement of photoelectric conversion efficiency and durability as well as high photoelectric conversion efficiency even when the semiconductor layer is a thin film. Based on these findings, the present invention has been completed.

That is, the objects of the present invention have been achieved by the following means.
<1> A photoelectric conversion element comprising:
   an electrically conductive support;
   a photoconductor layer including an electrolyte;
   a charge transfer layer including an electrolyte; and
   a counter electrode,
   in which the photoconductor layer has semiconductor fine particles carrying a metal complex dye represented by the following Formula (I),

   Formula (I) M(LA)(LD)(LX)_{mX}·(CI)_{mY}

   in the formula,
   M represents a metal ion,
   LA represents a tridentate ligand represented by the following Formula (AL-1),
   LD represents a bidentate ligand, or a tridentate ligand different from LA, and at least one of coordinating atoms in LD bonded to the metal ion M is a nitrogen atom, and at least one of the coordinating atoms is an anion,
   LX represents a monodentate ligand, and when LD is a bidentate ligand, mX represents 1, and when LD is a tridentate ligand, mX represents 0,
   CI represents a counterion necessary for neutralizing the charge of the metal complex dye, and
   mY represents an integer of 0 to 3; 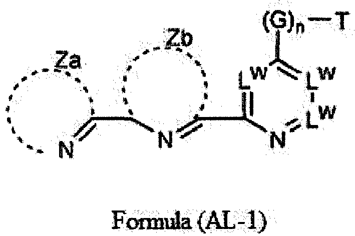 in the formula,
      Za and Zb each independently represent a non-metal atomic group necessary for forming a 5- or 6-membered ring, in which at least one of rings formed by Za and Zb, respectively, has one or more acidic groups,
      L^{W}'s each independently represent a nitrogen atom or CR^{W}, and R^{W} represents a hydrogen atom or a substituent, and
      G represents a ring group represented by any one of the following Formulae (X-1) to (X-3),
      n represents an integer of 2 to 7,
      T represents a hydrogen atom or a substituent, and
      a -(G)n-T group does not have an acidic group and an amino group; and 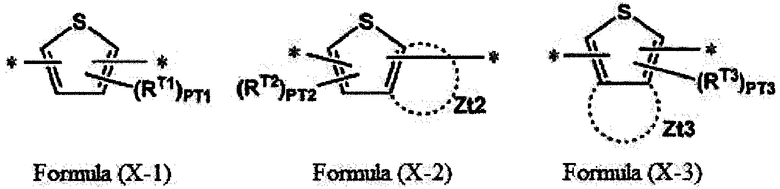 in the formulae,
         Zt2 and Zt3 each represent a non-metal atomic group necessary for forming a fused ring with a thiophene ring in Formula (X-2) or (X-3),
         R^{T1}, R^{T2}, and R^{T3} each independently represent a substituent,
         PT1 represents an integer of 0 to 2, and PT2 and PT3 each independently represent an integer of 0 or more, and is no more than the number of hydrogen atoms when the group represented by Formula (X-2) or (X-3) is unsubstituted, and
         * represents a binding position to a ring including L^{W}, or another G or T.
<2> The photoelectric conversion element as described in <1>, in which the ring group represented by Formula (X-1) is a ring group represented by any one of the following Formulae (X-1a) to (X-1c), 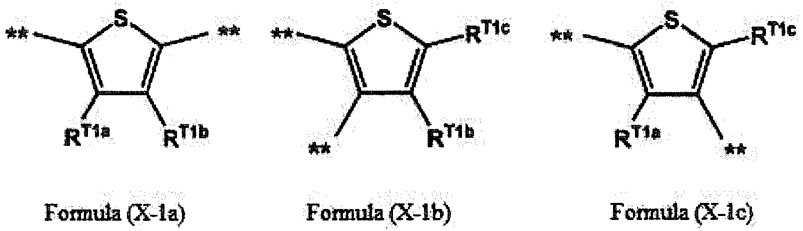 in the formulae, R^{T1a} to R^{T1c} each independently represent a hydrogen atom or a substituent, and ** represents a binding position to a ring including L^{W}, or another G or T.
<3> The photoelectric conversion element as described in <1>, in which the ring group represented by Formula (X-2) is a ring group represented by any one of the following Formulae (X-2a) to (X-2e), 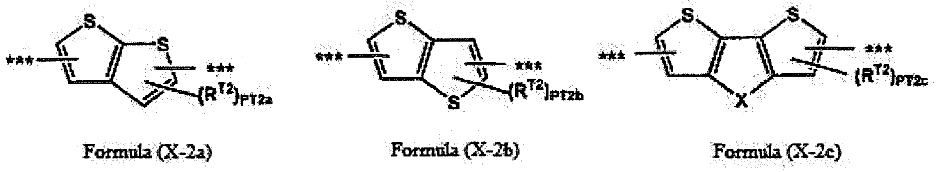 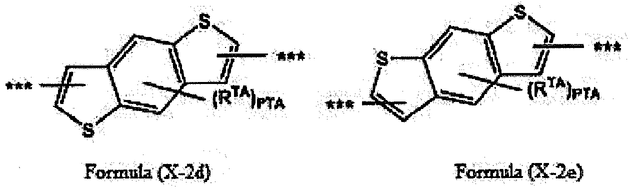 in the formulae,
   X represents -O-, -S-, -NR^{X2c}-, -C(R^{X2c})₂-, -(R^{X2c})C=C(R^{X2x})-, or -Si(R^{X2c})₂-,
   R^{X2c} represents a hydrogen atom or a substituent,
   R^{T2} and R^{TA} each independently represent a substituent,
   PT2a to PT2c each independently represent an integer of 0 to 2,
   PTA's each represent an integer of 0 to 4, and
   *** represents a binding position to a ring including L^{W}, or another G or T.
<4> The photoelectric conversion element as described in <1>, in which the ring group represented by Formula (X-3) is a ring group represented by the following Formula (X-3a) or (X-3b), 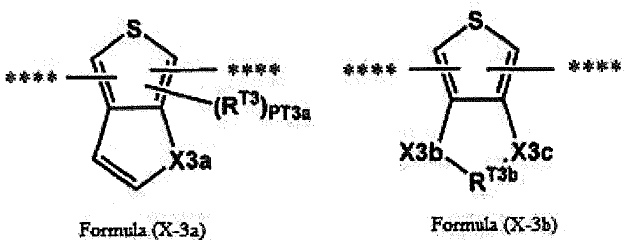 in the formula,
   X3a to X3c each independently represent -O- or -S-,
   R^{T3b} represents an alkylene group,
   R^{T3} represents a substituent,
   PT3a represents an integer of 0 to 2, and
   **** represents a binding position to a ring including L^{W}, or another G or T.
<5> The photoelectric conversion element as described in any one of <1> to <4>, in which the ring formed by Za is at least one selected from the group consisting of a pyridine ring, a pyrimidine ring, a pyrazine ring, a pyridazine ring, a triazine ring, a tetrazine ring, a quinoline ring, an isoquinoline ring, an imidazole ring, a pyrazole ring, a triazole ring, a thiazole ring, an oxazole ring, a benzimidazole ring, a benzotriazole ring, a benzoxazole ring, and a benzothiazole ring,
   the ring formed by Zb is at least one selected from the group consisting of a pyridine ring, a pyrimidine ring, a pyrazine ring, a pyridazine ring, a triazine ring, a tetrazine ring, a quinoline ring, an isoquinoline ring, an imidazole ring, a triazole ring, a thiazole ring, an oxazole ring, a benzimidazole ring, a benzotriazole ring, a benzoxazole ring, and a benzothiazole ring, and
   the hetero ring including L^{W} is at least one selected from the group consisting of a pyridine ring, a pyrimidine ring, a pyridazine ring, a triazine ring, a tetrazine ring, a quinoline ring, and an isoquinoline ring.
<6> The photoelectric conversion element as described in any one of <1> to <5>, in which M is Ru²⁺ or Os²⁺.
<7> The photoelectric conversion element as described in any one of <1> to <6>, in which LA is a tridentate ligand represented by the following Formula (AL-2), 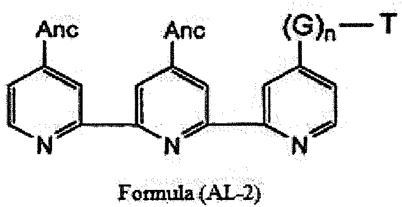 in the formula,
   Anc represents an acidic group, and
   G, T, and n have the same definitions as G, T, and n, respectively, in Formula (AL-1).
<8> The photoelectric conversion element as described in any one of <1> to <7>, in which the acidic group is a carboxyl group or a salt thereof.
<9> The photoelectric conversion element as described in any one of <1> to <8>, in which LD is a bidentate ligand represented by any one of the following Formulae (2L-1) to (2L-4), 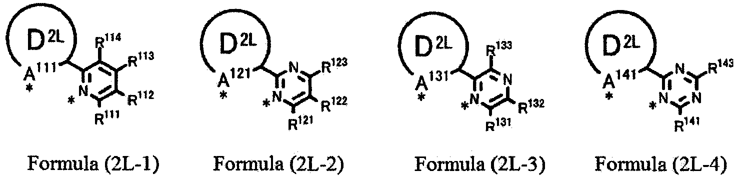 in the formulae,
   the ring D^{2L} represents an aromatic ring,
   A¹¹¹ to A¹⁴¹ each independently represent an anion of a nitrogen atom or an anion of a carbon atom,
   R¹¹¹ to R¹⁴³ each independently represent a hydrogen atom or a substituent not having an acidic group, and
   * represents a coordinating position to the metal ion M.
<10> The photoelectric conversion element as described in any one of <1> to <8>, in which LD is a tridentate ligand represented by any one of the following Formulae (3L-1) to (3L-4), 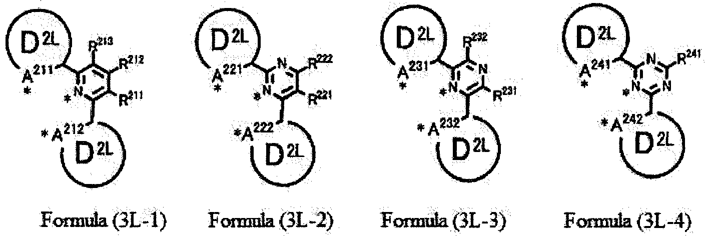 in the formulae,
   the ring D^{2L} represents an aromatic ring,
   A²¹¹ to A²⁴² each independently represent a nitrogen atom or a carbon atom, in which at least one of each of A²¹¹ and A²¹², A²²¹ and A²²², A²³¹ and A²³², and A²⁴¹ and A²⁴² is an anion,
   R²¹¹ to R²⁴¹ each independently represent a hydrogen atom or a substituent not having an acidic group, and
   * represents a coordinating position to the metal ion M.
<11> The photoelectric conversion element as described in any one of <1> to <10>, in which the metal complex dye represented by Formula (I) is represented by the following Formula (1-1) or (1-2), 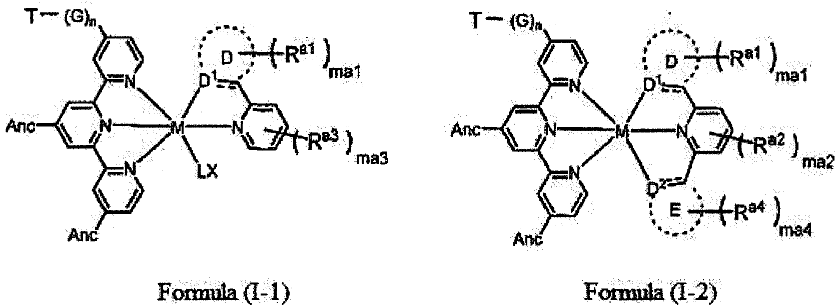 in the formulae,
   M and LX have the same definitions as M and LX, respectively, in Formula (I),
   G, T, and n have the same definitions as G, T, and n, respectively, in Formula (AL-1),
   Anc represents an acidic group,
   the ring D and the ring E each independently represent a 5- or 6-membered aromatic ring,
   D¹ and D² each independently represent an anion of a carbon atom or an anion of a nitrogen atom, in which a bond between D¹ and D² in the ring D and the ring E, and a carbon atom bonded to a pyridine ring is a single bond or a double bond,
   R^{a1} to R^{a4} each independently represent a substituent, and
   ma1, ma2, and ma4 each independently represent an integer of 0 to 3, ma3 represents an integer of 0 to 4, and when ma1 to ma4 each represent an integer of 2 or more, a plurality of R^{a1}'s to R^{a4}'s may each be bonded to each other to form a ring.
<12> The photoelectric conversion element as described in <11>, in which the ring D and the ring E are each independently a pyrazole ring, a triazole ring, or a benzene ring.
<13> A dye-sensitized solar cell comprising the photoelectric conversion element as described in any one of <1> to <12>.
<14> A metal complex dye represented by the following Formula (I),

   Formula (I) M(LA)(LD)(LX)_{mX}·(CI)_{mY}

   in the formula,
   M represents a metal ion,
   LA represents a tridentate ligand represented by the following Formula (AL-1),
   LD represents a bidentate ligand, or a tridentate ligand different from LA, and at least one of coordinating atoms in LD bonded to the metal ion M is a nitrogen atom, and at least one of the coordinating atoms is an anion,
   LX represents a monodentate ligand, and when LD is a bidentate ligand, mX represents 1, and when LD is a tridentate ligand, mX represents 0,
   CI represents a counterion necessary for neutralizing the charge of the metal complex dye, and
   mY represents an integer of 0 to 3; 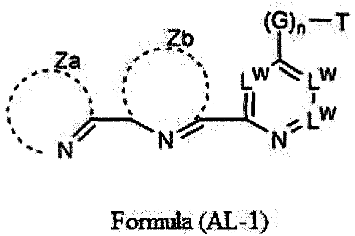 in the formula,
      Za and Zb each independently represent a non-metal atomic group necessary for forming a 5- or 6-membered ring, in which at least one of rings formed by Za and Zb, respectively, has one or more acidic groups,
      Lw's each independently represent a nitrogen atom or CR^{W}, and R^{W} represents a hydrogen atom or a substituent,
      G represents a ring group represented by any one of the following Formulae (X-1) to (X-3),
      n represents an integer of 2 to 7,
      T represents a hydrogen atom or a substituent, and
      a -(G)n-T group does not have an acidic group and an amino group; and 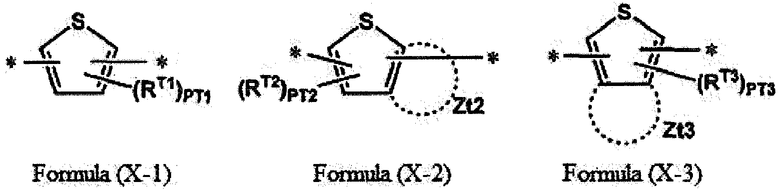 in the formulae,
         Zt2 and Zt3 each represent a non-metal atomic group necessary for forming a fused ring with a thiophene ring in Formula (X-2) or (X-3),
         R^{T1}, R^{T2}, and R^{T3} each independently represent a substituent,
         PT1 represents an integer of 0 to 2,
         PT2 and PT3 each independently represent an integer of 0 or more, and is no more than the number of hydrogen atoms when the group represented by Formula (X-2) or (X-3) is unsubstituted, and
         * represents a binding position to a ring including L^{W}, or another G or T.
<15> A dye solution comprising the metal complex dye as described in <14> and a solvent.
<16> A terpyridine compound or an esterified product thereof represented by the following Formula (AL-2), 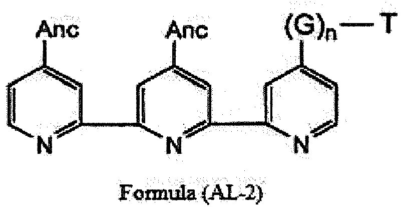 in the formula,
   Anc represents an acidic group,
   G represents a ring group represented by any one of the following Formulae (X-1) to (X-3),
   n represents 2 or 3,
   T represents a hydrogen atom or a substituent, and
   a -(G)n-T group does not have an acidic group and an amino group; and 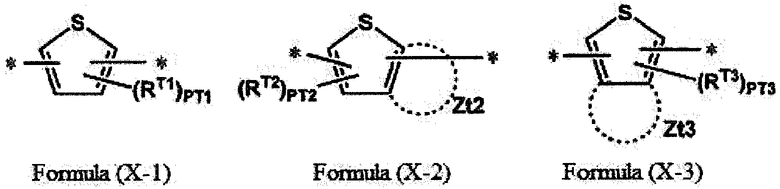 in the formulae,
      Zt2 and Zt3 each represent a non-metal atomic group necessary for forming a fused ring with a thiophene ring in Formula (X-2) or (X-3),
      R^{T1}, R^{T2}, and R^{T3} each independently represent a substituent,
      PT1 represents an integer of 0 to 2,
      PT2 and PT3 each independently represent an integer of 0 or more, and is no more than the number of hydrogen atoms when the group represented by Formula (X-2) or (X-3) is unsubstituted, and
      * represents a binding position to a ring including L^{W}, or another G or T.

In the present specification, unless otherwise specified, in a case where the E configuration or the Z configuration exists in the molecule for a double bond, the double bond may be either one of the two configurations or a mixture thereof.

When there are a plurality of substituents, linking groups, ligands, or the like (hereinafter referred to as substituents or the like) represented by specific symbols, or when a plurality of substituents and the like are defined at the same time, the respective substituents or the like may be the same as or different from each another unless otherwise specified. This also applies to the definition of the number of substituents or the like. Further, when a plurality of substituents or the like are close to each other (in particular, adjacent to each other), they may be linked to each other to form a ring, unless otherwise specified.

Furthermore, the ring, for example, an aromatic ring or an aliphatic ring may further be fused to form a fused ring. The aromatic ring or the aliphatic ring is preferably a 4- to 8-membered rings and more preferably a 5- or 6-membered rings.

In the present invention, an aromatic ring encompasses an aromatic hydrocarbon ring and an aromatic hetero ring. The aromatic hydrocarbon ring refers to a hydrocarbon ring exhibiting aromaticity. Although not being particularly limited, examples of the aromatic hydrocarbon ring include a benzene ring, a naphthalene ring, and a fluorene ring. The aliphatic ring refers to a ring other than the aromatic ring, and encompasses an aliphatic hydrocarbon ring and an aliphatic hetero ring. Examples of the aliphatic hydrocarbon ring include a saturated hydrocarbon ring, and an unsaturated hydrocarbon ring not exhibiting aromaticity, for example, a saturated monocyclic hydrocarbon ring (cycloalkane), a saturated polycyclic hydrocarbon ring, an unsaturated monocyclic hydrocarbon ring (cycloalkene and cycloalkyne), and an unsaturated polycyclic hydrocarbon ring.

The aromatic hetero ring and the aliphatic hetero ring may be collectively referred to as a hetero ring in some cases. The hetero ring refers to a ring having carbon atoms and a heteroatom (for example, a nitrogen atom, an oxygen atom, a sulfur atom, a silicon atom, a selenium atom, and a phosphorus atom) as ring-constituting atoms. The aromatic hetero ring refers to an aromatic ring including a heteroatom, and the aliphatic hetero ring refers to an aliphatic ring including a heteroatom.

In the present specification, expressions of a compound (including a complex and a dye) are used to mean inclusion of, in addition to the compound itself, salts and ions of the compound. Further, within a range exhibiting desired effects, the expressions are used to mean inclusion of modifications of a part of the structure. In addition, a compound in which substitution or non-substitution is not explicitly described is used to mean the inclusion of compounds which have an arbitrary substituent within a range exhibiting the desired effects. This also applies to substituents, linking groups, and ligands.

Moreover, in the present specification, a numerical value range represented by "(a value) to (a value)" means a range including the numerical values indicated before and after "to" as a lower limit value and an upper limit value, respectively.

The photoelectric conversion element and the dye-sensitized solar cell of the present invention have a metal complex dye using a combination of a tridentate ligand with a terminal portion having a nitrogen-containing aromatic ring in which a plurality of thiophene ring groups are introduced to a ring-constituting atom at the 4-position with respect to a coordinating atom by linkage, with a bidentate or tridentate ligand which coordinates to a metal ion through at least one nitrogen atom and an anion of at least one atom. Thus, excellent photoelectric conversion efficiency and high durability are exhibited even when the film thickness of the semiconductor layer is small. Therefore, according to the present invention, it is possible to provide a photoelectric conversion element and a dye-sensitized solar cell, each of which is less affected by the film thickness of a semiconductor layer, exhibits excellent photoelectric conversion efficiency, particularly when the film thickness is small, and has high durability; and a metal complex dye, a dye solution, and a terpyridine compound or an esterified product thereof, each of which is used in the photoelectric conversion element and the dye-sensitized solar cell.

The above or other characteristics and advantages of the present invention will be further clarified from the following description with reference to drawings appropriately attached hereto.

### BRIEF DESCRIPTION OF THE DRAWINGS

Fig. 1 is a cross-sectional view schematically showing a photoelectric conversion element in the first aspect of the present invention, including an enlarged view of the circled portion in the layer thereof, in a system in which the photoelectric conversion element is applied in cell uses.
Fig. 2 is a cross-sectional view schematically showing a dye-sensitized solar cell including a photoelectric conversion element in the second aspect of the present invention.
Fig. 3 is a visible absorption spectrum view of metal complex dyes D-1, D-16 to D-19, and D-24 of the present invention, synthesized in Example 1, in a TBAOH/methanol solvent.
Fig. 4 is a ¹H-NMR spectrum view of the terpyridine compound AC-1 synthesized in Example 1.
Fig. 5 is a ¹H-NMR spectrum view of the terpyridine compound AC-5 synthesized in Example 1.
Fig. 6 is a ¹H-NMR spectrum view of the terpyridine compound AC-6 synthesized in Example 1.

### DESCRIPTION OF THE PREFERRED EMBODIMENTS

### [Photoelectric Conversion Element and Dye-Sensitized Solar Cell]

The photoelectric conversion element of the present invention has an electrically conductive support, a photoconductor layer including an electrolyte, a charge transfer layer including an electrolyte, and a counter electrode (opposite electrode). The photoconductor layer, the charge transfer layer, and the counter electrode are provided in this order on the electrically conductive support.

In the photoelectric conversion element of the present invention, at least a portion of the semiconductor fine particles forming the photoconductor layer carries a metal complex dye represented by Formula (I) which will be described later, as a sensitizing dye. Here, the aspect in which the metal complex dye is carried on the surface of the semiconductor fine particles encompasses an aspect in which the metal complex dye is deposited on the surface of the semiconductor fine particles, an aspect in which the metal complex dye is adsorbed onto the surface of the semiconductor fine particles, and a mixture of the aspects. The adsorption includes chemical adsorption and physical adsorption, with the chemical adsorption being preferable.

The semiconductor fine particles may carry other metal complex dyes, together with the metal complex dye of Formula (I) which will be described later.

The semiconductor fine particles preferably carry a co-adsorbent which will be described later, together with the metal complex dye.

Moreover, the photoconductor layer includes an electrolyte. The electrolyte included in the photoconductor layer may be the same as or different from the electrolyte included in the charge transfer layer, but they are preferably the same. Here, the expression "electrolytes are the same" is used to mean inclusion of both of an aspect in which the components included in the electrolyte of the photoconductor layer are the same as the components included in the electrolyte of the charge transfer layer and the contents of both the components are the same, and an aspect in which the components included in the electrolyte of the photoconductor layer are the same as the components included in the electrolyte of the charge transfer layer but the contents of both the components are different.

The photoelectric conversion element of the present invention is not particularly limited in terms of configurations other than the configuration defined in the present invention, and may adopt known configurations regarding photoelectric conversion elements. The respective layers constituting the photoelectric conversion element of the present invention are designed depending on purposes, and may be formed into, for example, a single layer or multiple layers. Further, layers other than the layers may be included, as necessary.

The dye-sensitized solar cell of the present invention is formed by using the photoelectric conversion element of the present invention.

Hereinafter, preferred embodiments of the photoelectric conversion element and the dye-sensitized solar cell of the present invention will be described.

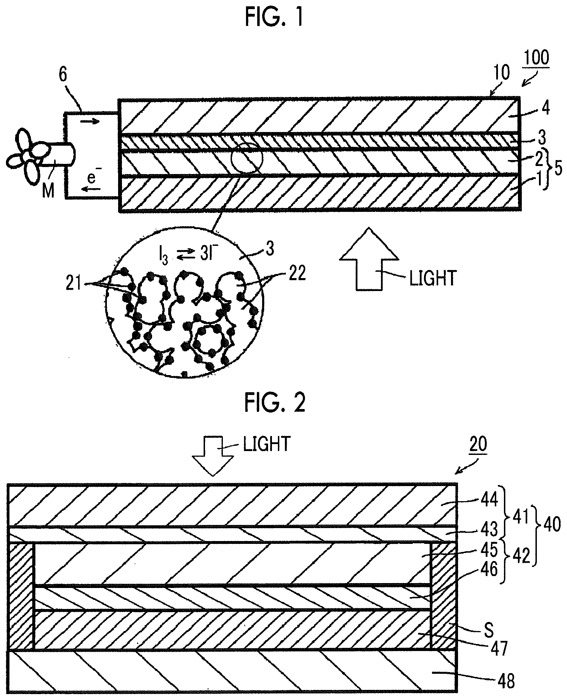

A system 100 shown in Fig. 1 is a system in which a photoelectric conversion element 10 in the first aspect of the present invention is applied in cell uses where an operating means M (for example, an electric motor) in an external circuit 6 is forced to work.

The photoelectric conversion element 10 includes semiconductor fine particles 22 sensitized by carrying an electrically conductive support 1 and a dye (metal complex dye) 21, a photoconductor layer 2 including an electrolyte between the semiconductor fine particles 22, a charge transfer layer 3 that is a hole transport layer, and a counter electrode 4.

In the photoelectric conversion element 10, the light-receiving electrode 5 has the electrically conductive support 1 and the photoconductor layer 2, and functions as a functional electrode.

In the system 100 in which the photoelectric conversion element 10 is applied, light incident to the photoconductor layer 2 excites the metal complex dye 21. The excited metal complex dye 21 has electrons having high energy, and these electrons are transferred from the metal complex dye 21 to a conduction band of the semiconductor fine particles 22, and further reach the electrically conductive support 1 by diffusion. At this time, the metal complex dye 21 is in an oxidized form (cation). While the electrons reaching the electrically conductive support 1 work in an external circuit 6, they reach the oxidized form of the metal complex dye 21 through the counter electrode 4 and the charge transfer layer 3, and reduce the oxidized form, whereby the system 100 functions as a solar cell.

A dye-sensitized solar cell 20 shown in Fig. 2 is constituted with a photoelectric conversion element in the second aspect of the present invention.

With respect to the photoelectric conversion element shown in Fig. 1, the photoelectric conversion element which becomes the dye-sensitized solar cell 20 is different in the configurations of the electrically conductive support 41 and the photoconductor layer 42, and also differs in that it has a spacer S, but except for these, has the same structure as the photoelectric conversion element 10 shown in Fig. 1. That is, the electrically conductive support 41 has a bilayered structure including a substrate 44 and a transparent electrically-conductive film 43 which is formed on the surface of the substrate 44. Further, the photoconductor layer 42 has a bilayered structure including a semiconductor layer 45 and a light-scattering layer 46 which is formed adjacent to the semiconductor layer 45. A spacer S is provided between the electrically conductive support 41 and the counter electrode 48. In the dye-sensitized solar cell 20, 40 is a light-receiving electrode, and 47 is a charge transfer layer.

In a similarly manner to the system 100 in which the photoelectric conversion element 10 is applied, the dye-sensitized solar cell 20 functions as a solar cell by light incident on the photoconductor layer 42.

The photoelectric conversion element and the dye-sensitized solar cell of the present invention are not limited to the above preferred aspects, and the configuration of each of the aspects can be combined as appropriate within a range not departing from the scope of the present invention.

In the present invention, the materials and the respective members for use in the photoelectric conversion element and the dye-sensitized solar cell can be prepared by ordinary methods. Reference can be made to, for example, US4,927,721A, US4,684,537A, US5,084,365A, US5,350,644A, US5,463,057A, US5,525,440A, JP1995-249790A (JP-H07-249790A), JP2001-185244A, JP2001-210390A, JP2003-217688A, JP2004-220974A, and JP2008-135197.

### <Metal Complex Dye Represented by Formula (I)>

The metal complex dye of the present invention is represented by the following Formula (I). The metal complex dye of the present invention is less affected by a change in the film thickness of a semiconductor layer, and can impart high photoelectric conversion efficiency and excellent heat stability to the photoelectric conversion element and the dye-sensitized solar cell by including a ligand LA represented by the following Formula (AL-1). Accordingly, the metal complex dye of the present invention is preferably used as a sensitizing dye in a dye-sensitized solar cell.

Formula (I) M(LA)(LD)(LX)_{mX}·(CI)_{mY}

In Formula (I),
M represents a metal ion,
LA represents a tridentate ligand represented by the following Formula (AL-1).

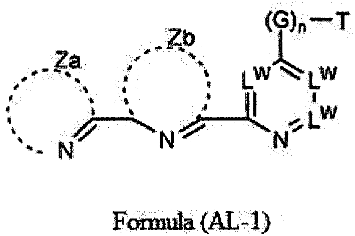

In the formula, Za and Zb each independently represent a non-metal atomic group necessary for forming a 5- or 6-membered ring, in which at least one of rings formed by Za and Zb, respectively, has one or more acidic groups, L^{W}'s each independently represent a nitrogen atom or CR^{W}, and R^{W} represents a hydrogen atom or a substituent.

G represents a group represented by any one of the following Formulae (X-1) to (X-3), n represents an integer of 2 to 7, T represents a hydrogen atom or a substituent, and a -(G)n-T group does not have an acidic group and an amino group.

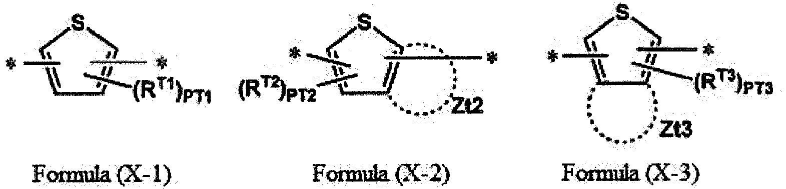

In the formulae, Zt2 and Zt3 each represent a non-metal atomic group necessary for forming a fused ring with a thiophene ring in Formula (X-2) or (X-3).

R^{T1}, R^{T2}, and R^{T3} each independently represent a substituent, PT1 represents an integer of 0 to 2, and PT2 and PT3 each independently represent an integer of 0 or more, and is no more than the number of hydrogen atoms when the group represented by Formula (X-2) or (X-3) is unsubstituted.

* represents a binding position to a ring including L^{W}, or another G or T.

LD represents a bidentate ligand, or a tridentate ligand different from LA. At least one of coordinating atoms in LD, bonded to the metal ion M, is a nitrogen atom, and at least one of the coordinating atoms is an anion.

LX represents a monodentate ligand. When LD is a bidentate ligand, mX represents 1, and when LD is a tridentate ligand, mX represents 0.

CI represents a counterion necessary for neutralizing the charge of the metal complex dye, and mY represents an integer of 0 to 3, preferably 0 or 1, and more preferably 0.

### - Metal Ion M -

M is a central metal ion of the metal complex dye, and examples thereof include ions of elements belonging to Groups 6 to 12 on the long-form periodic table of the elements. Examples of such metal ions include respective ions of Ru, Fe, Os, Cu, W, Cr, Mo, Ni, Pd, Pt, Co, Ir, Rh, Re, Mn, and Zn. The metal ion M may be one kind of ion, or two or more kinds of ions.

In the present invention, the metal ion M is preferably Os²⁺, Ru²⁺, or Fe²⁺, more preferably Os²⁺ or Ru²⁺, and among these, Ru²⁺ is particularly preferable.

In a state of being incorporated in the photoelectric conversion element, the valence of M may be changed by the redox reaction with the surrounding material.

### - Ligand LA -

The ligand LA is a tridentate ligand or compound which is represented by Formula (AL-1) and coordinates to a metal ion M through three nitrogen atoms in Formula (AL-1).

This ligand LA has at least one acidic group (also referred to as an adsorptive group) on at least one of the ring formed by Za and the ring formed by Zb, each of which will be described later. The ligand LA is a ligand which makes the metal complex dye of the present invention carried on the semiconductor fine particles.

The ligand LA has a "-(G)n-T" group on the ring-constituting carbon atom at the 4-position with respect to a ring-constituting nitrogen atom that coordinates to a metal ion M of a ring formed of a nitrogen atom, a carbon atom, and L^{W} (also referred to as a terminal nitrogen-containing ring or a hetero ring including L^{W}). It is thought that in the ligand LA, if the "-(G)n-T" group is bonded to the ring-constituting carbon atom at the 4-position of the hetero ring including L^{W}, the absorbance of a metal complex dye having the ligand LA increases. Thus, a photoelectric conversion element and a dye-sensitized solar cell, each containing the metal complex dye having enhanced absorbance in the photoconductor layer, have improved photoelectric conversion efficiency. Further, even when the film thickness of a semiconductor layer that will provide a photoconductor layer is small, excellent photoelectric conversion efficiency is exhibited. Moreover, the durability of the photoelectric conversion element and the dye-sensitized solar cell is also improved. Accordingly, this ligand LA is preferably used as a ligand of a metal complex dye for use in a dye-sensitized solar cell.

In Formula (AL-1), Za and Zb each independently represent a non-metal atomic group necessary for forming a 5-membered ring or a 6-membered ring. Za and Zb are each preferably a non-metal atomic group selected from a carbon atom and the heteroatoms, and more preferably a non-metal atomic group selected from a carbon atom, a nitrogen atom, an oxygen atom, a sulfur atom, and a phosphorus atom.

The rings formed by Za and Zb are preferably an aromatic hetero ring as a 5-membered ring and an aromatic hetero ring as a 6-membered ring. These rings encompass a monocycle as well as a fused ring formed by the fusion of at least one of an aromatic ring or an aliphatic ring to the monocycle. Further, the ring formed by Za and the ring formed by Zb may have a substituent, which is preferably selected from the substituent group T^{R} which will be described later, and a fused ring formed by the mutual bonding of the rings through this substituent may be formed. Examples of such a fused ring include a 1,10-phenanthroline ring.

The aromatic hetero ring as a 5-membered ring may be any one of 5-membered rings including the heteroatom as a ring-constituting atom. It is preferably, for example, at least one of a pyrazole ring, an imidazole ring, a triazole ring, a thiazole ring, an oxazole ring, a benzimidazole ring, a benzotriazole ring, a benzoxazole ring, and a benzothiazole ring. The aromatic hetero ring as a 6-membered ring may be any one of 6-membered rings including the heteroatom as a ring-constituting atom. It is preferably, for example, at least one of a pyridine ring, a pyrimidine ring, a pyrazine ring, a pyridazine ring, a triazine ring, a tetrazine ring, a quinoline ring, and an isoquinoline ring.

The rings formed by Za and Zb are each at least one selected from the group consisting of the group including the aromatic hetero rings as a 5-membered ring and the group including the aromatic hetero rings as a 6-membered ring, and aromatic hetero rings which are suitable for the structures of the respective rings represented by Formula (AL-1) are preferably selected.

The ring formed by Za is preferably at least one selected from the group consisting of a pyridine ring, a pyrimidine ring, a pyrazine ring, a pyridazine ring, a triazine ring, a tetrazine ring, a quinoline ring, an isoquinoline ring, an imidazole ring, a pyrazole ring, a triazole ring, a thiazole ring, an oxazole ring, a benzimidazole ring, a benzotriazole ring, a benzoxazole ring, and a benzothiazole ring.

The ring formed by Zb is preferably at least one selected from the group consisting of a pyridine ring, a pyrimidine ring, a pyrazine ring, a pyridazine ring, a triazine ring, a tetrazine ring, a quinoline ring, an isoquinoline ring, an imidazole ring, a triazole ring, a thiazole ring, an oxazole ring, a benzimidazole ring, a benzotriazole ring, a benzoxazole ring, and a benzothiazole ring.

Among those, the hetero rings formed by Za and Zb are more preferably each an imidazole ring, a pyridine ring, or a pyrimidine ring, and particularly preferably are both pyridine rings.

At least one of the hetero rings formed by Za and Zb has one or more acidic groups, and it is preferable that each of the hetero rings has one or more acidic groups. The number of the acidic groups contained in each of the hetero rings formed by Za and Zb is preferably 1 to 3, more preferably 1 or 2, and still more preferably 1. It is more preferable that hetero rings formed by Za and Zb each have one acidic group.

The substitution position of the acidic group is not particularly limited. In each of the rings, the substitution position is preferably a ring-constituting atom that is the farthest from the nitrogen atom coordinating to the metal ion M, and in a case where the ring is a 6-membered ring, the substitution position is preferably the 4-position with respect to the nitrogen atom.

In the present invention, the acidic group is a substituent which has a dissociative proton and has a pKa of 11 or less. The pKa of the acidic group can be determined in accordance with the "SMD/M05-2X/6-31G*" method described in J. Phys. Chem. A2011, 115, pp. 6641-6645. Examples thereof include: an acid group showing acidity, such as a carboxyl group, a phosphonyl group, a phosphoryl group, a sulfo group, and a boric acid group; or groups having these acidic groups. Examples of the group having an acid group include groups having an acid group and a linking group. The linking group is not particularly limited, and examples thereof include a divalent group, and preferably an alkylene group, an alkenylene group, an alkynylene group, an arylene group, and a heteroarylene group. This linking group may have a group selected from the substituent group T^{R} which will be described later as a substituent. Preferred examples of the acidic group having an acid group and a linking group include carboxymethyl, carboxyvinylene, dicarboxyvinylene, cyanocarboxyvinylene, 2-carboxy-1-propenyl, 2-carboxy-1-butenyl, and carboxyphenyl.

The acidic group is preferably a carboxyl group, a phosphonyl group, a sulfo group, or a group having a carboxyl group, and more preferably a carboxyl group.

The acidic group may be in the form of a dissociated anion due to release of a proton or in the form of a salt when the acidic group is included in the metal complex dye represented by Formula (1). When the acidic group is in the form of a salt, the counterion is not particularly limited, and examples thereof include those exemplified as positive ions in the following counterion CI. In addition, the acidic group may be esterified as described later.

The hetero rings formed by Za and Zb may or may not have a substituent other than the acidic group. Examples of the substituent which may be contained in these hetero rings include a group selected from the substituent group T^{R} which will be described later.

In Formula (AL-1), the hetero ring including L^{W} encompasses a monocycle and a fused ring, and in a case where the hetero ring including L^{W} is a fused ring, a fused ring with the hetero ring formed by Zb is also encompassed.

L^{W} represents a nitrogen atom or CR^{W}. R^{W} represents a hydrogen atom or a substituent, with a hydrogen atom being preferable. The substituent which can be adopted as R^{W} is not particularly limited, and examples thereof include a group selected from the substituent group T^{R} which will be described later (preferably excluding a thiophene ring group). In a case where the hetero ring including L^{W} has a plurality of R^{W}'s, R^{W}'s may be bonded to each other to form a ring.

As the hetero ring including L^{W}, an aromatic hetero ring which is suitable for the ring structure in Formula (AL-1) is preferably selected from the aromatic rings as 6-membered rings, described as the hetero rings formed by Za and Zb. The hetero ring is more preferably at least one of a pyridine ring, a pyrimidine ring, a pyridazine ring, a triazine ring, a tetrazine ring, a quinoline ring, or an isoquinoline ring, still more preferably a pyridine ring or a pyrimidine ring, and particularly preferably a pyridine ring.

The ligand LA has a group represented by "-(G)n-T" in the hetero ring including L^{W}. This group is a group formed by 2 to 7 bondings of thiophene-containing fused rings including a thiophene ring or a thiophene ring through single bonds.

G that forms the group is a thiophene ring, or a thiophene-containing fused ring, and is represented by any one of the following Formulae (X-1) to (X-3).

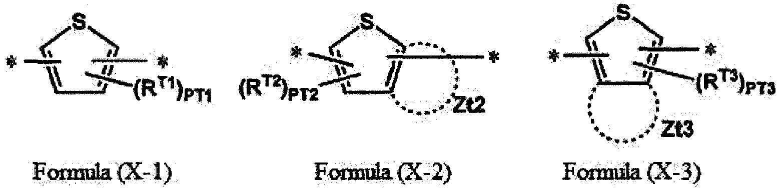

The thiophene ring group represented by Formula (X-1) may have a substituent R^{T1}. Examples of the substituent R^{T1} include a group (excluding an amino group) selected from the substituent group T^{R} which will be described later. The substituent R^{T1} is preferably an alkyl group, an alkenyl group, an alkynyl group, an alkoxy group, an alkylthio group, an arylthio group, a cycloalkyl group, an aryl group, an alkenyloxy group, an alkynyloxy group, an aryloxy group, a cyano group, or a halogen atom. Among those, an alkyl group, an alkenyl group, an alkynyl group, an alkoxy group, an alkylthio group, an arylthio group, and an aryloxy group are more preferable; an alkyl group, an alkenyl group, an alkynyl group, an alkoxy group, and an alkylthio group are still more preferable; and an alkyl group is particularly preferable. The number of carbon atoms in the alkyl group that can be adopted as R^{T1} is preferably 1 to 20, more preferably 1 to 12, and still more preferably 1 to 9.

In a case where the thiophene ring group represented by Formula (X-1) has the substituent R^{T1}, the substitution position is not particularly limited. The number PT1 of the substituents R^{T1} is 0 to 2, and preferably 0 or 1. * represents a binding position to a ring including Lw, or another G or T. The binding position of the thiophene ring group represented by Formula (X-1) is not particularly limited, and may be any one of the ring-constituting carbon atoms. It is preferable that at least one binding position is the 2-position with respect to the ring-constituting sulfur atom.

In Formula (X-2), Zt2 represents a non-metal atomic group necessary for forming a fused ring with the thiophene ring in Formula (X-2). The non-metal atomic group is selected from a carbon atom, a hydrogen atom, and the heteroatoms, according to the thiophene-containing fused ring group represented by Formula (X-2).

The thiophene-containing fused ring group represented by Formula (X-2) is a fused ring group formed by the fusion of at least one aromatic ring or aliphatic ring with the ring-constituting carbon atoms at the 2- and 3-positions with respect to the ring-constituting sulfur atom in the thiophene ring in Formula (X-2). The ring to be fused with the thiophene ring is not particularly limited, but is preferably an aromatic ring or an aliphatic hetero ring, and more preferably an aromatic hetero ring. Further, the ring to be fused is not particularly limited, but is preferably a 5- or 6-membered ring, and more preferably a 5-membered ring. Examples of such a ring include a pyrrole ring, a thiophene ring, a furan ring, a silole ring, a phosphole ring, and a selenophene ring. Among those, a thiophene ring, a furan ring, and a silole ring are preferable, and a thiophene ring is more preferable.

The number of rings (including the thiophene rings in the formula) that form the thiophene-containing fused ring group represented by Formula (X-2) is not particularly limited, but is preferably 2 to 5, and more preferably 2 or 3.

The thiophene-containing fused ring group represented by Formula (X-2) may have a substituent R^{T2}. The substituent R^{T2} has the same definition as the substituent R^{T1}, and preferred examples thereof are is also the same. In a case where the thiophene-containing fused ring group represented by Formula (X-2) has the substituent R^{T2}, the substitution position is not particularly limited. The substituent R^{T2} may be bonded to the ring-constituting carbon atom of the thiophene ring, or may be bonded to the ring-constituting carbon atom of a ring to be fused.

The number PT2 of the substituents R^{T2} contained in the thiophene-containing fused ring group is an integer of 0 or more. The upper limit is no more than the number of hydrogen atoms contained in the fused ring group when the thiophene-containing fused ring group represented by Formula (X-2) is unsubstituted. If PT2 falls within the range, it is not particularly limited, but is preferably 0 to 2, and more preferably 0 or 1.

* represents a binding position to a ring including L^{W}, or another G or T. The binding position of the thiophene-containing fused ring group represented by Formula (X-2) is not particularly limited, and may by any one of the ring-constituting carbon atoms. Preferably, at least one binding position is the 2-position with respect to the ring-constituting sulfur atom.

In Formula (X-3), Zt3 represents a non-metal atomic group necessary for forming a fused ring with the thiophene ring in Formula (X-3). The non-metal atomic group is selected from a carbon atom, a hydrogen atom, and the heteroatom, according to the thiophene-containing fused ring group represented by Formula (X-3).

The thiophene-containing fused ring group represented by Formula (X-3) is a fused ring group formed by the fusion of at least one aromatic ring or aliphatic ring with the ring-constituting carbon atoms at the 3- and 4-positions with respect to the ring-constituting sulfur atom in the thiophene ring in Formula (X-3). A first aspect of the ring fused with the thiophene ring has the same definition as the ring fused with the thiophene ring in Formula (X-2), and preferred examples thereof are also the same. A second aspect is an aliphatic hetero ring formed by the bonding of an alkylenedioxy group or alkylenedithio group at the 3- and 4-positions of the thiophene ring in Formula (X-3) to the ring-constituting carbon atom. In the second aspect, an alkylenedioxy group is preferable, and examples thereof include ethylenedioxy and propylenedioxy.

The number of rings (including the thiophene ring in the formula) that form the thiophene-containing fused ring group represented by Formula (X-3) is not particularly limited, but is preferably 2 to 4, and more preferably 2.

The thiophene-containing fused ring group represented by Formula (X-3) may have a substituent R^{T3}. The substituent R^{T3} has the same definition as the substituent R^{T1}, and preferred examples thereof are is also the same. In a case where the thiophene-containing fused ring group represented by Formula (X-3) has the substituent R^{T3}, the substitution position is not particularly limited. The substituent may be bonded to the ring-constituting carbon atom of the thiophene ring, or may be bonded to the ring-constituting carbon atom of the ring to be fused.

The number PT3 of the substituents R^{T3} contained in the thiophene-containing fused ring group is an integer of 0 or more. The upper limit is no more than the number of hydrogen atoms contained in the fused ring group when the thiophene-containing fused ring group represented by Formula (X-3) is unsubstituted. If PT3 falls within the range, it is not particularly limited, but is preferably 0 to 2, and more preferably 0 or 1.

* represents a binding position to a ring including L^{W}, or another G or T. The binding position of the thiophene-containing fused ring group represented by Formula (X-3) is not particularly limited, and may by any one of the ring-constituting carbon atoms. Preferably, at least one binding position is the 2-position with respect to the ring-constituting sulfur atom.

The thiophene ring group represented by Formula (X-1) is preferably a ring group represented by any one of the following Formulae (X-1a) to (X-1c).

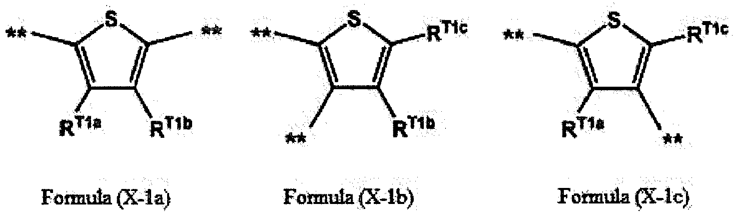

In the formulae, R^{T1a} to R^{T1c} each independently represent a hydrogen atom or a substituent. The substituent has the same definition as R^{T1} in Formula (X-1), and preferred examples thereof are is also the same. In the present invention, there is no case where R^{T1a} and R^{T1b}, and R^{T1b} and R^{T1c} may each be bonded to each other to form a ring. ** represents a binding position to a ring including L^{W}, or another G or T. In the thiophene ring groups represented by Formulae (X-1a) to (X-1c), any one of two **'s may be bonded to the ring including L^{W} or to G adjacent to the side of the ring including L^{W}.

Among the thiophene ring groups represented by the respective formulae, the thiophene ring group represented by Formula (X-1a) is preferable.

The thiophene-containing fused ring group represented by Formula (X-2) is preferably a ring group represented by any one of the following Formulae (X-2a) to (X-2e).

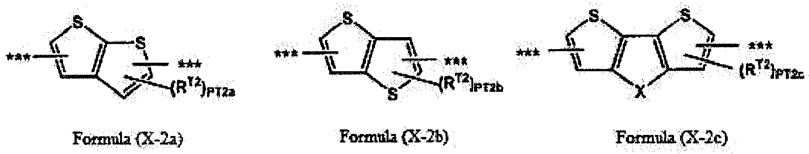

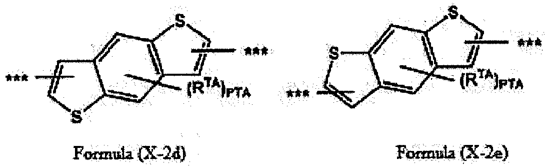

In the formula, R^{T2} and R^{TA} each represent a substituent. The substituents R^{T2} and R^{TA} have the same definitions as R^{T2} of Formula (X-2), and preferred examples thereof are also the same. The numbers PT2a, PT2b, and PT2c of the substituents R^{T2} are each an integer of 0 to 2, and more preferably 0 or 1. R^{T2} may be bonded to any thiophene ring in the formulae. In the present invention, in a case where two substituents R^{T2} are included, it is preferable that two substituents R^{T2} are not bonded to each other to form a ring.

The numbers PTA of the substituents R^{TA} are each an integer of 0 to 4, more preferably 0 to 2, and particularly preferably 2. R^{TA}'s may be bonded to any ring of a thiophene ring and a benzene ring in the following formulae. In the present invention, in a case where two substituents R^{TA} are included, it is preferable that two substituents R^{TA} are not bonded to each other to form a ring.

In the ring groups represented by the respective formulae, *** represents a binding position to a ring including L^{W}, or another G or T. The binding positions of these ring groups are not particularly limited, and may be any of the ring-constituting carbon atoms. The binding positions of the ring groups represented by the respective formulae are all preferably two ring-constituting carbon atoms adjacent to the ring-constituting sulfur atoms in the thiophene ring in the respective formulae.

In Formula (X-2c), X represents -O-, -S-, -NR^{X2c}-, -C(R^{X2c})₂-, -(R^{X2c})C=C(R^{X2c})-, or -Si(R^{X2c})₂-. Among those, X is preferably -O-, -S-, -C(R^{X2c})₂-, or -Si(R^{X2c})₂-, and more preferably -C(R^{X2x})₂-, -O-, or -S-. Here, R^{X2c} represents a hydrogen atom or a substituent. This substituent has the same definition as R^{T2}, but is preferably an alkyl group.

Among the thiophene-containing fused ring groups represented by the respective formulae, the thiophene ring group represented by Formula (X-2b), Formula (X-2c), Formula (X-2d), or Formula (X-2e) is preferable; the thiophene ring group represented by Formula (X-2b), Formula (X-2c), or Formula (X-2d) is more preferably; and the thiophene ring group represented by Formula (X-2b) or Formula (X-2c) is particularly preferable.

The thiophene-containing fused ring group represented by Formula (X-3) is preferably a ring group represented by the following Formula (X-3a) or (X-3b), and particularly preferably a ring group represented by the following Formula (X-3b).

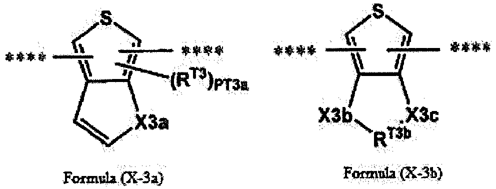

In the formula, X3a to X3c each independently represent -O- or -S-. X3a is preferably -S-, and X3b and X3c are preferably both -O-.

In the ring groups represented by the respective formulae, **** represents a binding position to a ring including L^{W}, or another G or T. The binding position of the ring group represented by Formula (X-3a) is not particularly limited, and may be any one of the ring-constituting carbon atoms. The binding positions of the ring groups represented by Formulae (X-3a) and (X-3b) are both preferably the 2- and 5-positions of the thiophene ring.

In the ring group represented by Formula (X-3a), R¹³ represents a substituent. The substituent has the same definition as R^{T3} of Formula (X-3), and preferred examples thereof are also the same. The number PT3a of the substituents R^{T3} is an integer of 0 to 2, and more preferably 0 or 1. In the present invention, even in a case where the ring group has two substituents R^{T3}'s, it is preferable that the two substituents R^{T3}'s are not bonded to each other to form a ring. R^{T3} may be bonded to the thiophene ring in the formula, or may be bonded to a ring having X3a as a ring-constituting atom.

In the ring group represented by Formula (X-3b), R^{T3b} represents an alkylene group. In a case where R^{T3b} is an alkylene group, the -X3b-R^{T3b}-X3c- group is preferably an alkylenedioxy group. This alkylenedioxy group has the same definition as the alkylenedioxy group in Formula (X-3), and preferred examples thereof are also the same.

In the group represented by "-(G)n-T", the number n of G's is an integer of 2 to 7, preferably an integer of 2 to 5, and more preferably 2 or 3.

Here, the combination of the bonded G's is not particularly limited. The G's to be combined may be the same G's or different G's. Here, the same G's mean G's in which the thiophene ring structures represented by the respective formulae or thiophene-containing fused ring structures are the same, and also include G's having difference in the presence or absence, the kinds, and the binding positions of substituents.

Preferred examples of the combination of G's include -Formula (X-1a)-Formula (X-1a)-, -Formula (X-1a)-Formula (X-2b)-, -Formula (X-1a)-Formula (X-2c)-, -Formula (X-1a)-Formula (X-2e)-, -Formula (X-la)-Formula (X-3b)-, -Formula (X-2b)-Formula (X-2b)-, -Formula (X-2b)-Formula (X-3b)-, -Formula (X-2c)-Formula (X-3b)-, -Formula (X-3a)-Formula (X-3b)-, and -Formula (X-3b)-Formula (X-3b)-.

More preferred examples of the combination of G's are specifically shown below. The following specific examples all have no substituent, and the respective examples may have a substituent. The substituent is as described above. Further, in the following examples, the wavy line represents a binding position to a ring including Lw, or another G or T.

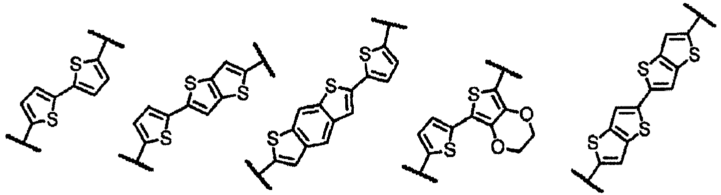

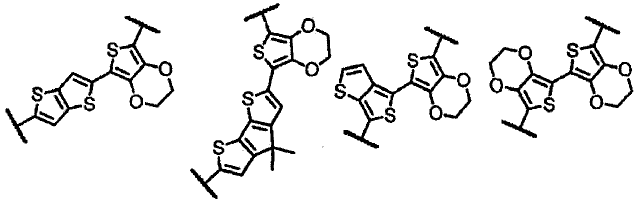

Furthermore, more preferred examples of the combination of G's include a combination "-(G)n-" of G's in the specific examples of the ligand LA which will be described later, and also include a combination "-(G)n-" of G's contained in the ligand LA in the metal complex dyes D-1 to D-33 synthesized in Examples which will be described later.

In the present invention, as the combination "-(G)n-" of G's, the "-(G)n-" in the specific examples of the ligand LA which will be described later and the "-(G)n-" contained in the ligand LA in the metal complex dyes D-1 to D-33 are still more preferable, and the "-(G)n-" contained in the ligand LA in the metal complex dyes D-1 to D-33 is particularly preferable.

In the group represented by "-(G)n-T", T represents a hydrogen atom or a substituent. The substituent is not particularly limited, but has the same definition as R^{T1}, preferred examples thereof are the same, and among those, an alkyl group or an alkylthio group is preferable. The number of the carbon atoms in the alkyl group is preferably 1 to 20, more preferably 1 to 12, and more preferably 1 to 9. The number of carbon atoms in the alkylthio group is preferably 1 to 20, more preferably 1 to 12, still more preferably 1 to 9, and particularly preferably 1 to 6.

As the combination "-(G)n-" of G's, a combination of two Formulae (X-1a) is the most preferable. As the group represented by "-(G)n-T" including the most preferred combination of G's, a group represented by the following Formula (TF-1) is preferable.

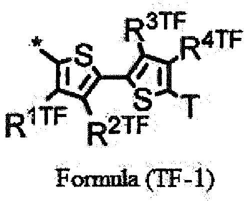

In the formula, R^{1TF} to R^{4TF} each independently represent a hydrogen atom or an alkyl group. As each of R^{1TF} to R^{4TF}, a hydrogen atom or an alkyl group is appropriately selected. Among those, both of an aspect in which R^{1TF}, R^{2TF}, and R^{4TF} are each a hydrogen atom, and R^{3TF} is an alkyl group, and an aspect in which R^{1TF} and R^{3TF} are each a hydrogen atom, and R^{2TF} and R^{4TF} are each an alkyl group are particularly preferable. In a case where R^{1TF} to R^{4TF} are each an alkyl group, a plurality of alkyl group's may be the same as or different from each other.

The alkyl group that can be contained in R^{1TF} to R^{4TF} has the same definition as the alkyl group of R^{T1}, and is selected from the alkyl groups in the substituent group T^{R} which will be described later' but the number of carbon atoms thereof is preferably 3 to 12, and more preferably 5 to 9.

T represents a hydrogen atom or a substituent, and is as described above.

* represents a binding portion to a ring including L^{W}.

The group represented by "-(G)n-T" does not have the acidic group. In this case, the respective substituents and T's which may be contained in G's in the number of n are selected from the substituent group T^{R} which will be described later

Furthermore, the group represented by "-(G)n-T" does not have an amino group. In this case, the respective substituents and T's which may be contained in G's in the number of n may be an amino group or a substituent not having an amino group, which is selected from the substituent group T^{R} which will be described later

In the present invention, examples of the amino group include an alkylamino group, an arylamino group, and a heteroarylamino group, in addition to an unsubstituted amino group (-NH₂).

The ligand LA is preferably a tridentate ligand (terpyridine compound) represented by the following Formula (AL-2).

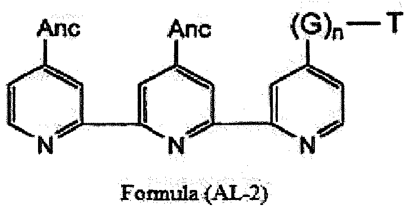

In the formula, two Anc's each independently represent an acidic group. The acidic group has the same definition as the acidic group in Formula (AL-1), and preferred examples thereof are also the same.

G, T, and n have the same definitions as G, T, and n, respectively, in Formula (AL-1).

The terpyridine compound is the ligand LA itself, but in the present invention, the ligand LA can also be used as a precursor compound of the ligand LA as described later. Accordingly, in the present invention, the term, a ligand LA, encompasses a precursor compound of the ligand LA, in addition to the ligand LA itself (the terpyridine compound). Preferred examples of the precursor compound include an esterified product in which at least one of the acidic groups A of the terpyridine compound is esterified (also referred to as an esterified product of the terpyridine compound).

This esterified product is a compound having the acidic group protected, which is an ester capable of being regenerated into an acidic group by hydrolysis or the like, and is not particularly limited. Examples thereof include an alkyl esterified product, an aryl esterified product, and a heteroaryl esterified product of the acidic group. Among these, the alkyl esterified product is preferable. The alkyl group which forms an alkyl esterified product is not particularly limited, but an alkyl group having 1 to 10 carbon atoms is preferable, an alkyl group having 1 to 6 carbon atoms is more preferable, and an alkyl group having 1 to 4 carbon atoms is still more preferable. The aryl group which forms an aryl esterified product and the heteroaryl group which forms a heteroaryl esterified product are each not particularly limited, and examples thereof include the substituent group T which will be described later. These groups may have at least one substituent selected from the substituent group T which will be described later.

It is preferable that two acidic groups to be esterified are used. In this case, the two esters may be the same as or different from each other.

The ligand LA can be synthesized in accordance with ordinary methods. For example, the ligand LA represented by Formula (LI-4) can be synthesized by subjecting a compound represented by Formula (L1-1) and a compound represented by Formula (L1-2) to a coupling reaction, and hydrolyzing an ester group of a precursor compound represented by Formula (L1-3), as shown in the following scheme. In this synthesis method, an esterified product of a carboxyl group is shown as the precursor compound, but the present invention is not limited thereto, and any of precursor compounds obtained by esterification of any one of the acidic groups may be used.

The coupling reaction herein can be carried out by, for example, "a Stille coupling reaction", a "Suzuki coupling method" described in "Experimental Chemistry Course, Fifth Edition", Maruzen Co., Ltd., edited by The Chemical Society of Japan, Vol. 13, pp. 92-117, or methods equivalent thereto. Further, the hydrolysis can be carried out in accordance with, for example, the method described in "Experimental Chemistry Course, Fifth Edition", Maruzen Co., Ltd., edited by The Chemical Society of Japan, Vol. 16, pp. 10-15. For example, the synthesis methods in Examples which will be described later may also be mentioned.

In the present invention, the metal complex dye of the present invention can be synthesized using the ligand LA synthesized by the hydrolysis of the precursor compound. Further, the metal complex dye of the present invention can also be synthesized by forming a metal complex dye using a precursor compound, and then hydrolyzing an ester group in accordance with the above method, as in Example 1 which will be described later.

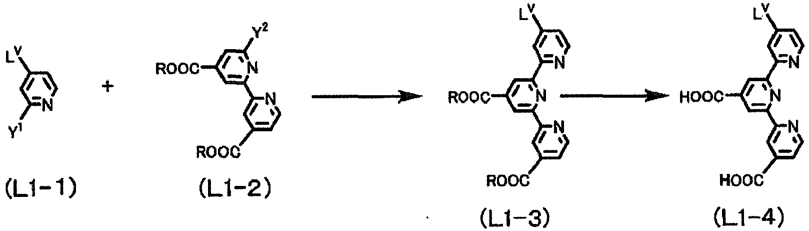

In the formulae, L^{V} has the same definitions as the group represented by "-(G)n-T". Y¹ represents a trialkyl tin group, a boronic acid group, a boronic acid ester group, a halogen atom, or a perfluoroalkylsulfonyloxy group.

In Formula (L1-2), in a case where Y¹ of Formula (L1-1) is a trialkyl tin group, a boronic acid group, or a boronic acid ester group, Y² represents a halogen atom or a perfluoroalkylsulfonyloxy group, and in a case where Y¹ of Formula (L1-1) is a halogen atom or a perfluoroalkylsulfonyloxy group, Y² represents a trialkyl tin group, a boronic acid group, or a boronic acid ester group.

In Formulae (L1-2) and (L1-3), R represents an alkyl group, an aryl group, or a heteroaryl group.

Specific examples of the ligand LA are shown below. Examples of the ligand LA also include the ligand LA in the metal complex dye which will be described later. Other examples thereof include the compounds in which at least one of -COOH's is formed into a salt of a carboxyl group, with respect to the ligands LA in the following specific examples and the specific examples of the metal complex dye. In these compounds, examples of the counter cation that forms a salt of a carboxyl group include the positive ions described as CI below. Further, examples of the esterified product of the terpyridine compound include the compounds in which at least one of acidic groups is esterified, with respect to the ligands LA in the following specific examples and the specific examples of the metal complex dye. The present invention is not limited to these ligands LA, or salts or esterified products thereof.

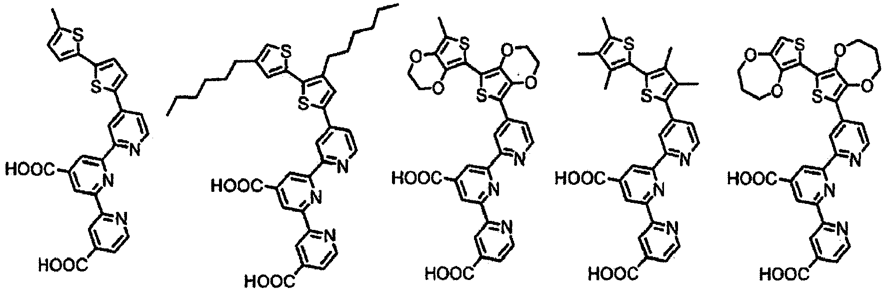

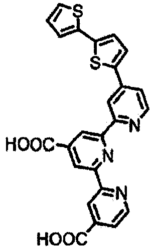

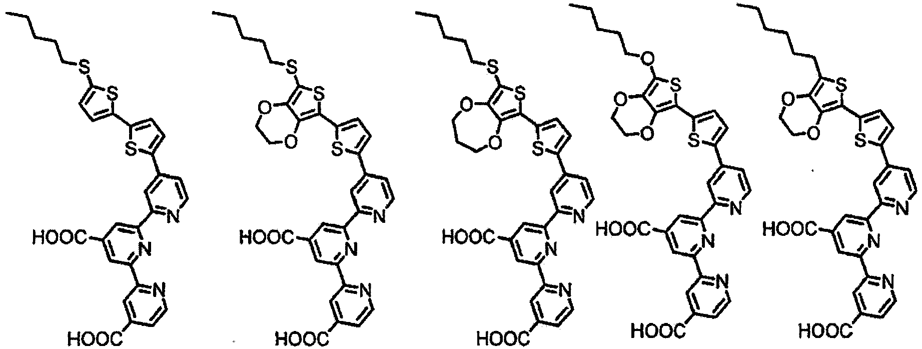

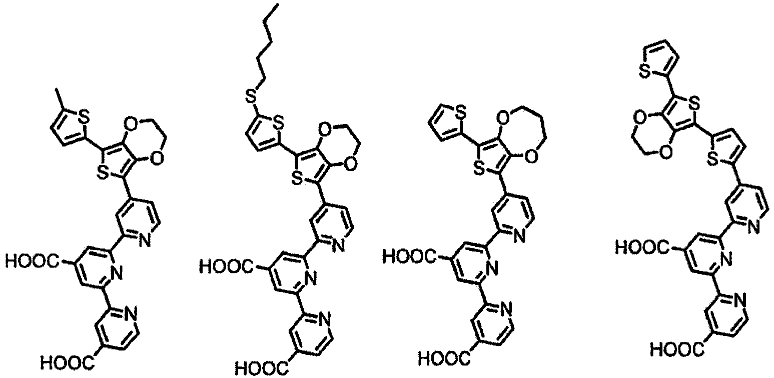

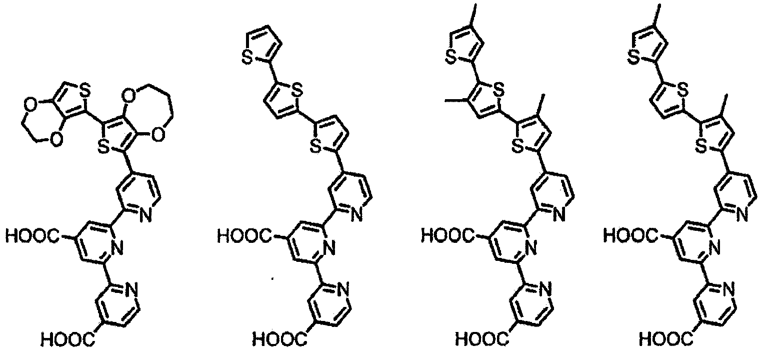

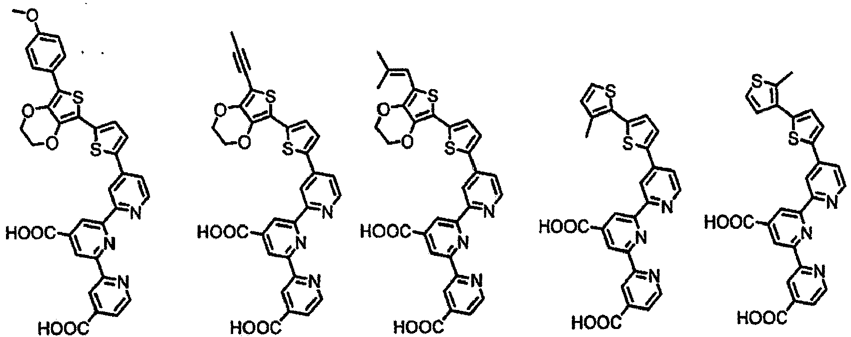

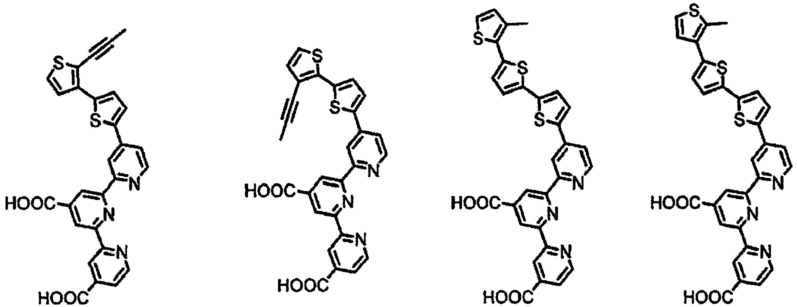

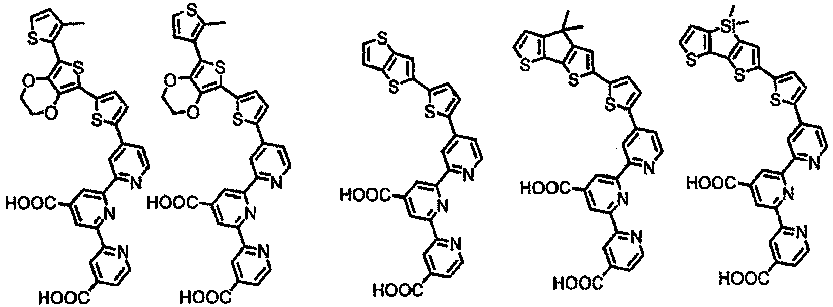

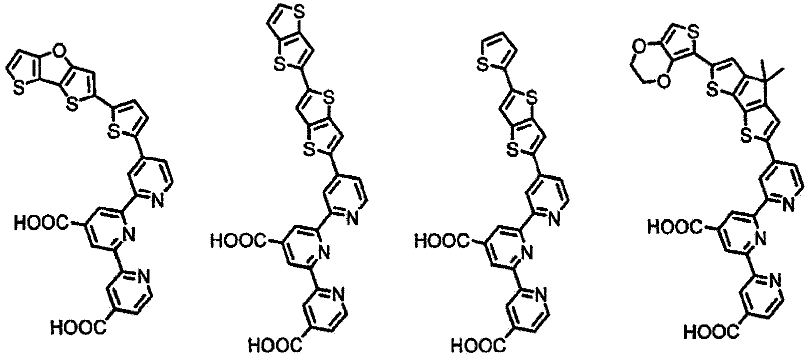

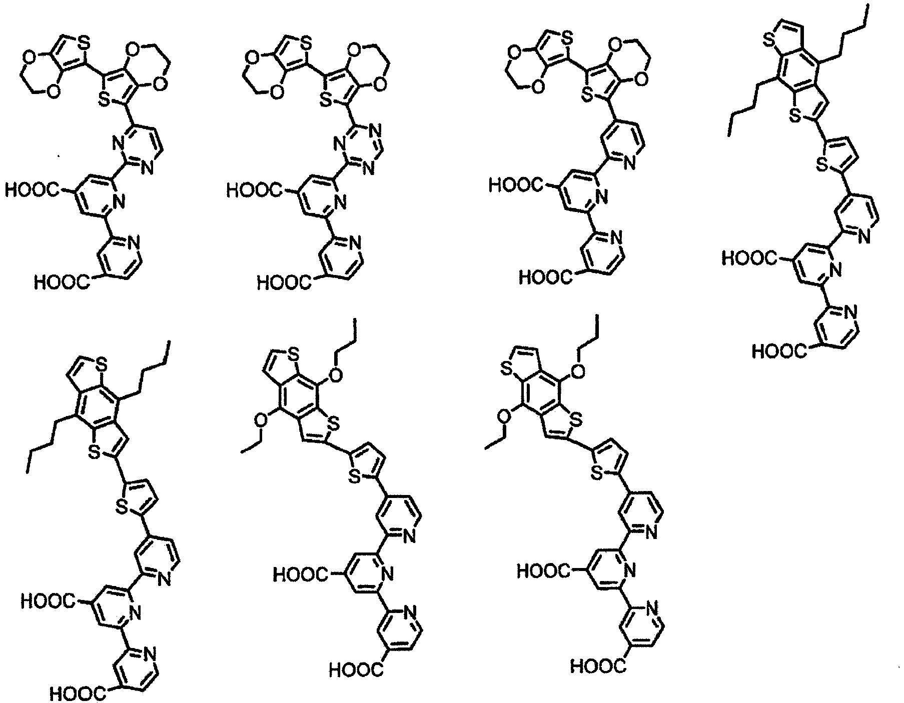

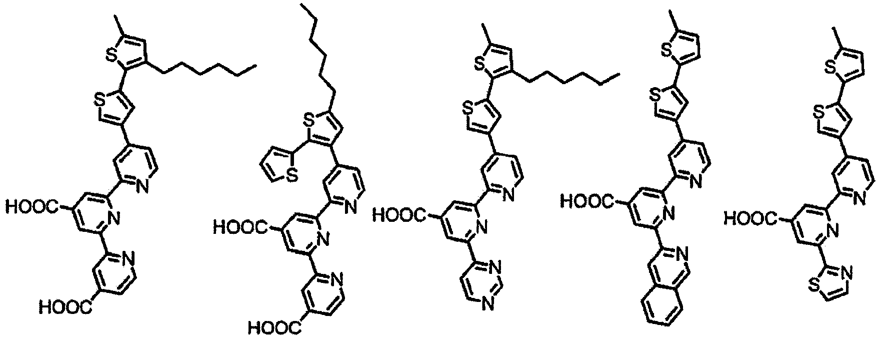

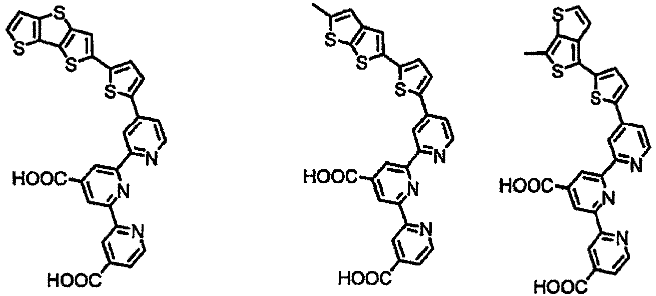

### - Ligand LD -

LD is a bidentate ligand, or a tridentate ligand different from the ligand LA.

It is preferable that this ligand LD does not have an acidic group adsorbed on the surface of semiconductor fine particles. Even when the ligand LD includes a group corresponding to the acidic group, it is preferable that the group is not adsorbed on the surface of semiconductor fine particles.

In the ligand LD, at least one of coordinating atoms bonded to a metal ion M is nitrogen atom. This nitrogen atom preferably coordinates to the metal ion M through a lone electron pair. Examples of such a nitrogen atom include a nitrogen atom, not having a hydrogen atom as a ring-constituting atom. Examples thereof include a nitrogen atom of a pyridine ring.

In the ligand LD, at least one of coordinating atoms is an anion. The expression, "being an anion", means that a hydrogen atom in a molecule or a hydrogen atom bonded to a coordinating atom can be dissociated and bonded to the metal ion M.

Here, the coordinating atom which becomes an anion may be a nitrogen atom coordinating to the metal ion M, or may be another atom, for example, a carbon atom. In the present invention, it is preferable that the nitrogen atom coordinating to the metal ion M is different from the coordinating atom which will become an anion.

If the metal complex dye has the ligand LD coordinating to the metal ion M through the nitrogen atom and the anion of a coordinating atom, together with the ligand LA, the heat stability of the photoelectric conversion element or the dye-sensitized solar cell is enhanced, and in particular, high durability as well as high photoelectric conversion efficiency are exhibited.

The ligand LD is preferably a ligand represented by the following Formula (DL).

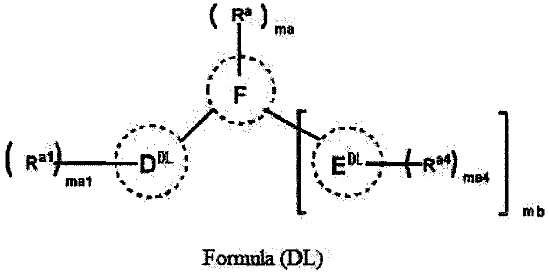

In the formula, the ring D^{DL}, the ring E^{DL}, and the ring F each independently represent an aromatic ring as a 5- or 6-membered ring. R^{a}, R^{a1}, and R^{a4} each independently represent a substituent not having an acidic group. mb represents 0 or 1.

ma1 and ma4 each independently represent an integer of 0 to 3. When mb is 0, ma represents an integer of 0 to 4, and when mb is 1, ma represents an integer of 0 to 3.

Here, when ma, ma1, and ma4 are each an integer of 2 or more, a plurality of R^{a}'s, a plurality of R^{a1}'s, and a plurality of R^{a4}'s may be the same as or different from each other, and they may be bonded to each other to form a ring. Further, R^{a} and R^{a1}, and R^{a} and R^{a4} may be linked to each other to form a ring.

Examples of the aromatic ring as a 5- or 6-membered ring in the ring D^{DL}, the ring E^{DL}, and the ring F include an aromatic hydrocarbon ring and an aromatic hetero ring, with an aromatic hetero ring being preferable. The respective rings of the ring D^{DL}, the ring E^{DL}, and the ring F may be fused with at least one of an aromatic ring and an aliphatic hydrocarbon ring.

In a case where the ring D^{DL}, the ring E^{DL}, and the ring F are each an aromatic hydrocarbon ring, a benzene ring is preferable.

The aromatic hetero ring may be any of aromatic rings including the heteroatom as a ring-constituting atom, and is preferably, for examples, a non-fused 6-membered ring, a 6-membered ring fused with a 5-membered ring, a 5-membered ring fused with a benzene ring, or a 6-membered ring fused with a benzene ring, more preferably a non-fused 6-membered ring or a 6-membered ring fused with a 5-membered ring, and still more preferably a non-fused 6-membered ring.

Examples of such an aromatic hetero ring include, as a 6-membered ring, a pyridine ring, a pyrimidine ring, a pyrazine ring, a triazine ring, a quinoline ring, and a quinazoline ring; and as a 5-membered ring, a pyrrole ring, an imidazole ring, a pyrazole ring, an oxazole ring, a thiazole ring, a benzimidazole ring, a benzoxazole ring, a benzothiazole ring, an indole ring, an indazole ring, a triazole ring, a thiophene ring, and a furan ring.

The ring D^{DL} and the ring E^{DL} are each preferably a pyrrole ring, a pyrazole ring, an imidazole ring, a triazole ring, or a benzene ring, and more preferably a pyrazole ring, a triazole ring, or a benzene ring.

The ring F is preferably a nitrogen atom-containing aromatic hetero ring, more preferably a pyridine ring, a pyrimidine ring, a pyrazine ring, or a triazine ring, still more preferably a pyridine ring or a pyrimidine ring, and particularly preferably a pyridine ring.

Here, the ring D^{DL}, the ring E^{DL}, and the ring F each include a coordinating atom bonded to the metal ion M. The coordinating atom is not particularly limited, but is preferably a carbon atom, a nitrogen atom, a sulfur atom, an oxygen atom, or an anion of any of these atoms.

The anion bonded to the metal ion M is not particularly limited, and preferred examples thereof include carbon anions such as a =C⁻- ion, and nitrogen anions such as >N⁻ ion.

Examples of the substituents of R^{a}, R^{a1}, and R^{a4} include a group selected from the substituent group T^{R} which will be described later.

Among those, R^{a} is preferably an aromatic hetero ring group, an aryl group, an ethenyl group, an ethynyl group, a halogen atom, an alkyl group, an amino group (including an alkylamino group, a dialkylamino group, an arylamino group, a diarylamino group, an N-alkyl-N-arylamino group, and the like), an alkoxy group, an aryloxy group, an alkylthio group, an arylthio group, or a silyl group, and more preferably an aromatic hetero ring group, an aryl group, an ethenyl group, an ethynyl group, an alkyl group, an alkoxy group, or an amino group (including an alkylamino group, a dialkylamino group, an arylamino group, a diarylamino group, and the like). Further, a group formed by the combination of the respective groups is also preferable.

The number of carbon atoms in the respective substituents which can be adopted as R^{a} is not particularly limited, but is preferably the same as the number of carbon atoms in the substituent which can be adopted as R^{AA}, with respect to the same substituent as the substituent which can be adopted as R^{AA}, which will be described later, among the respective substituents which can be adopted as R^{a}, and more preferably, a preferred range of the number of carbon atoms is the same as that of the substituent which can be adopted as R^{AA}. The number of carbon atoms is the same as the number of carbon atoms in the respective substituents of the substituent group T^{R} which will be described later, and a preferred range thereof is also the same, with respect to a substituent other than the substituent which can be adopted as R^{AA}, which will be described later, among the respective substituents which can be adopted as R^{a}. This also applies to the respective substituents which can be adopted as R^{a1} or R^{a4}.

As each of R^{a1} and R^{a4}, an alkyl group, a cycloalkyl group, an alkenyl group (preferably an ethenyl group), an alkynyl group (preferably an ethynyl group), an aryl group, a hetero ring group (preferably an aromatic hetero ring group), a halogen atom, an alkoxy group, an alkoxycarbonyl group, a cycloalkoxycarbonyl group, an aryloxy group, an alkylthio group, an arylthio group, an amino group, a cyano group, an alkylsulfonyl group, an arylsulfonyl group, a halogenated alkyl group (for example, a fluoroalkyl group), or a halogenated aryl group is preferable; a halogenated alkyl group, a halogenated aryl group, a halogen atom, a cyano group, an alkylsulfonyl group, or an arylsulfonyl group is more preferable; and a halogenated alkyl group, a halogenated aryl group, a halogen atom, or a cyano group is still more preferable. Further, a group formed by the combination of the respective groups is also preferable. The halogenated alkyl group and the halogenated aryl group will be described later.

It is preferable that R^{a}, R^{a1}, and R^{a4} each have a group R^{VU} represented by the following Formula (V^{U}-1) or (V^{U}-2) as a substituent, and it is particularly preferable that R^{a} has the following group R^{VU}.

In a case where the ring F has R^{a}, the position (substitution position) at which R^{a} is bonded in the ring F is not particularly limited. In a case where the ring F is a 5-membered ring, the 3-position with respect to a ring-constituting nitrogen atom coordinating to the metal atom M is preferable. In a case where the ring F is a 6-membered ring, the 3- or 4-position is preferable, and the 4-position is more preferable, with respect to a ring-constituting nitrogen atom coordinating to the metal atom M.

Furthermore, in a case where the ring D^{DL} and the ring E^{DL} each have R^{a1} or R^{a4}, the position at which R^{a1} or R^{a4}, in each of the ring D^{DL} and the ring E^{DL}, is bonded is not particularly limited.

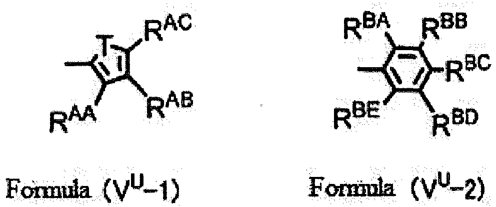

In Formula (V^{U}-1), T represents an oxygen atom, a sulfur atom, -NR^{CA}-, -C(R^{CA})₂-, or -Si(R^{CA})₂-, and R^{CA}'s each represent a hydrogen atom or a substituent. R^{AA} represents a substituent, and R^{AB} and R^{AC} each independently represent a hydrogen atom or a substituent.

In Formula (V^{U}-2), R^{BA} to R^{BE} each independently represent a hydrogen atom or a substituent, and at least one of R^{BA}, R^{BB}, R^{BD}, or R^{BE} represents a substituent.

The number of the groups R^{VU} contained in the ligand LD may be any number of 1 or more, and is preferably 1 to 3, and more preferably 1 or 2.

In Formula (V^{U}-1), T is an oxygen atom, a sulfur atom, -NR^{CA}-, -C(R^{CA})₂-, or -Si(R^{CA})₂-, with a sulfur atom being preferable. Here, R^{CA}'s each represent a hydrogen atom or a substituent, with a hydrogen atom being preferable. Examples of the substituent which can be adopted as R^{CA} include a group selected from the substituent group T^{R} which will be described later.

R^{AA} represents a substituent. The substituent which can be adopted as R^{AA} is not particularly limited, and examples thereof include a group selected from the substituent group T^{R} which will be described later. The substituent is preferably an alkyl group, a cycloalkyl group, an alkoxy group, a cycloalkoxy group, an aryloxy group, an alkylthio group, a cycloalkylthio group, an arylthio group, an amino group, an alkylamino group, a cycloalkylamino group, an arylamino group, a hetero ring amino group, a silyl group, or a silyloxy group.

Among the respective groups, the substituent which can be adopted as R^{AA} is more preferably an alkyl group, a cycloalkyl group, an alkoxy group, a cycloalkoxy group, an alkylthio group, a cycloalkylthio group, an amino group, an alkylamino group, a cycloalkylamino group, or an arylamino group, still more preferably an alkyl group, a cycloalkyl group, an alkoxy group, a cycloalkoxy group, an alkylamino group, a cycloalkylamino group, or an arylamino group, particularly preferably an alkyl group, an alkoxy group, or an alkylamino group, and most preferably an alkyl group or an alkoxy group.

The substituent which can be adopted as R^{AA} is preferably bonded to a thiophene ring (in a case where T is a sulfur atom) in view of photoelectric conversion efficiency.

The substituent which can be adopted as R^{AA} may further be substituted with a group selected from the substituent group T^{R} which will be described later.

The alkyl group encompasses a linear alkyl group and a branched alkyl group. The number of carbon atoms in the alkyl group is preferably 1 to 30, more preferably 4 to 30, still more preferably 5 to 26, and particularly preferably 6 to 20. Examples of the alkyl group include methyl, ethyl, n-butyl, t-butyl, n-pentyl, n-hexyl, n-octyl, 2-ethylhexyl, n-decyl, 3,7-dimethyloctyl, isodecyl, s-decyl, n-dodecyl, 2-butyloctyl, n-hexadecyl, isohexadecyl, n-eicosy, n-hexacosyl, isooctacosyl, trifluoromethyl, and pentafluoroethyl.

The number of carbon atoms in the cycloalkyl group is preferably 3 to 30, more preferably 5 to 30, still more preferably 6 to 26, and particularly preferably 6 to 20. Examples of the cycloalkyl group include cyclopropyl, cyclopentyl, cyclohexyl, cycloheptyl, and cyclooctyl. The cycloalkyl group may also be fused with an aliphatic ring, an aromatic ring, or a hetero ring.

The alkoxy group encompasses a linear alkoxy group and a branched alkoxy group. The alkyl moiety of the alkoxy group has the same definition as the alkyl group, and preferred examples thereof are also the same. Examples of the alkoxy group include methoxy, ethoxy, n-propoxy, i-propoxy, n-butoxy, t-butoxy, n-pentoxy, n-hexyloxy, n-octyloxy, 2-ethylhexyloxy, 3,7-dimethyloctyloxy, n-decyloxy, isodecyloxy, s-decyloxy, 2-butyloctyloxy, n-dodecyloxy, n-hexadecyloxy, isohexadecyloxy, n-eicosyoxy, n-hexacosyloxy, and isooctacosyloxy.

The cycloalkyl moiety of the cycloalkoxy group has the same definition as the cycloalkyl group, and preferred examples thereof are also the same. Examples of the cycloalkoxy group include cyclopropyloxy, cyclopentyloxy, cyclohexyloxy, cycloheptyloxy, and cyclooctyloxy.

The aryloxy group encompasses a carbocyclic aryloxy group in which an aryl group is an aromatic carbon-based ring (aromatic hydrocarbon ring) group, and a heteroaryloxy group in which an aryl group is an aromatic hetero ring group. The number of carbon atoms in the aryloxy group is preferably 3 to 30, more preferably 3 to 25, still more preferably 3 to 20, and particularly preferably 3 to 16. Examples of the aryloxy group include phenoxy, naphthoxy, imidazoyloxy, benzimidazoyloxy, pyridin-4-yloxy, pyrimidinyloxy, quinazolinyloxy, purinyloxy, and thiophen-3-yloxy. As the hetero ring of the heteroaryloxy group, a thiophene ring is preferable.

The alkylthio group encompasses a linear alkylthio group and a branched alkylthio group. The alkyl moiety of the alkylthio group has the same definition as the alkyl group, and preferred examples thereof are also the same. Examples of the alkylthio group include methylthio, ethylthio, n-propylthio, i-propylthio, n-butylthio, t-butylthio, n-pentylthio, n-hexylthio, n-octylthio, 2-ethylhexylthio, 3,7-dimethyloctylthio, n-decylthio, isodecylthio, s-decylthio, n-dodecylthio, 2-butyloctylthio, n-hexadecylthio, isohexadecylthio, n-eicosythio, n-hexacosylthio, and isooctacosylthio.

The cycloalkyl moiety of the cycloalkylthio group has the same definition as the cycloalkyl group, and preferred examples thereof are also the same. Examples of the cycloalkylthio group include cyclopropylthio, cyclopentylthio, cyclohexylthio, cycloheptylthio, and cyclooctylthio.

The arylthio group encompasses a carbocyclic arylthio group in which an aryl group is an aromatic carbon-based ring, and a heteroarylthio group in which an aryl group is an aromatic hetero ring. The number of carbon atoms in the arylthio group is preferably 3 to 30, more preferably 3 to 25, still more preferably 3 to 20, and particularly preferably 3 to 16. Examples of the arylthio group include phenylthio, naphthylthio, imidazoylthio, benzimidazoylthio, pyridin-4-ylthio, pyrimidinylthio, quinazolinylthio, purinylthio, and thiophen-3-ylthio. As the hetero ring of the heteroarylthio group, a thiophene ring is preferable.

The alkylamino group encompasses an N-alkylamino group and an N,N-dialkylamino group, and the number of carbon atoms in the alkyl group is preferably 1 to 30, and more preferably 2 to 30. Examples of the alkylamino group include ethylamino, diethylamino, 2-ethylhexylamino, bis(2-ethylhexyl)amino, and n-octadecylamino.

The cycloalkylamino group encompasses an N-cycloalkylamino group and an N,N-dicycloalkylamino group. The cycloalkyl moiety of the cycloalkylamino group has the same definition as the cycloalkyl group, and preferred examples thereof are also the same. Examples of the cycloalkylamino group include cyclopropylamino, dicyclopropylamino, N-cyclopropyl-N-ethylamino, cyclopentylamino, dicyclopentylamino, N-cyclopentyl-N-methylamino, cyclohexylamino, dicyclohexylamino, cycloheptylamino, and cyclooctylamino.

The arylamino group encompasses a carbocyclic arylamino group in which an aryl group is an aromatic carbon-based ring, and a heteroarylamino group in which an aryl group is an aromatic hetero ring. Further, the carbocyclic arylamino group encompasses an N-arylamino group, an N-alkyl-N-arylamino group, and an N,N-diarylamino group. The heteroarylamino group encompasses an N-heteroarylamino group, an N-alkyl-N-heteroarylamino group, an N-aryl-N-heteroarylamino group, and an N,N-diheteroarylamino group.

The number of carbon atoms in the arylamino group is preferably 3 to 30, more preferably 3 to 25, still more preferably 3 to 20, and particularly preferably 3 to 16. Examples of the arylamino group include phenylamino, N-phenyl-N-ethylamino, naphthylamino, imidazoylamino, benzimidazoylamino, pyridin-4-ylamino, pyrimidinylamino, quinazolinylamino, purinylamino, and thiophen-3-ylamino.

The heterocyclic amino group is a heterocyclic amino group (aliphatic heterocyclic amino group) other than a heteroarylamino group. The number of carbon atoms is preferably 0 to 30, more preferably 1 to 25, still more preferably 2 to 20, and particularly preferably 2 to 16. Further, in the hetero ring, the ring-constituting heteroatom is preferably selected from an oxygen atom, a sulfur atom, and a nitrogen atom, and in terms of the number of ring members, 5- to 7-membered rings are preferable, and 5- or 6-membered rings are more preferable. Examples of the heterocyclic amino group include pyrrolidin-3-ylamino, imidazolidinylamino, benzimidazolidinylamino, piperidin-4-ylamino, and tetrahydrothiophen-3-ylamino.

The silyl group encompasses an alkylsilyl group, a cycloalkylsilyl group, an arylsilyl group, an alkyloxysilyl group, a cycloalkyloxysilyl group, and an aryloxysilyl group. The silyl group is preferably an alkylsilyl group, a cycloalkylsilyl group, or an arylsilyl group. The number of carbon atoms in the silyl group is preferably 3 to 30, more preferably 3 to 24, still more preferably 3 to 20, and particularly preferably 3 to 18. Examples of the silyl group include trimethylsilyl, triethylsilyl, t-butyldimethylsilyl, cyclohexyldimethylsilyl, triisopropylsilyl, t-butyldiphenylsilyl, methyldimethoxysilyl, phenyldimethoxysilyl, and phenoxydimethylsilyl.

The silyloxy group encompasses an alkylsilyloxy group, a cycloalkylsilyloxy group, and an arylsilyloxy group. The number of carbon atoms in the silyloxy group is preferably 3 to 30, more preferably 3 to 24, still more preferably 3 to 20, and particularly preferably 3 to 18. Examples of the silyloxy group include trimethylsilyloxy, triethylsilyloxy, t-butyldimethylsilyloxy, triisopropylsilyloxy, cyclohexyldimethylsilyloxy, and t-butyldiphenylsilyloxy.

R^{AB} represents a hydrogen atom or a substituent, with a hydrogen atom being preferable.

R^{AC} represents a hydrogen atom or a substituent

The substituent which can be adopted as R^{AB} and R^{AC} has the same definition as R^{AA}, and preferred examples thereof are also the same. In a case where R^{AB} or R^{AC} is a substituent, the respective substituents of R^{AA} to R^{AC} may be the same as or different from each other.

In the group R^{VU} represented by Formula (V^{U}-2), R^{BA} to R^{BE} each independently represent a hydrogen atom or a substituent. The substituent which can be adopted as each of R^{BA} to R^{BE} has the same definition as R^{AA}, and preferred examples thereof are also the same.

Here, at least one of R^{BA}, R^{BB}, R^{BD}, or R^{BE} is a substituent. It is particularly preferable that at least one or both of R^{BA} and R^{BE} are a substituent, and all of R^{BB}, R^{BC}, and R^{BD} are a hydrogen atom; or at least one or both of R^{BB} and R^{BD} are a substituent, and all of R^{BA}, R^{BC}, and R^{BE} are a hydrogen atom.

In a case where two or more members out of R^{BA} to R^{BE} are substituents, two or more substituents may be the same as or different from each other.

In Formula (DL), ma, ma1, and ma4 are each preferably an integer of 0 to 2, and more preferably 1 or 2.

The ligand represented by Formula (DL) is preferably represented by the following Formula (DL-1) or (DL-2).

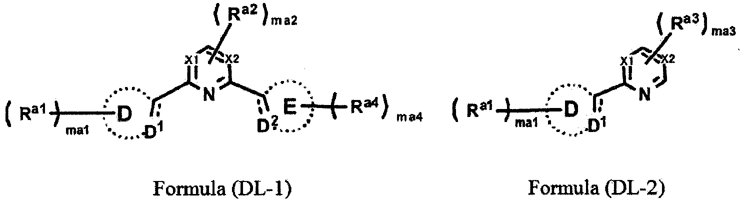

R^{a2} and R^{a3} each independently represent a substituent not having an acidic group. ma2 represents 0 or 1, with 1 being preferable. ma3 represents an integer of 0 to 2, with 1 or 2 being more preferable.

X1 and X2 each independently represent CR^{a5} or a nitrogen atom. R^{a5} represents a hydrogen atom or a substituent. This substituent has the same definition as R^{a} in Formula (DL), and a preferred range thereof is also the same. A ring including X1 and X2 (also referred to as a ring F) has the same definition as the ring F in Formula (DL), and a preferred range thereof is also the same.

R^{a1}, R^{a4}, ma1, and ma4 have the same definitions as R^{a1}, R^{a4}, ma1, and ma4, respectively, in Formula (DL), and preferred ranges thereof are also the same.

The substituents represented by R^{a2} and R^{a3} have the same definitions as R^{a} in Formula (DL), and preferred ranges thereof are also the same.

When ma1, ma3, and ma4 are each an integer of 2 or more, a plurality of R^{a1}'s, R^{a3}'s, and R^{a4}'s each may be the same as or different from each other, and may be bonded to each other to form a ring.

The ring D and the ring E each independently represent an aromatic ring as a 5- or 6-membered ring. Examples of such an aromatic ring include the rings mentioned as the ring D^{DL} and the ring E^{DL} in Formula (DL), and preferred aromatic rings are also the same as the rings mentioned as the ring D^{DL} and the ring E^{DL}.

Furthermore, the bond between D¹ and D² in the ring D and the ring E, and a carbon atom bonded to the ring F may be a single bond or a double bond.

D¹ and D² each independently represent an anion of a carbon atom or an anion of a nitrogen atom.

The ring D and the ring E are each preferably a pyrrole ring, an imidazole ring, a pyrazole ring, a triazole ring, or a benzene ring, with a pyrazole ring, a triazole ring, or a benzene ring being more preferable.

In a case where the ligand LD is a bidentate ligand, a bidentate ligand represented by any one of the following Formulae (2L-1) to (2L-4) is preferable.

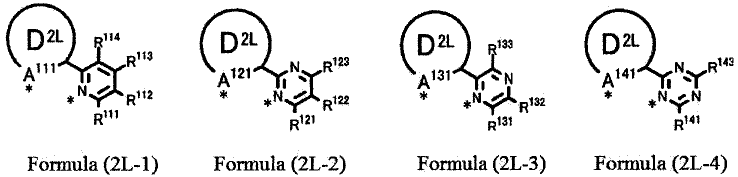

In the formula, * represents a coordinating position (binding position) to a metal ion M. The ring D^{2L} represents an aromatic ring. A¹¹¹ to A¹⁴¹ each independently represent an anion of a nitrogen atom or an anion of a carbon atom. R¹¹¹ to R¹⁴³ each independently represent a hydrogen atom or a substituent not having an acidic group.

Here, A¹¹¹ to A¹⁴¹ are each an anion of a carbon atom or an anion of a nitrogen atom, in which a hydrogen atom bonded to a nitrogen atom or a carbon atom constituting the ring D^{2L} is dissociated. In Formulae (2L-1) to (2L-4), examples of the ring D^{2L} include an aromatic hydrocarbon ring, an oxygen-containing aromatic hetero ring, a sulfur-containing aromatic hetero ring, and a nitrogen-containing aromatic hetero ring.

Examples of the aromatic hydrocarbon ring include a benzene ring and a naphthalene ring, among which a benzene ring is preferable, and a benzene ring substituted with a halogen atom, a halogenated alkyl group, or a halogenated aryl group is more preferable. The halogenated alkyl group is an alkyl group substituted with a halogen atom, with a fluorinated alkyl group (for example, a trifluoromethyl group) being preferable. As the halogenated aryl group, a phenyl group substituted with 1 to 5 halogen atoms is preferable.

As the oxygen-containing aromatic hetero ring, a furan ring is preferable, and as the sulfur-containing aromatic hetero ring, a thiophene ring is preferable. As the nitrogen-containing aromatic hetero ring, a pyrrole ring, a pyrazole ring, an imidazole ring, or a triazole ring is preferable.

Preferred examples of the ring D^{2L} include the respective rings in which one of ring-constituting atoms of a benzene ring, a thiophene ring, or a furan ring becomes an anion, or the respective rings represented by the following Formulae (a-1) to (a-5), (a-la), (a-2a), (a-lb), and (a-4a).

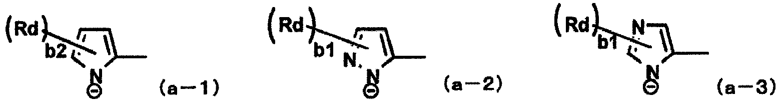

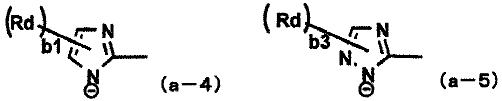

In the formula, Rd represents a substituent not having an acidic group. b1 represents an integer of 0 to 2, b2 represents an integer of 0 to 3, and b3 represents 0 or 1. When b1 is 2 or when b2 is 2 or more, the plurality of Rd' s may be the same as or different from each other. Further, a plurality of Rd's may be bonded to each other to form a ring. Examples of Rd include a group selected from the substituent group T^{R} which will be described later.

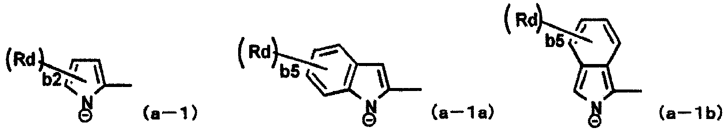

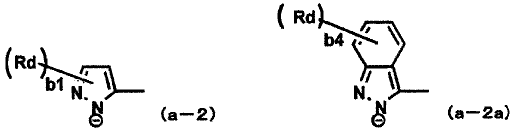

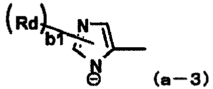

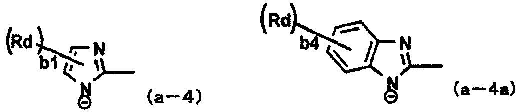

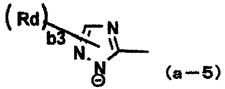

In the formulae, Rd, and b1 to b3 have the same definitions as Rd, and b1 to b3, respectively, in Formula (a-1) to (a-5), and preferred ranges thereof are also the same. b4 represents an integer of 0 to 4, and b5 represents an integer of 0 to 5. In Formulae (a-1a) and (a-1b), Rd also represents the group which may also be contained in a pyrrole ring, in addition to a benzene ring.

Rd is preferably a linear or branched alkyl group, a cycloalkyl group, an alkenyl group, a fluoroalkyl group, an aryl group, a halogen atom, an alkoxycarbonyl group, a cycloalkoxycarbonyl group, a cyano group, an alkylsulfonyl group, an arylsulfonyl group, or a group formed by the combination of these groups, more preferably a linear or branched alkyl group, a cycloalkyl group, an alkenylnyl group, an aryl group, or a group formed by the combination of these groups, and still more preferably a linear or branched halogenated alkyl group or a halogenated aryl group.

The substituents represented by R¹¹¹ to R¹⁴³ have the same definitions as R^{a} in Formula (DL), and preferred ranges thereof are also the same.

Preferably at least one, and more preferably one or two of R¹¹¹ to R¹¹⁴, R¹²¹ to R¹²³, R¹³¹ to R¹³³, and R¹⁴¹ to R¹⁴³, respectively, are substituents.

In a case where the ligand LD is tridentate ligand, it is preferably a tridentate ligand represented by any one of the following Formulae (3L-1) to (3L-4).

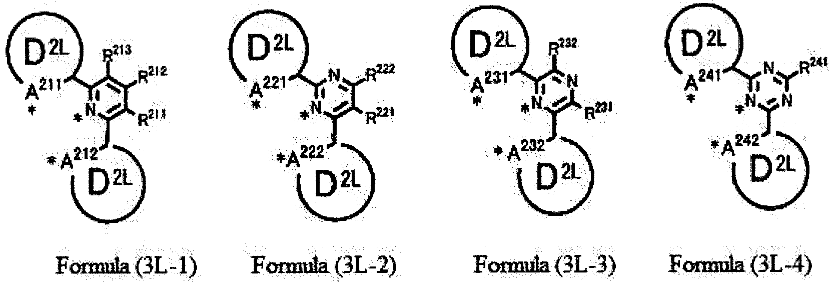

In the formulae, * represents a coordinating position (binding position) to a metal ion M. The ring D^{2L} represents an aromatic ring. A²¹¹ to A²⁴² each independently represent a nitrogen atom or a carbon atom, in which at least one of A²¹¹ and A²¹², A²²¹ and A²²², A²³¹ and A²³², and A²⁴¹ and A²⁴², respectively, is an anion. R²¹¹ to R²⁴¹ each independently represent a hydrogen atom, or a substituent not having an acidic group.

A²¹¹ to A²⁴² which are anions have the same definitions as A¹¹¹ to A¹⁴¹, respectively, in Formulae (2L-1) to (2L-4). A²¹¹ to A²⁴², which do not have an anion, are each a nitrogen atom not having a hydrogen atom.

The ring D^{2L} in Formulae (3L-1) to (3L-4) has the same definition as the ring D^{2L} in Formulae (2L-1) to (2L-4), and a preferred range thereof is also the same. The ring D^{2L} is more preferably an aromatic ring including any one of A²¹¹ to A²⁴² and a carbon atom or an aromatic ring including two carbon atoms. Here, two ring D^{2L}'s in the respective formulae may be the same as or different from each other.

The substituents R²¹¹ to R²⁴¹ have the same definitions as R^{a} in Formula (DL), and preferred examples thereof are also the same.

In the present invention, the bidentate or tridentate ligand in the ligand LD, in which the atom coordinating to the metal ion M is a nitrogen anion or a carbon anion, and an arylamino group or a diarylamino group is contained in the substituent, is preferable, in particular, due to absorption at a longer wavelength.

Specifically, the preferred ligand is the ligand in which at least one of the atoms coordinating to the metal ion M is a nitrogen anion or a carbon anion, and the ligand has the following Formula (SA) as a partial structure.

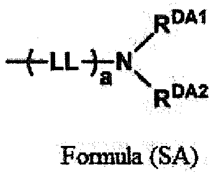

In the formula, R^{DA1} represents an aryl group (aromatic hydrocarbon ring group) or a heteroaryl group (aromatic hetero ring group), and R^{DA2} represents an alkyl group, an aryl group, or an aromatic hetero ring group. R^{DA1} and R^{DA2} may be bonded to each other to form a ring. LL represents an ethenyl group, an ethynyl group, an arylene group, or a heteroarylene group. a represents an integer of 0 to 5, and when a is 2 or more, LL's present in plural numbers may be the same as or different from each other.

The group represented by Formula (SA) is preferably substituted with an aromatic hydrocarbon ring coordinating to the metal ion M or a nitrogen-containing aromatic hetero ring, and more preferably substituted with a nitrogen atom-containing aromatic hetero ring.

In the group represented by Formula (SA), it is preferable that at least one of R^{DA1} or R^{DA2} is an aryl group or a heteroaryl group, and it is more preferable that both of R^{DA1} and R^{DA2} are aryl groups. The aryl group or the heteroaryl group may have a substituent, and examples of such a substituent include a group selected from the substituent group T^{R} which will be described later.

The aryl group is not particularly limited, and examples thereof include a phenyl group and a naphthyl group, with a phenyl group being preferable. The heteroaryl group is not particularly limited, but is preferably a furanyl group or a thienyl group.

LL may form a fused structure together with an aromatic hydrocarbon ring including a coordinating atom of the ligand or a nitrogen-containing aromatic hetero ring. For example, LL may be an ethenyl group, and this ethenyl group may be bonded to a nitrogen-containing aromatic hetero ring including a coordinating atom of the ligand to form a quinoline ring.

Examples of the arylene group in LL include a phenylene group and a naphthylene group, and the heteroarylene group is preferably a divalent 5- or 6-membered ring which contains an oxygen atom, a sulfur atom, or a nitrogen atom as a ring-constituting atom, and may be fused with a benzene ring or a hetero ring.

Examples of the hetero ring of the heteroarylene group include a furan ring, a thiophene ring, a pyrrole ring, and a pyridine ring, with a furan ring or a thiophene ring being preferable.

The ethenyl group, the arylene group, or the heteroarylene group in LL may have a substituent, and examples of the substituent include a group selected from the substituent group T^{R} which will be described later.

In Formula (SA), it is preferable that a is 0, or that a is 1 and LL is an ethenyl group, an ethynyl group, a phenylene group, or a heteroarylene group; it is more preferable that a is 0, or that a is 1 and LL is a phenylene group or a heteroarylene group; it is still more preferable that a is 0, or that a is 1 and LL is a phenylene group, a divalent furan ring group, or a divalent thiophene ring group; and it is particularly preferable that a is 0.

In the present invention, it is also preferable that R^{DA1} and R^{DA2} are bonded to each other to form a ring.

The ring thus formed is preferably a 5- or 6-membered ring, and more preferably a ring formed by the bonding of R^{DA1} and R^{DA2} which are both an aryl group.

The rings formed by the mutual bonding of R^{DA1} and R^{DA2} are preferably the following rings.

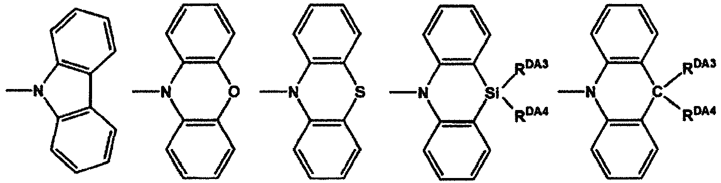

Here, R^{DA3} and R^{DA4} each independently represent an alkyl group.

The ring may have a substituent, and examples of such a substituent include a group selected from the substituent group T^{R} which will be described later.

The ligand represented by Formula (DL) can be synthesized by, for example, the method described in US2010/0258175A1, JP4298799B, or Angew. Chem. Int. Ed., 2011, 50, pp. 2054-2058, the methods described in the reference documents listed in these documents, or the methods equivalent thereto.

Specific examples of the ligand represented by Formula (DL) are shown below. Further, examples of this ligand LD also include the ligand LD in the metal complex dye which will be described later. The present invention is not limited to these ligands LD. In the following specific examples, Me represents methyl, and * represents a binding position at which rings are bonded to each other, or a pyridine ring and the substituent R²⁰¹ are bonded to each other.

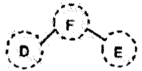

| LD No. | Ring D | Ring F | Ring E |
|---|---|---|---|
| LD-3-1 | 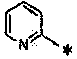 | 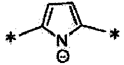 | 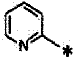 |
| LD-3-2 | 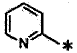 | 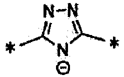 | 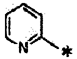 |
| LD-3-3 | 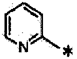 | 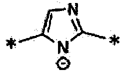 | 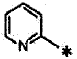 |
| LD-3-4 | 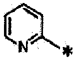 | 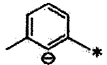 | 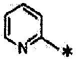 |
| LD-3-5 | 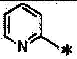 | 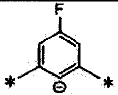 | 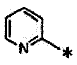 |
| LD-3-6 | 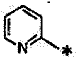 | 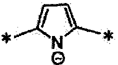 | 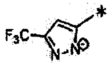 |
| LD-3-7 | 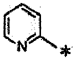 | 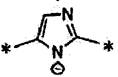 | 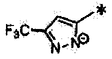 |
| LD-3⁻8 | 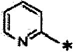 | 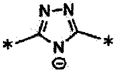 | 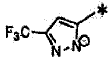 |
| LD-3-9 | 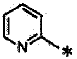 | 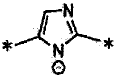 | 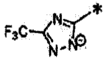 |
| LD-3-10 | 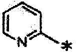 | 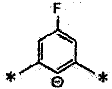 | 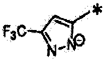 |
| LD-3-11 | 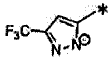 | 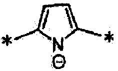 | 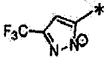 |
| LD-3-12 | 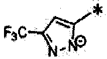 | 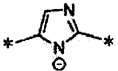 | 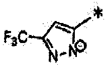 |
| LD-3-13 | 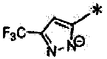 | 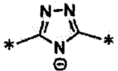 | 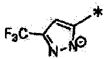 |
| LD-3-14 | 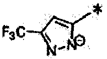 | 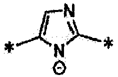 | 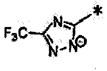 |
| LD-3-15 | 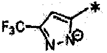 | 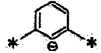 | 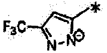 |
| LD-3-16 | 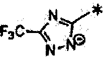 | 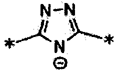 | 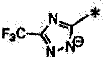 |

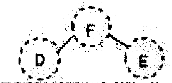

| LD No. | Ring D | Ring F | Ring E |
|---|---|---|---|
| LD-3-17 | 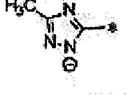 | 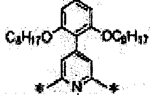 | 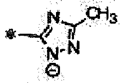 |
| LD-3-18 | 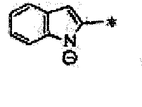 | 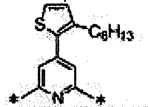 | 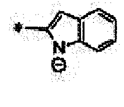 |
| LD-3-19 | 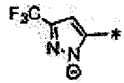 | 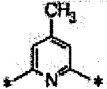 | 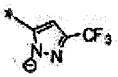 |
| LD-3-20 | 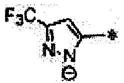 | 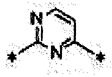 | 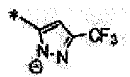 |
| LD-3-21 | 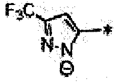 | 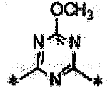 | 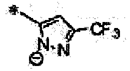 |
| LD-3-22 | 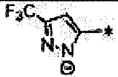 | 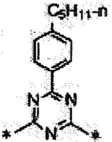 | 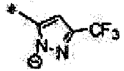 |
| LD-3-23 | 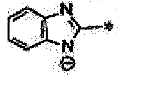 | 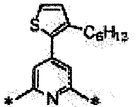 | 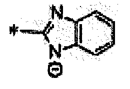 |
| LD-3-24 | 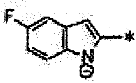 | 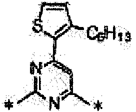 | 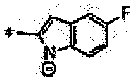 |
| LD-3-25 | 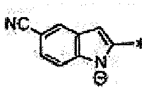 | 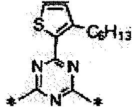 | 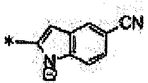 |

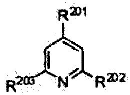

| LD No | R203 | R201 | R202 |
|---|---|---|---|
| LD-2-1 | 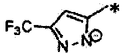 | H | 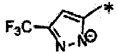 |
| LD-2-2 | 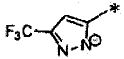 | 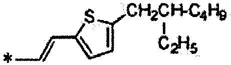 | 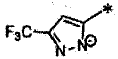 |
| LD-2-3 | 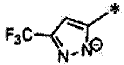 | 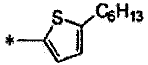 | 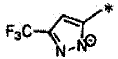 |
| LD-2-4 | 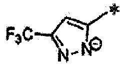 | 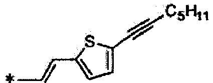 | 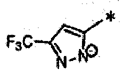 |
| LD-2-5 | 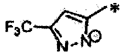 | 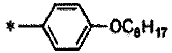 | 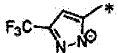 |
| LD-2-6 | 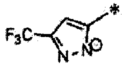 | 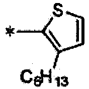 | 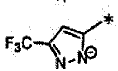 |
| LD-2-7 | 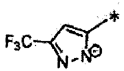 | 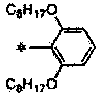 | 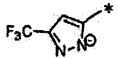 |
| LD-2-8 | 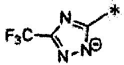 | 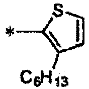 | 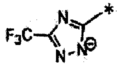 |
| LD-2-9 | 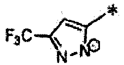 | 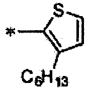 | 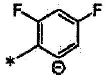 |
| LD-2-10 | 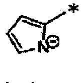 | 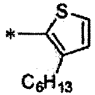 | 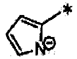 |
| LD-2-11 | 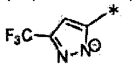 | 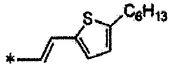 | 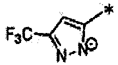 |
| LD-2-12 | 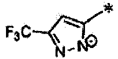 | 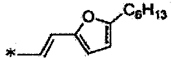 | 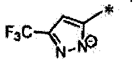 |

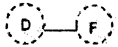

| LD No | Ring D | Ring F |
|---|---|---|
| LD-6-1 | 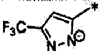 | 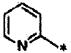 |
| LD-6-2 | 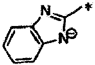 | 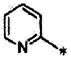 |
| LD-6-3 | 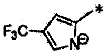 | 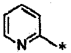 |
| LD-6-4 | 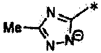 | 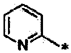 |
| LD-6-5 | 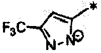 | 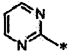 |
| LD-6-6 | 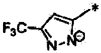 | 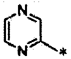 |
| LD-6-7 | 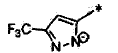 | 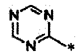 |
| LD-6-8 | 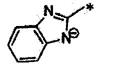 | 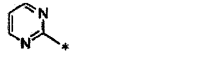 |
| LD-6-9 | 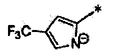 | 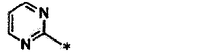 |
| LD-6-10 | 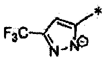 | 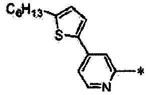 |
| LD-6-11 | 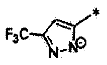 | 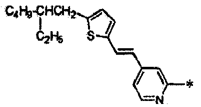 |
| LD-6-12 | 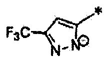 | 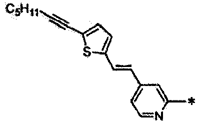 |
| L0-6-13 | 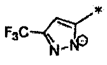 | 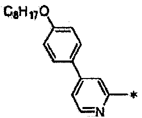 |
| LD-6-14 | 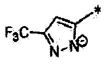 | 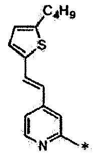 |
| LD-6-15 | 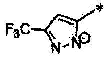 | 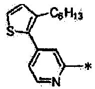 |
| LD-6-16 | 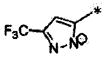 | 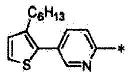 |

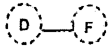

| LD No | Ring D | RingF |
|---|---|---|
| LD-6-17 | 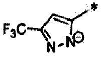 | 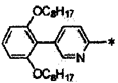 |
| LD-6-18 | 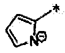 | 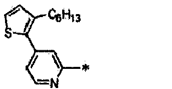 |
| LD-6-19 | 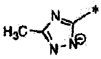 | 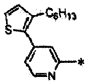 |
| LD-6-20 | 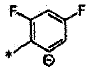 | 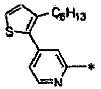 |
| LD-6-21 | 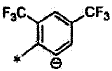 | 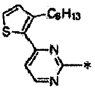 |
| LD-6-22 | 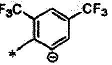 | 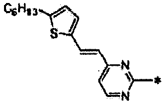 |
| LD-6-23 | 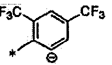 | 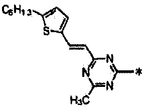 |
| LD-6-24 | 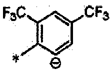 | 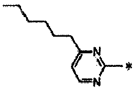 |
| LD-6-25 | 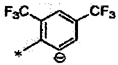 | 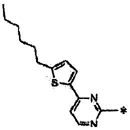 |
| LD-6-26 | 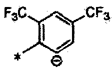 | 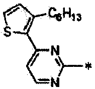 |
| LD-6-27 | 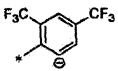 | 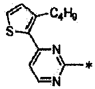 |
| LD-6-28 | 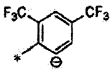 | 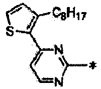 |
| LD-6-29 | 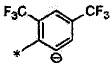 | 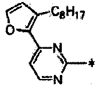 |

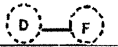

| LD No | Ring D | Ring F |
|---|---|---|
| LD-6-30 | 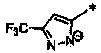 | 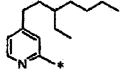 |
| LD-6-31 | 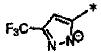 | 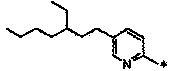 |
| LD-6-32 | 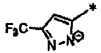 | 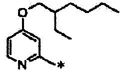 |
| LD-8-33 | 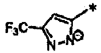 | 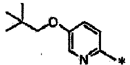 |
| LD-6-34 | 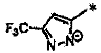 | 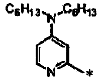 |
| LD-6-35 | 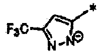 | 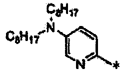 |
| LD-6-36 | 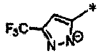 | 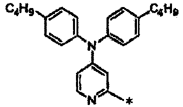 |
| LD-6-37 | 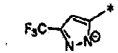 | 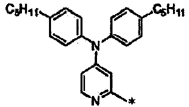 |
| LD-6-38 | 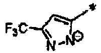 | 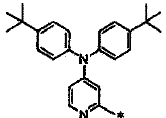 |
| LD-6-39 | 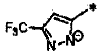 | 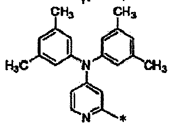 |
| LD-6-40 | 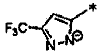 | 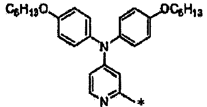 |
| LD-6-41 | 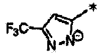 | 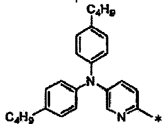 |

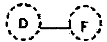

| LD No | Ring D | Ring F |
|---|---|---|
| LD-6-42 | 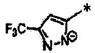 | 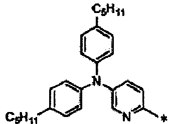 |
| LD-6-43 | 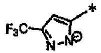 | 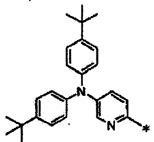 |
| LD-6-44 | 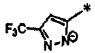 | 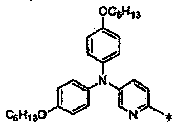 |
| LD-6-45 | 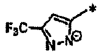 | 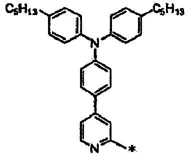 |
| LD-6-46 | 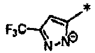 | 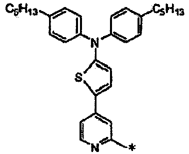 |
| LD-6-47 | 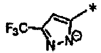 | 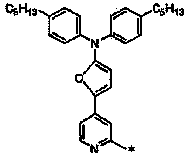 |
| LD-8-48 | 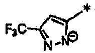 | 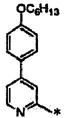 |
| LD-6-49 | 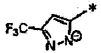 | 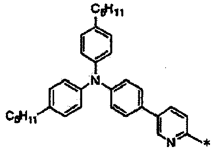 |

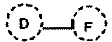

| LD No | Ring D | Ring F |
|---|---|---|
| LD-6-50 | 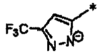 | 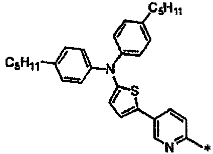 |
| LD-6-51 | 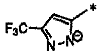 | 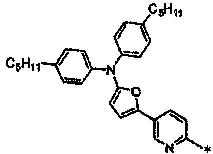 |
| LD-6-52 | 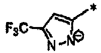 | 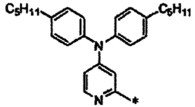 |
| LD-6-53 | 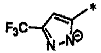 | 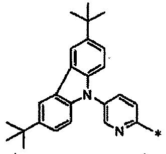 |
| LD-6-54 | 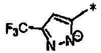 | 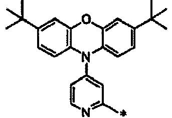 |
| LD-6-55 | 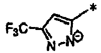 | 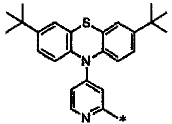 |
| LD-6-56 | 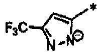 | 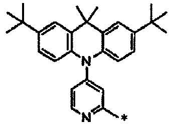 |
| LD-6-67 | 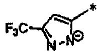 | 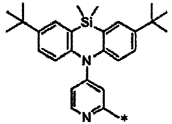 |

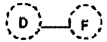

| LD No | Ring D | Ring F |
|---|---|---|
| LD-6-58 | 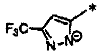 | 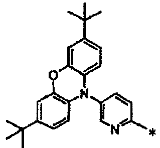 |
| LD-6-59 | 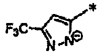 | 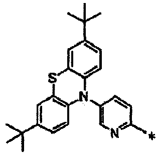 |
| LD-6-60 | 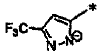 | 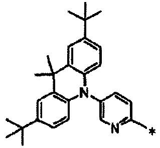 |
| LD-6-61 | 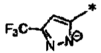 | 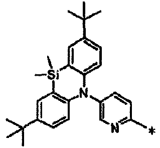 |
| LD-6-62 | 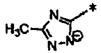 | 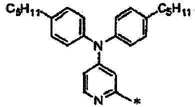 |
| LD-6-63 | 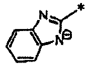 | 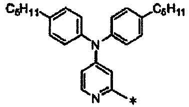 |
| LD-6-64 | 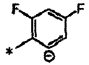 | 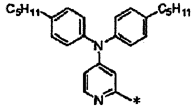 |
| LD-6-65 | 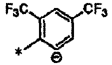 | 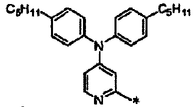 |
| LD-4-66 | 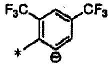 | 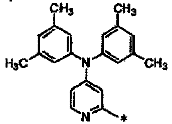 |

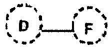

| LD No | Ring D | Ring F |
|---|---|---|
| LD-8-67 | 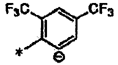 | 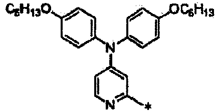 |
| LD-6-68 | 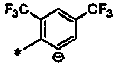 | 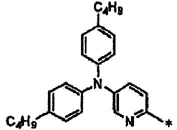 |
| LD-6-49 | 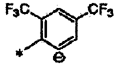 | 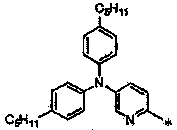 |
| LD-6-70 | 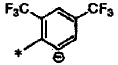 | 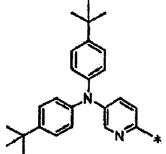 |
| LD-6-71 | 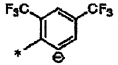 | 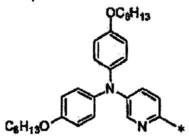 |
| LD-6-72 | 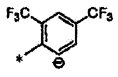 | 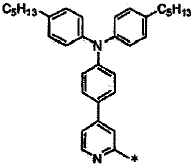 |
| LD-6-73 | 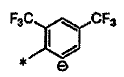 | 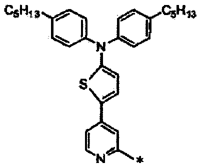 |
| LD-6-74 | 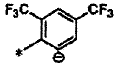 | 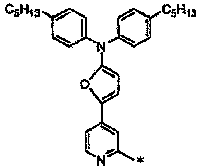 |

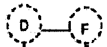

| LD No | Ring D | Ring F |
|---|---|---|
| LD-6-75 | 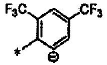 | 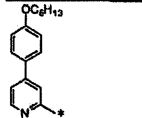 |
| LD-6-76 | 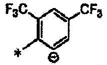 | 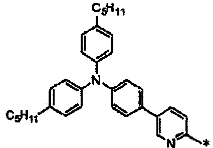 |
| LD-6-71 | 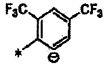 | 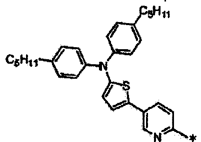 |
| LD-6-78 | 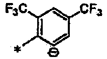 | 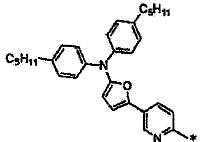 |
| LD-6-79 | 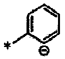 | 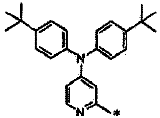 |
| LD-4-80 | 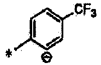 | 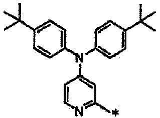 |
| LD-6-81 | 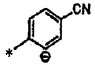 | 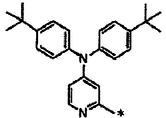 |
| LD-6-82 | 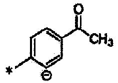 | 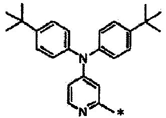 |
| LD-6-83 | 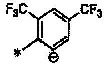 | 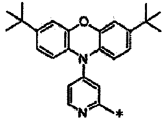 |

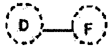

| LD No | Ring D | Ring F |
|---|---|---|
| LD-6-84 | 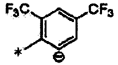 | 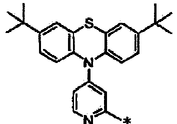 |
| LD-6-85 | 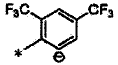 | 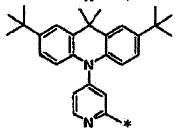 |
| LD-6-86 | 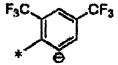 | 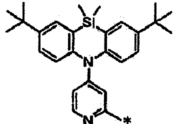 |
| LD-6-67 | 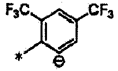 | 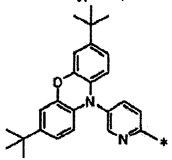 |
| LD-6-88 | 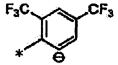 | 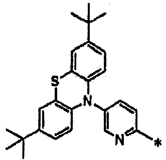 |
| LD-6-69 | 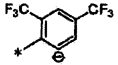 | 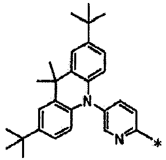 |
| LD-6-90 | 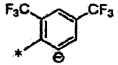 | 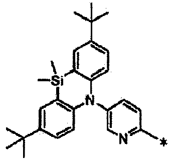 |
| LD-6-91 | 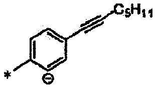 | 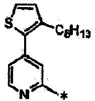 |
| LD-6-92 | 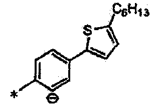 | 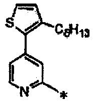 |

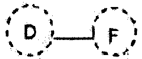

| LD No | Ring D | Ring F |
|---|---|---|
| LD-6-93 | 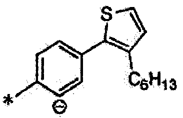 | 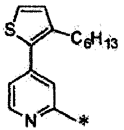 |
| LD-6-94 | 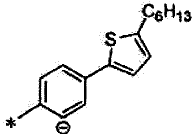 | 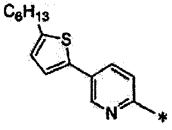 |
| LD-6-95 | 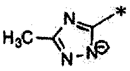 | 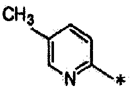 |
| LD-6-96 | 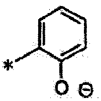 | 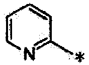 |
| LD-6-97 | 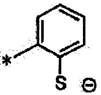 | 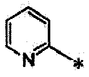 |
| LD-6-98 | 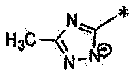 | 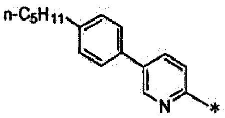 |

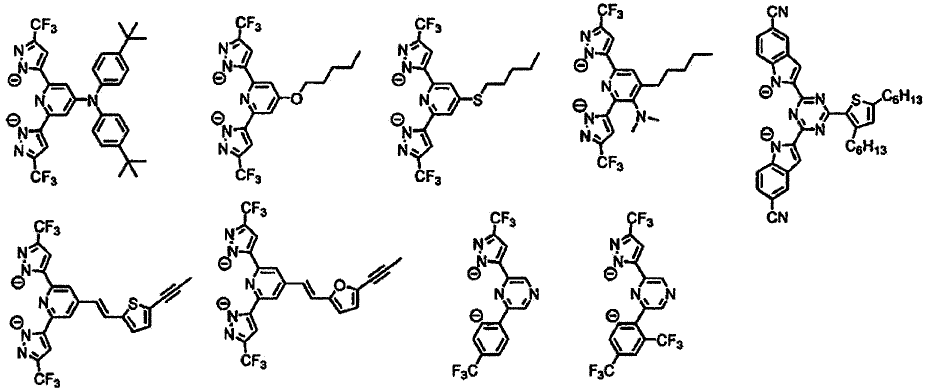

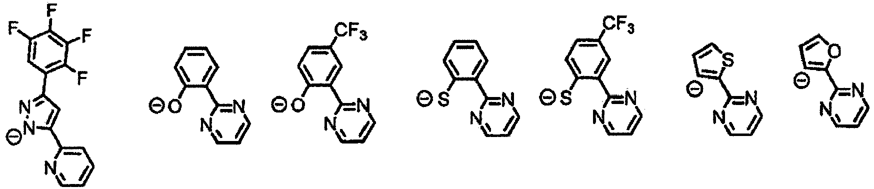

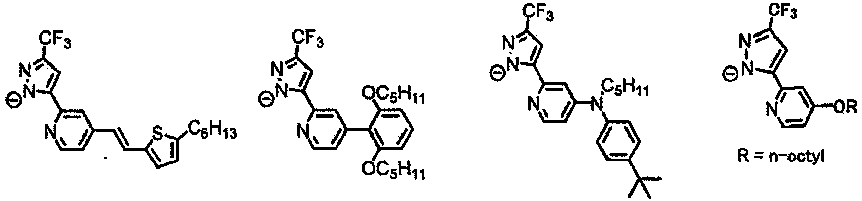

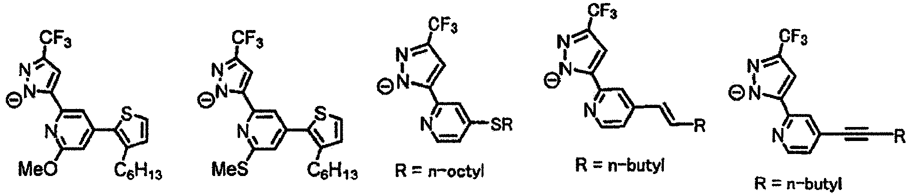

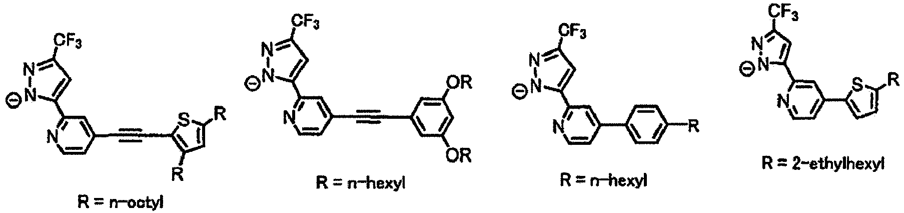

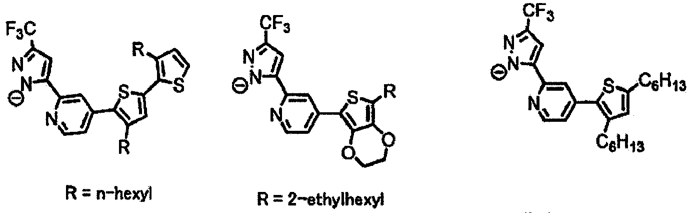

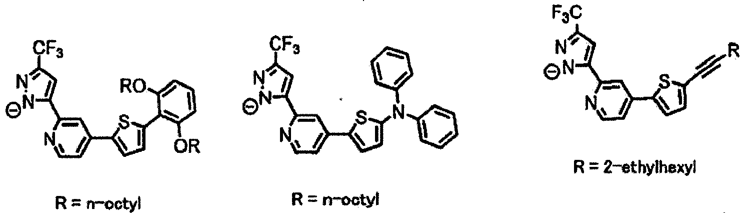

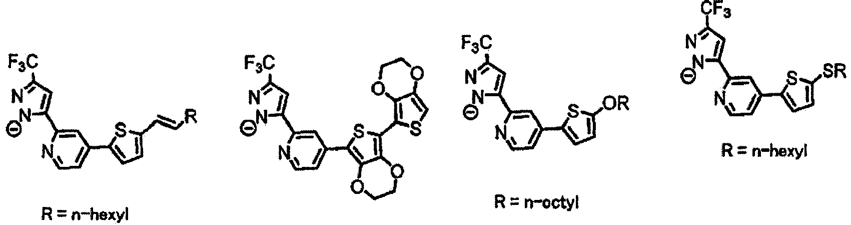

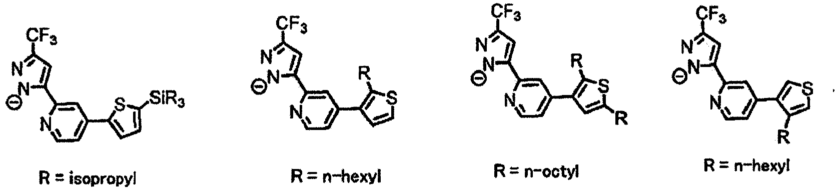

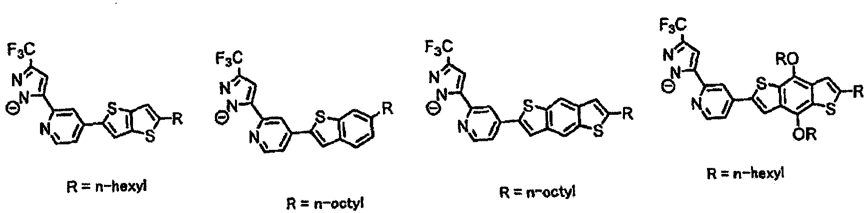

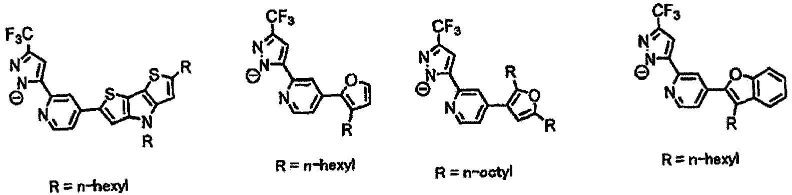

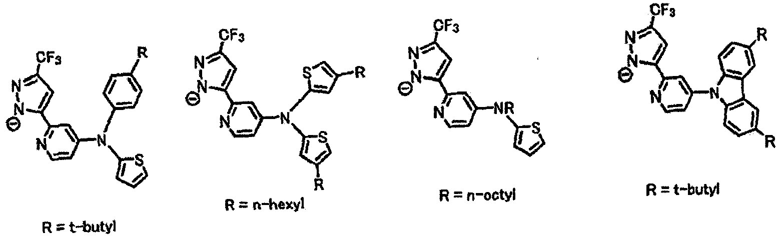

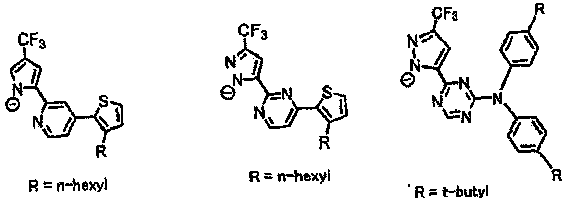

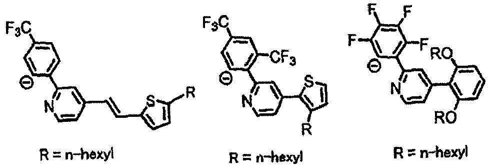

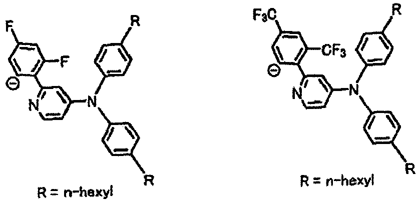

### - Ligand LX -

The ligand LX may be a monodentate ligand, and is preferably, for example, a group or atom selected from the group consisting of an acyloxy group, an acylthio group, a thioacyloxy group, a thioacylthio group, an acylaminooxy group, a thiocarbamate group, a dithiocarbamate group, a thiocarbonate group, a dithiocarbonate group, a trithiocarbonate group, an acyl group, a thiocyanate group, an isothiocyanate group, a cyanate group, an isocyanate group, a cyano group, an alkylthio group, an arylthio group, an alkoxy group, an aryloxy group, and a halogen atom, or anions thereof.

In a case where the ligand LX includes an alkyl group, an alkenyl group, an alkynyl group, an alkylene group, or the like, these groups may or may not have a substituent. Further, in a case where an aryl group, a hetero ring group, a cycloalkyl group, or the like is included, these may or may not have a substituent, and may be a monocycle or a fused ring.

Among those, the ligand LX is preferably a cyanate group, an isocyanate group, a thiocyanate group, or an isothiocyanate group, or an anion thereof, more preferably an isocyanate group (an isocyanate anion) or an isothiocyanate group (an isothiocyanate anion), and particularly preferably an isothiocyanate group (an isothiocyanate anion).

### - Counterion CI for Neutralizing Charge -

CI represents a counterion when the counterion is necessary for neutralizing the charge of the metal complex dye. Generally, whether the metal complex dye is cationic or anionic, or whether the metal complex dye has a net ionic charge depends on the metal, the ligand, and the substituent in the metal complex dye.

When the substituent has a dissociative group or the like, the metal complex dye may have a negative charge arising from dissociation. In this case, an electric charge of the metal complex dye as a whole is electrically neutralized by CI.

In a case where the counterion CI is a positive counterion, the counterion CI is, for example, an inorganic or organic ammonium ion (for example, a tetraalkyl ammonium ion and a pyridinium ion), a phosphonium ion (for example, a tetraalkylphosphonium ion and an alkyltriphenylphosphonium ion), an alkali metal ion (a Li ion, a Na ion, a K ion, and the like), an alkaline earth metal ion, a metal complex ion, or a proton. As the positive counterion, an inorganic or organic ammonium ion (a tetraethylammonium ion, a tetrabutylammonium ion, a tetrahexylammonium ion, a tetraoctylammonium ion, a tetradecylammonium ion, and the like), an alkali metal ion, and a proton are preferable.

In a case where the counterion CI is a negative counterion, the counterion CI is, for example, an inorganic anion or an organic anion. Examples thereof include a hydroxide ion, a halogen anion (for example, a fluoride ion, a chloride ion, a bromide ion, and an iodide ion), a substituted or unsubstituted alkylcarboxylate ion (for example, an acetate ion and a trifluoroacetate ion), a substituted or unsubstituted arylcarboxylate ion (for example, a benzoate ion), a substituted or unsubstituted alkylsulfonate ion (for example, a methanesulfonate ion and a trifluoromethanesulfonate ion), a substituted or unsubstituted arylsulfonate ion (for example, a p-toluene sulfonate ion and a p-chlorobenzene sulfonate ion), an aryldisulfonate ion (for example, a 1,3-benzene disulfonate ion, a 1,5-naphthalene disulfonate ion, and a 2,6-naphthalene disulfonate ion), an alkylsulfate ion (for example, a methylsulfate ion), a sulfate ion, a thiocyanate ion, a perchlorate ion, a tetrafluoroborate ion, a hexafluorophosphate ion, and a picrate ion. Alternatively, as a charge balance counterion, an ionic polymer or another dye with an opposite charge from the dye in interest may be used, or a metal complex ion (for example, a bisbenzene-1,2-dithiolatonickel (III)) may also be used. As the negative counterion, a halogen anion, a substituted or unsubstituted alkylcarboxylate ion, a substituted or unsubstituted alkylsulfonate ion, a substituted or unsubstituted arylsulfonate ion, an aryldisulfonate ion, a perchlorate ion, and a hexafluorophosphate ion are preferable, and a halogen anion and a hexafluorophosphate ion are more preferable.

### - Metal Complex Dye -

The metal complex dye of the present invention is represented by Formula (I).

In the metal complex dye represented by Formula (I), the ligand LA, the ligand LD, and the ligand LX are as described above, and the combination of these ligands is not particularly limited. A preferred combination of the ligands is a combination of the preferred ligand LA, the preferred ligand LD, and the preferred ligand LX.

Formula (I) M(LA)(LD)(LX)_{mX}·(CI)_{mY}

In the formula, M, LA, LD, LX, mX, CI, and mY are as described above, and preferred examples thereof are also the same.

The metal complex dye represented by Formula (I) is preferably a metal complex dye represented by the following Formula (1-1) or (1-2).

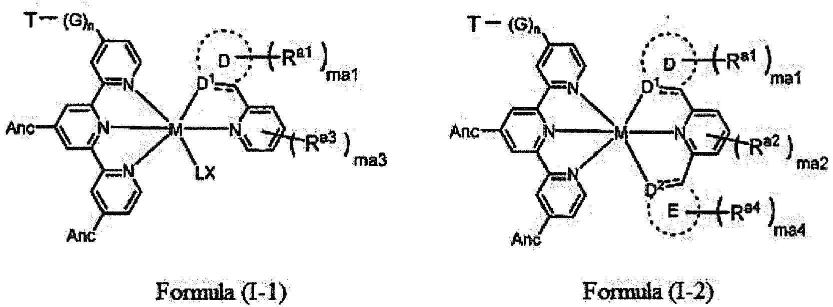

In the formula, M and LX each have the same definitions as M and LX in Formula (I).

G, T, and n each have the same definitions as G, T, and n in Formula (AL-1). Anc represents an acidic group, and has the same definition as the acidic group of Formula (AL-1), and preferred examples thereof are also the same.

The ring D and the ring E each independently represent a 5- or 6-membered aromatic ring. D¹ and D² each independently represent an anion of a carbon atom or an anion of a nitrogen atom. Here, the bond between D¹ and D² in the ring D and the ring E, and a carbon atom bonded to a pyridine ring is a single bond or a double bond. The ring D and the ring E have the same definitions as the ring D and the ring E of Formulae (DL-1) and (DL-2), and preferred examples thereof are also the same.

R^{a1} to R^{a4} each independently represent a substituent. R^{a1} to R^{a4} each have the same definitions as R^{a1} to R^{a4} of Formulae (DL-1) and (DL-2), and preferred examples thereof are also the same.

ma1, ma2, and ma4 each independently represent an integer of 0 to 3. ma3 represents an integer of 0 to 4. ma1 to ma4 each have the same definitions as ma1 to ma4 of Formulae (DL-1) and (DL-2), and preferred examples thereof are also the same. When ma1 to ma4 each represent an integer of 2 or more, a plurality of R^{a1}'s to R^{a4}'s may each be bonded to each other to form a ring.

The metal complex dye represented by Formula (I) can be synthesized by, for example, the method described in JP2013-084594A, the method described in JP4298799B, the method described in each specification of US2013/0018189A1, US2012/0073660A1, US2012/0111410A1, and US2010/0258175A1, the method described in Angew. Chem. Int. Ed., 2011, 50, pp. 2054-2058, the methods described in the reference documents listed in these documents, the patent documents regarding solar cells, known methods, or the methods equivalent thereto.

The metal complex dye represented by Formula (I) has the ligand LA and has excellent absorption characteristics in a long-wavelength region. The metal complex dye has a maximum absorption wavelength in a solution, preferably in a range from 300 to 1,000 nm, more preferably in a range from 350 to 950 nm, and particularly preferably in a range from 370 to 900 nm.

Specific examples of the metal complex dye represented by Formula (I) are shown in the following description (including Examples). Further, the specific examples in the following description and the specific examples in Examples also include metal complex dyes in which at least one of -COOH's is formed into a salt of the carboxyl group. In these metal complex dyes, examples of the counter cation that forms a salt of a carboxyl group include the positive ions described for the CI. The present invention is not limited to these metal complex dyes. In a case where these metal complex dyes have optical isomers or geometric isomers, the metal complex dye may be any of these isomers or a mixture of these isomers.

The following specific examples each independently represent the specific examples of each of the ligands LA, LD, and LX, irrespective of the specific combinations of the ligands LA, LD, and LX in the respective specific examples. Further, in the specific examples, Me represents methyl.

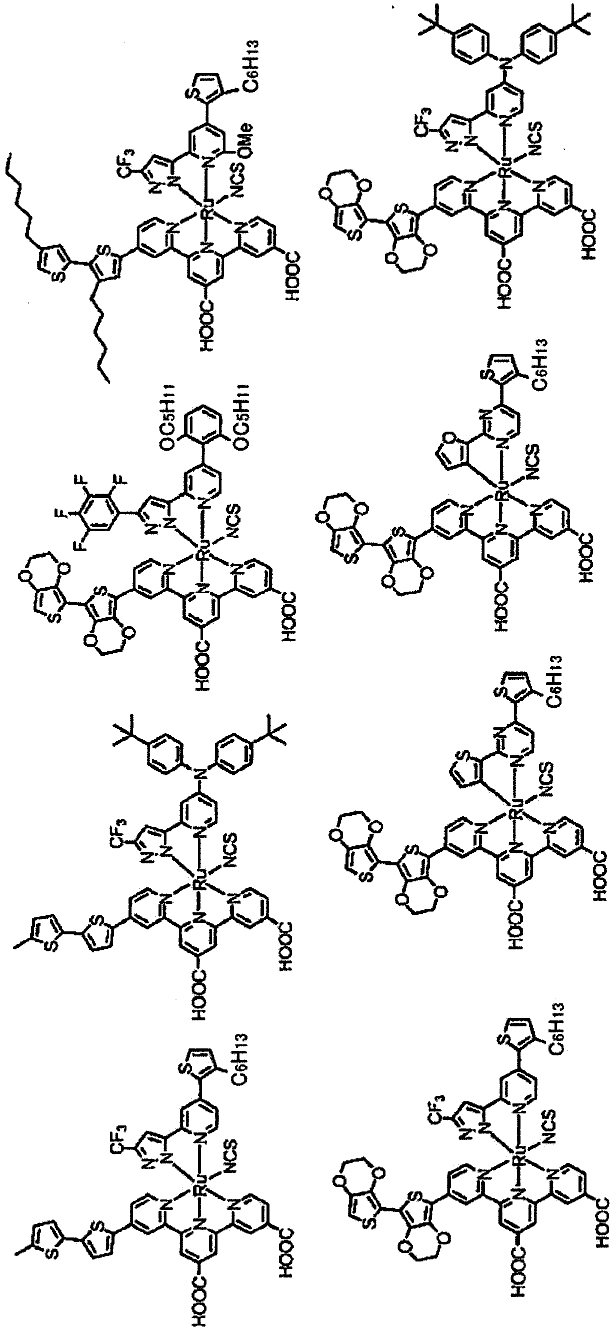

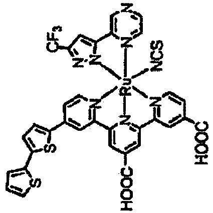

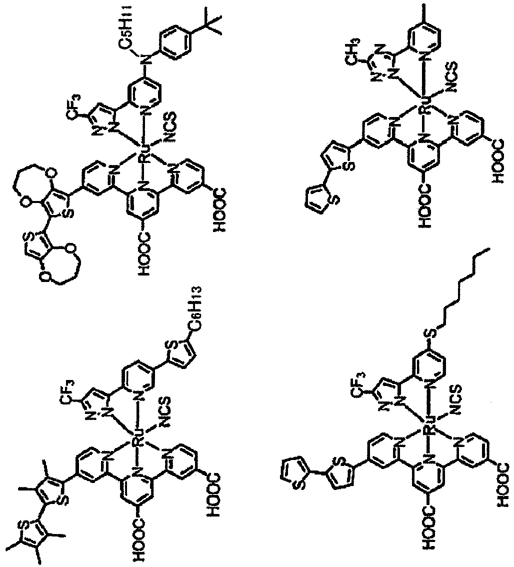

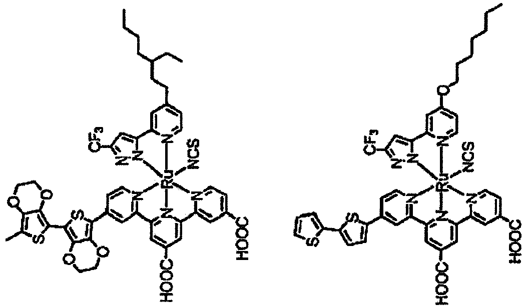

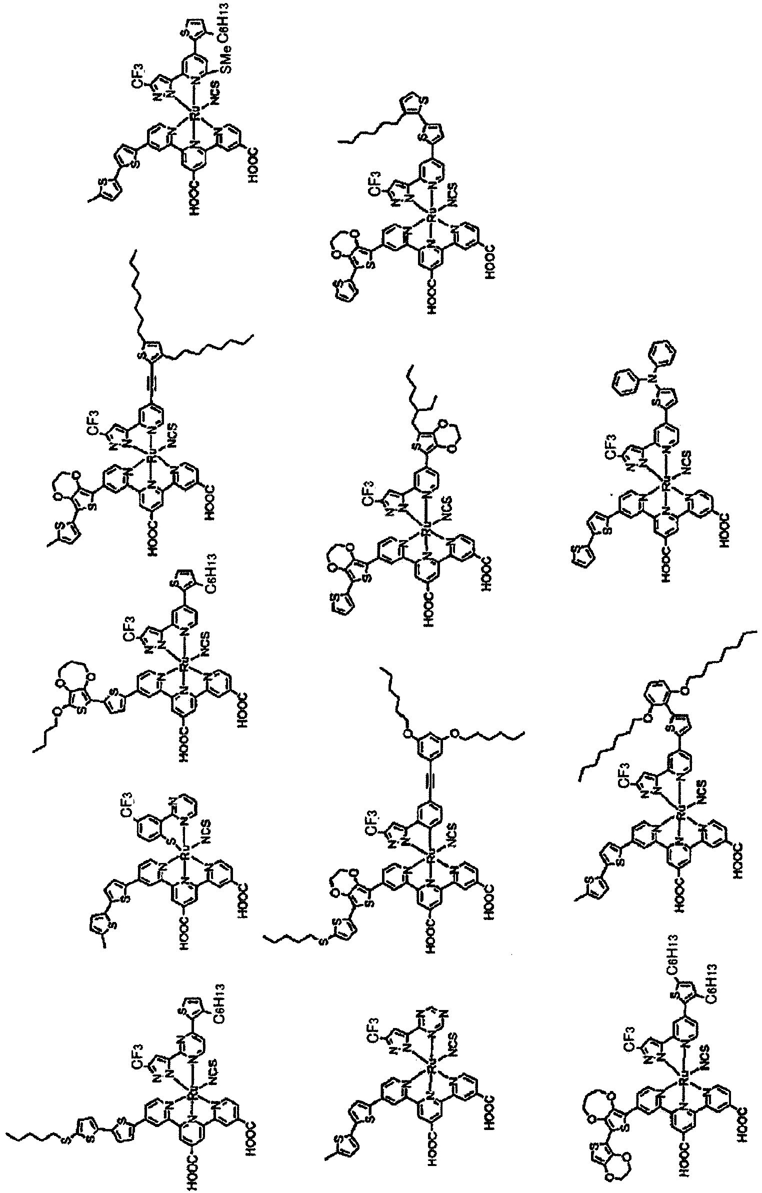

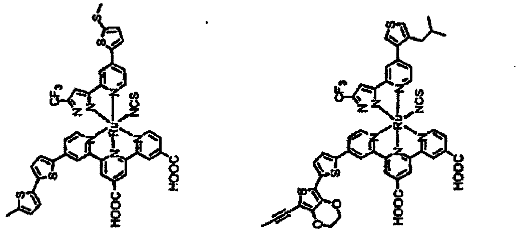

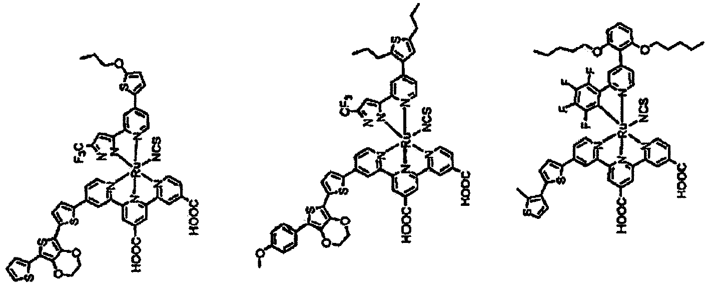

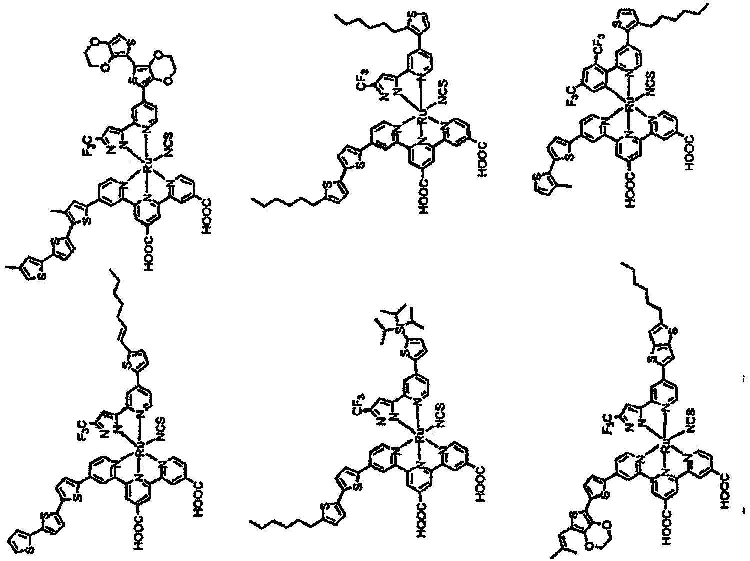

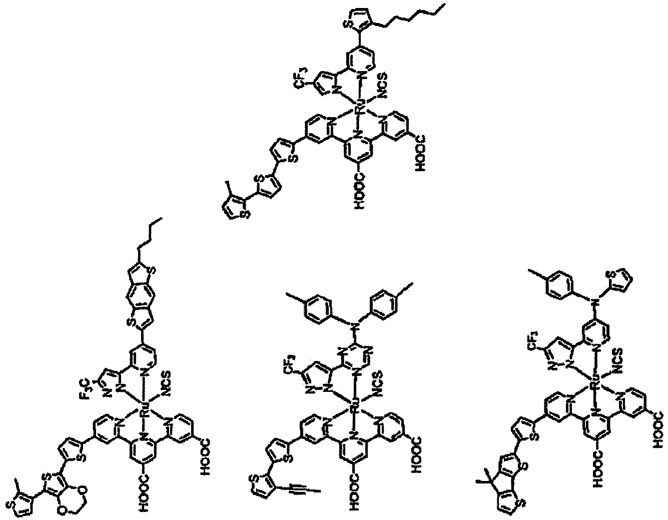

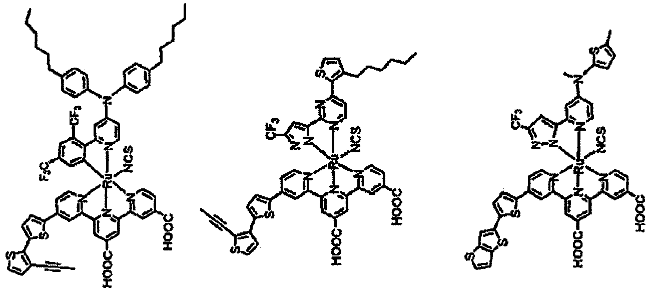

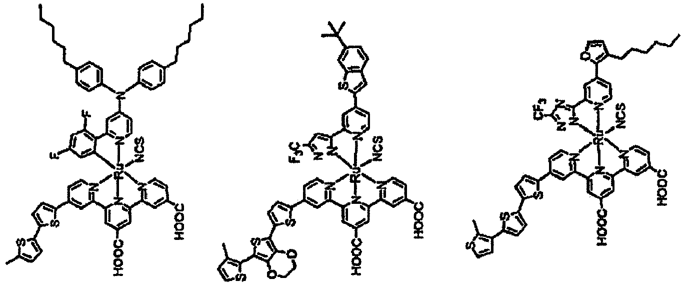

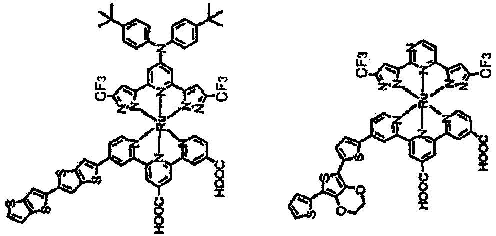

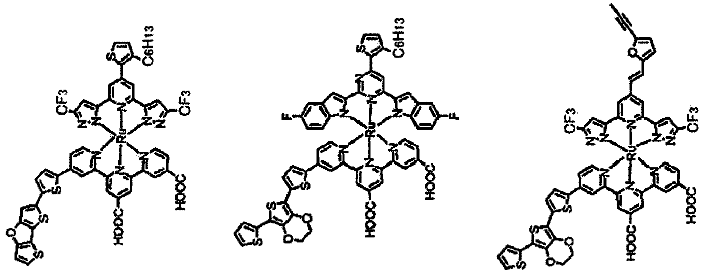

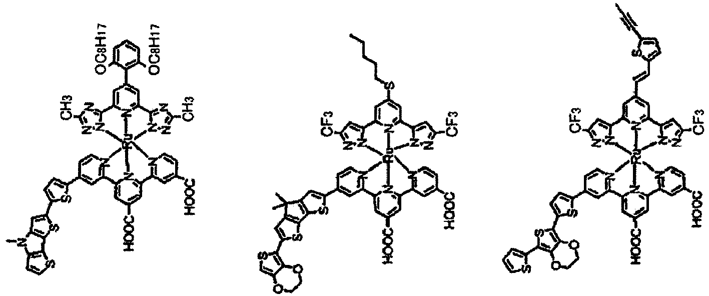

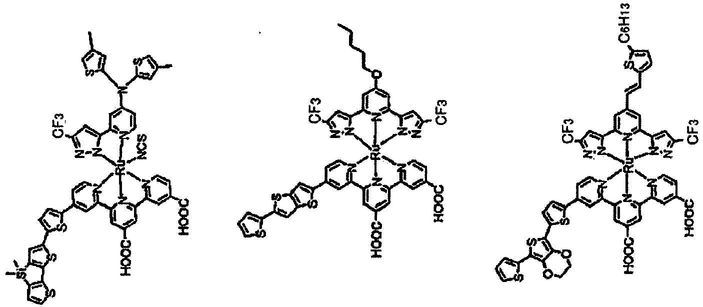

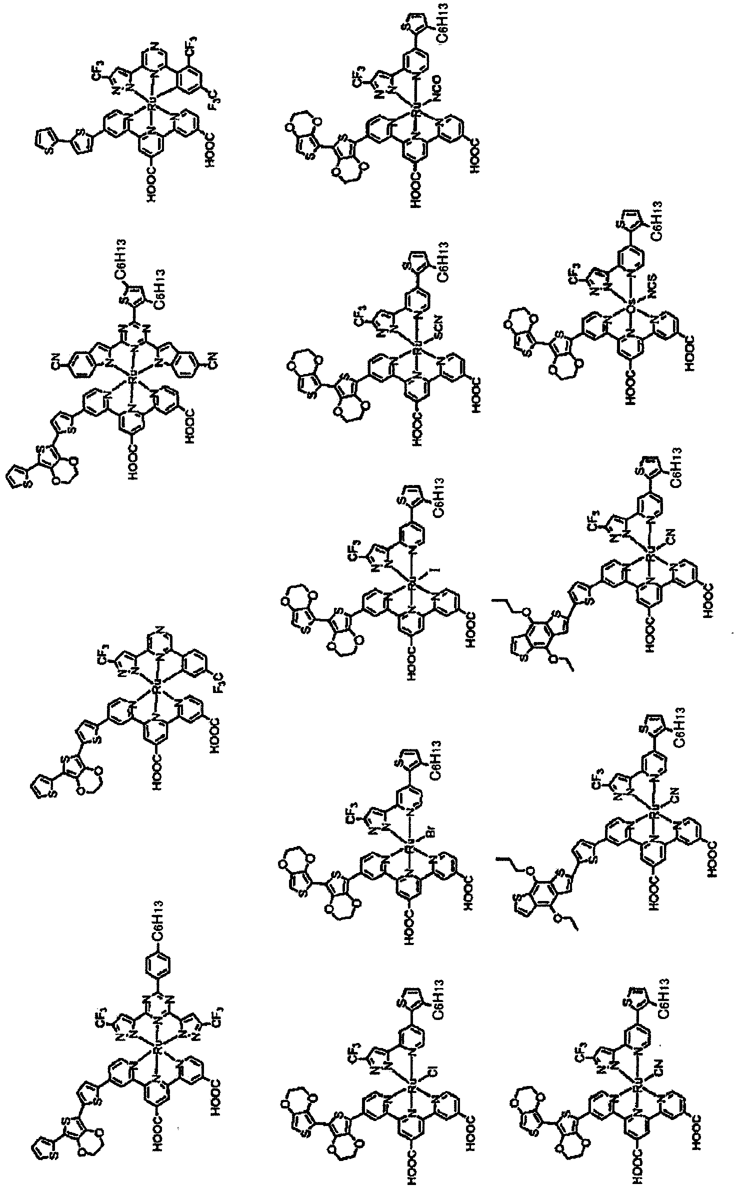

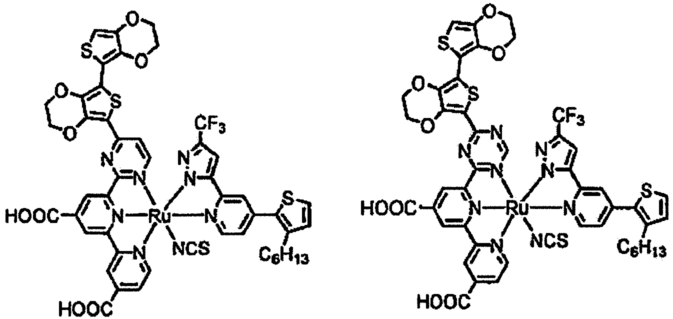

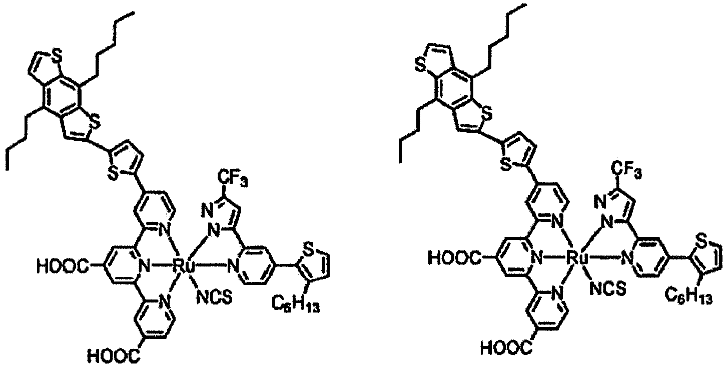

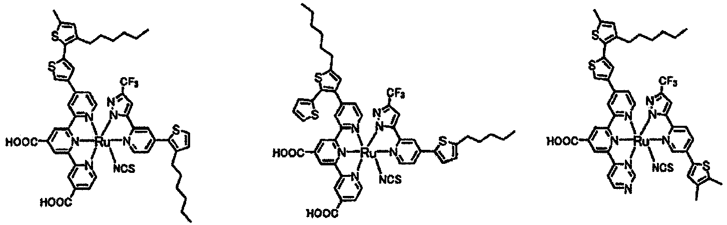

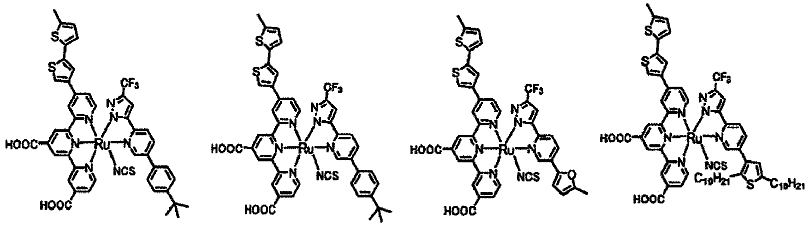

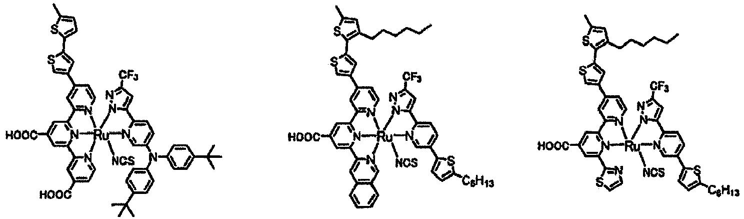

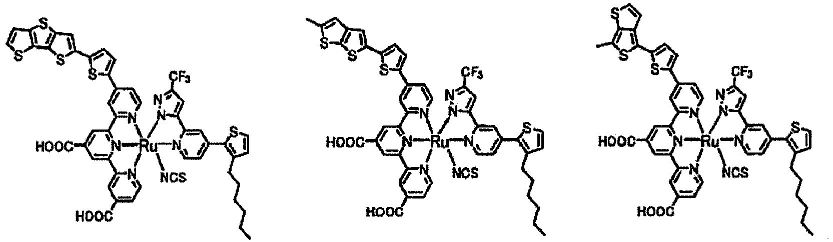

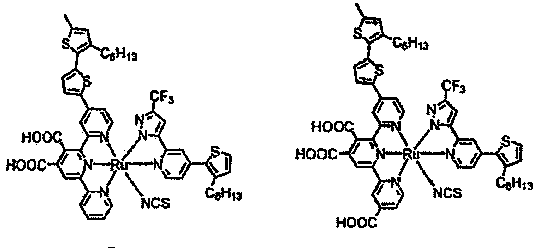

### <Substituent Group T^{R}>

In the present invention, preferred examples of the substituent include the groups selected from the following substituent group T^{R}.

Incidentally, in the present specification, in a case where there is only a simple description of a substituent, reference is made to this substituent group T^{R}, and further, in a case where each of the groups, for example, an alkyl group is merely described, preferred ranges and specific examples for the corresponding group for the substituent group T^{R} are applied.

Moreover, in the present specification, in a case where an alkyl group is described as separate from a cycloalkyl group (for example, the description of the substituents which may be adopted as R^{AA}), the alkyl group is used to mean inclusion of both of a linear alkyl group and a branched alkyl group. On the other hand, in a case where an alkyl group is not described as separate from a cycloalkyl group (a case where an alkyl group is simply described), and unless otherwise specified, the alkyl group is used to mean inclusion of a linear alkyl group, a branched alkyl group, and a cycloalkyl group. This shall apply to a group (an alkoxy group, an alkylthio group, an alkenyloxy group, and the like) including a group (an alkyl group, an alkenyl group, an alkynyl group, and the like) which can adopt a cyclic structure, and a compound (the alkyl esterified product and the like) including a group which can adopt a cyclic structure. In the following description of the substituent group T^{R}, for example, a group with a linear or branched structure and a group with a cyclic structure may be sometimes separately described for clarification of both groups, as in the alkyl group and the cycloalkyl group.

Examples of the groups included in the substituent group T^{R} include the following groups or the groups formed by the combination of a plurality of the following groups:
an alkyl group (preferably an alkyl group having 1 to 20 carbon atoms, for example, methyl, ethyl, isopropyl, n-butyl, t-butyl, pentyl, hexyl, heptyl, octyl, 1-ethylpentyl, benzyl, 2-ethoxyethyl, 1-carboxymethyl, and trifluoromethyl), an alkenyl group (preferably an alkenyl group having 2 to 20 carbon atoms, for example, vinyl, allyl, butenyl, and oleyl), an alkynyl group (preferably an alkynyl group having 2 to 20 carbon atoms, for example, ethynyl, butynyl, and phenylethynyl), a cycloalkyl group (preferably a cycloalkyl group having 3 to 20 carbon atoms, for example, cyclopropyl, cyclopentyl, cyclohexyl, and 4-methylcyclohexyl), an cycloalkenyl group (preferably a cycloalkenyl group having 5 to 20 carbon atoms, for example, cyclopentenyl and cyclohexenyl), an aryl group (an aromatic hydrocarbon ring group, and preferably an aryl group having 6 to 26 carbon atoms, for example, phenyl, 1-naphthyl, 4-methoxyphenyl, 2-chlorophenyl, 3-methylphenyl, difluorophenyl, and tetrafluorophenyl), a hetero ring group (preferably a hetero ring group having 2 to 20 carbon atoms, and more preferably a 5- or 6-membered hetero ring group having at least one oxygen atom, sulfur atom, or nitrogen atom. The hetero ring encompasses an aromatic ring and an aliphatic ring. Examples of the aromatic hetero ring group (for example, a heteroaryl group) include the following groups: for example, 2-pyridyl, 4-pyridyl, 2-imidazolyl, 2-benzimidazolyl, 2-thiazolyl, and 2-oxazolyl), an alkoxy group (preferably an alkoxy group having 1 to 20 carbon atoms, for example, methoxy, ethoxy, isopropyloxy, and benzyloxy), an alkenyloxy group (preferably an alkenyloxy group having 2 to 20 carbon atoms, for example, vinyloxy and allyloxy), an alkynyloxy group (preferably an alkynyloxy group having 2 to 20 carbon atoms, for example, 2-propynyloxy and 4-butynyloxy), a cycloalkyloxy group (preferably a cycloalkyloxy group having 3 to 20 carbon atoms, for example, cyclopropyloxy, cyclopentyloxy, cyclohexyloxy, and 4-methylcyclohexyloxy), an aryloxy group (preferably an aryloxy group having 6 to 26 carbon atoms, for example, phenoxy, 1-naphthyloxy, 3-methylphenoxy, and 4-methoxyphenoxy), a heterocyclic oxy group (for example, imidazolyloxy, benzimidazolyloxy, thiazolyloxy, benzothiazolyloxy, triazinyloxy, and purinyloxy),
an alkoxycarbonyl group (preferably an alkoxycarbonyl group having 2 to 20 carbon atoms, for example, ethoxycarbonyl and 2-ethylhexyloxycarbonyl), a cycloalkoxycarbonyl group (preferably a cycloalkoxycarbonyl group having 4 to 20 carbon atoms, for example, cyclopropyloxycarbonyl, cyclopentyloxycarbonyl, and cyclohexyloxycarbonyl), an aryloxycarbonyl group (preferably an aryloxycarbonyl group having 6 to 20 carbon atoms, for example, phenyloxycarbonyl, and naphthyloxycarbonyl), an amino group (preferably an amino group having 0 to 20 carbon atoms, including an alkylamino group, an alkenylamino group, an alkynylamino group, a cycloalkylamino group, a cycloalkenylamino group, an arylamino group, and a heterocyclic amino group, for example, amino, N,N-dimethylamino, N,N-diethylamino, N-ethylamino, N-allylamino, N-(2-propynyl)amino, N-cyclohexylamino, N-cyclohexenylamino, anilino, pyridylamino, imidazolylamino, benzimidazolylamino, thiazolylamino, benzothiazolylamino, and triazinylamino), a sulfamoyl group (preferably a sulfamoyl group having 0 to 20 carbon atoms, preferably an alkyl-, cycloalkyl-, and aryl-sulfamoyl group, for example, N,N-dimethylsulfamoyl, N-cyclohexylsulfamoyl, and N-phenylsulfamoyl), an acyl group (preferably an acyl group having 1 to 20 carbon atoms, for example, acetyl, cyclohexylcarbonyl, and benzoyl), an acyloxy group (preferably an acyloxy group having 1 to 20 carbon atoms, for example, acetyloxy, cyclohexylcarbonyloxy, and benzoyloxy), a carbamoyl group (preferably a carbamoyl group having 1 to 20 carbon atoms, preferably alkyl-, cycloalkyl-, and aryl-carbamoyl groups, for example, N,N-dimethylcarbamoyl, N-cyclohexylcarbamoyl, and N-phenylcarbamoyl),
an acylamino group (preferably an acylamino group having 1 to 20 carbon atoms, for example, acetylamino, cyclohexylcarbonylamino, and benzoylamino), a sulfonamido group (preferably a sulfonamido group having 0 to 20 carbon atoms, preferably alkyl-, cycloalkyl-, and aryl-sulfonamido groups, for example, methane sulfonamide, benzene sulfonamide, N-methyl methane sulfonamide, N-cyclohexyl sulfonamide, and N-ethyl benzene sulfonamide), an alkylthio group (preferably an alkylthio group having 1 to 20 carbon atoms, for example, methylthio, ethylthio, isopropylthio, pentylthio, and benzylthio), a cycloalkylthio group (preferably a cycloalkylthio group having 3 to 20 carbon atoms, for example, cyclopropylthio, cyclopentylthio, cyclohexylthio, and 4-methylcyclohexylthio), an arylthio group (preferably an arylthio group having 6 to 26 carbon atoms, for example, phenylthio, 1-naphthylthio, 3-methylphenylthio, and 4-methoxyphenylthio), an alkyl-, cycloalkyl-, and aryl-sulfonyl group (preferably a sulfonyl group having 1 to 20 carbon atoms, for example, methylsulfonyl, ethylsulfonyl, cyclohexylsulfonyl, and benzenesulfonyl),
a silyl group (preferably a silyl group having 1 to 20 carbon atoms, preferably alkyl-, aryl-, alkoxy-, and aryloxy-substituted silyl groups, for example, trimethylsilyl, triethylsilyl, triisopropylsilyl, triphenylsilyl, diethylbenzylsilyl, and dimethylphenylsilyl), a silyloxy group (preferably a silyloxy group having 1 to 20 carbon atoms, preferably alkyl-, aryl-, alkoxy-, and aryloxy-substituted silyloxy groups, for example, triethylsilyloxy, triphenylsilyloxy, diethylbenzylsilyloxy, and dimethylphenylsilyloxy), a hydroxyl group, a cyano group, a nitro group, and a halogen atom (for example, a fluorine atom, a chlorine atom, a bromine atom, and iodine atom).

Examples of the group selected from the substituent group T^{R} more preferably include an alkyl group, an alkenyl group, a cycloalkyl group, an aryl group, a hetero ring group, an alkoxy group, a cycloalkoxy group, an aryloxy group, an alkoxycarbonyl group, a cycloalkoxycarbonyl group, an amino group, an acylamino group, a cyano group, and a halogen atom, and particularly preferably include an alkyl group, an alkenyl group, a hetero ring group, an alkoxy group, an alkoxycarbonyl group, an amino group, an acylamino group, and a cyano group.

When the compound, the substituent, or the like includes an alkyl group, an alkenyl group, or the like, these may be substituted or unsubstituted. Further, when the compound, the substituent, or the like includes an aryl group, a hetero ring group, or the like, these may be a monocycle or a fused ring, and may be substituted or unsubstituted.

Next, preferred aspects of the main members of the photoelectric conversion element and the dye-sensitized solar cell will be described with respect to Figs. 1 and 2.

### <Electrically Conductive Support>

The electrically conductive support is not particularly limited as long as it has electrical conductivity and is capable of supporting a photoconductor layer 2 or the like. The electrically conductive support is preferably an electrically conductive support 1 formed of a material having conductivity, such as a metal, or an electrically conductive support 41 having a glass or plastic substrate 44 and a transparent electrically-conductive film 43 formed on the surface of the substrate 44.

Between them, the electrically conductive support 41 in which the transparent electrically-conductive film 43 is formed by applying an electrically conductive metal oxide onto the surface of the substrate 44 is more preferable. Examples of the substrate 44 formed of plastics include the transparent polymer films described in paragraph No. 0153 of JP2001-291534A. Further, as a material which forms the substrate 44, ceramics (JP2005-135902A) or electrically conductive resins (JP2001-160425A) can be used, in addition to glass and plastics. As the metal oxide, tin oxide (TO) is preferable, and indium-tin oxide (tin-doped indium oxide; ITO), and fluorine-doped tin oxide such as tin oxide which has been doped with tin (FTO) are particularly preferable. In this case, the coating amount of the metal oxide is preferably 0.1 to 100 g, per square meter of the surface area of the substrate 44. In the case of using the electrically conductive support 41, it is preferable that light is incident from the substrate 44.

It is preferable that the electrically conductive supports 1 and 41 are substantially transparent. The expression, "substantially transparent", means that the transmittance of light (at a wavelength of 300 to 1,200 nm) is 10% or more, preferably 50% or more, and particularly preferably 80% or more.

The thickness of the electrically conductive supports 1 and 41 is not particularly limited, but is preferably 0.05 µm to 10 mm, more preferably 0.1 µm to 5 mm, and particularly preferably 0.3 µm to 4 mm.

In the case of providing a transparent electrically-conductive film 43, the thickness of the transparent electrically-conductive film 43 is preferably 0.01 to 30 µm, more preferably 0.03 to 25 µm, and particularly preferably 0.05 to 20 µm.

The electrically conductive supports 1 and 41 may be provided with a light management function at the surface, and may have, for example, the anti-reflection film having a high refractive index film and a low refractive index oxide film alternately laminated described in JP2003-123859A, and the light guide function described in JP2002-260746A on the surface.

### <Photoconductor Layer>

As long as the photoconductor layer has semiconductor fine particles 22 carrying the dye 21 and an electrolyte, it is not particularly limited in terms of other configurations. Preferred examples thereof include the photoconductor layer 2 and the photoconductor layer 42.

### - Semiconductor Fine Particles (Layer Formed by Semiconductor Fine Particles) -

The semiconductor fine particles 22 are preferably fine particles of chalcogenides of metals (for example, oxides, sulfides, and selenides) or of compounds having perovskite type crystal structures. Preferred examples of the chalcogenides of metals include oxides of titanium, tin, zinc, tungsten, zirconium, hafnium, strontium, indium, cerium, yttrium, lanthanum, vanadium, niobium, or tantalum, cadmium sulfide, and cadmium selenide. Preferred examples of the compounds having perovskite type crystal structures include strontium titanate and calcium titanate. Among these, titanium oxide (titania), zinc oxide, tin oxide, and tungsten oxide are particularly preferable.

Examples of the crystal structure of titania include structures of an anatase type, a brookite type, and a rutile type, with the structures of an anatase type and a brookite type being preferable. A titania nanotube, nanowire, or nanorod may be used singly or in mixture with titania fine particles.

The particle diameter of the semiconductor fine particles 22 is expressed in terms of an average particle size using a diameter when a projected area is converted into a circle, and is preferably 0.001 to 1 µm as primary particles, and 0.01 to 100 µm as an average particle size of dispersions. Examples of the method for coating the semiconductor fine particles 22 on the electrically conductive supports 1 or 41 include a wet method, a dry method, and other methods.

It is preferable that the semiconductor fine particles 22 have a large surface area so that they may adsorb a large amount of the dye 21. For example, in a state where the semiconductor fine particles 22 are coated on the electrically conductive support 1 or 41, the surface area is preferably 10 times or more, and more preferably 100 times or more, with respect to the projected surface area. The upper limit of this value is not particularly limited, and the upper limit is usually about 5,000 times. In general, as the thickness of the semiconductor layer 45 (having the same definition as the photoconductor layer 2 in the photoelectric conversion element 10) formed by the semiconductor fine particles 22 increases, the amount of dye 21 that can be carried per unit area increases, and therefore, the light absorption efficiency increases. However, since the diffusion distance of generated electrons increases correspondingly, the loss due to charge recombination also increases.

As described above, it can be expected that in the photoelectric conversion element and the dye-sensitized solar cell, as the diffusion distance of excited electrons is smaller, the electron transport efficiency more increases. However, when the thickness of the semiconductor layer is decreased, the photoelectric conversion efficiency may be reduced in some cases. The photoelectric conversion element and the dye-sensitized solar cell of the present invention have the metal complex dye of the present invention, which uses a combination of the ligand LA and the ligand LD. Thus, even in a case where the semiconductor layer has the same thickness as that the related art or has a smaller thickness than that in the related art, excellent photoelectric conversion efficiency is exerted. Thus, according to the present invention, the effect of the film thickness of the semiconductor layer is little and excellent photoelectric conversion efficiency is exerted.

Although a preferred thickness of the semiconductor layer 45 (the photoconductor layer 2 in the photoelectric conversion element 10) may vary depending on the utility of the photoelectric conversion element, the thickness is typically 0.1 to 100 µm. In the case of using the photoelectric conversion element as a dye-sensitized solar cell, the thickness of the photoconductor layer is more preferably 1 to 50 µm, and still more preferably 3 to 30 µm.

In the present invention, by using the metal complex dye represented by Formula (I), the thickness of the semiconductor layer 45 can be reduced. For example, among the preferred ranges, the thickness can be adjusted to 8 µm or less, and to 6 µm or less.

It is preferable that the semiconductor fine particles 22 may be calcined at a temperature of 100°C to 800°C for 10 minutes to 10 hours after being applied on the electrically conductive support 1 or 41, so as to bring about cohesion of the particles. In the case of using glass as a material for the electrically conductive support 1 or the substrate 44 is used, the temperature is preferably 60°C to 600°C.

The coating amount of the semiconductor fine particles 22 per square meter of the surface area of the electrically conductive support 1 or 41 is preferably 0.5 to 500 g, and more preferably 5 to 100 g.

It is preferable that a short circuit-preventing layer is formed between the electrically conductive support 1 or 41 and the photoconductor layer 2 or 42 so as to prevent reverse current due to a direct contact between the electrolyte included in the photoconductor layer 2 or 42 and the electrically conductive support 1 or 41.

In addition, it is preferable to use a spacer S (see Fig. 2) or a separator, so as to prevent contact between the light-receiving electrode 5 or 40 and the counter electrode 4 or 48.

### - Dye -

In the photoelectric conversion element 10 and the dye-sensitized solar cell 20, at least one kind of metal complex dye represented by Formula (I) is used as a sensitizing dye. The metal complex dye represented by Formula (I) is as described above.

In the present invention, examples of the dye that can be used in combination with the metal complex dye of Formula (I) include an Ru complex dye, a squarylium cyanine dye, an organic dye, a porphyrin dye, and a phthalocyanine dye.

Examples of the Ru complex dye include the Ru complex dyes described in JP1995-500630A (JP-H07-500630A) (in particular, the dyes synthesized in Examples 1 to 19 described in from line 5 on left lower column on page 5 to line 7 on right upper column on page 7), the Ru complex dyes described in JP2002-512729A (in particular, dyes synthesized in Examples 1 to 16 described in line 3 from the bottom of page 20 to line 23 on page 29), the Ru complex dyes described in JP2001-59062A (in particular, the dyes described in paragraph Nos. 0087 to 0104), the Ru complex dyes described in JP2001-6760A (in particular, the dyes described in paragraph Nos. 0093 to 0102), the Ru complex dyes described in JP2001-253894A (in particular, the dyes described in paragraph Nos. 0009 and 0010), the Ru complex dyes described in JP2003-212851A (in particular, the dyes described in paragraph No. 0005), the Ru complex dyes described in WO2007/91525A (in particular, the dyes described in [0067]), the Ru complex dyes described in JP2001-291534A (in particular, the dyes described in paragraph Nos. 0120 to 0144), the Ru complex dyes described in JP2012-012570A (in particular, the dyes described in paragraph Nos. 0095 to 0103), the Ru complex dyes described in JP2013-084594A (in particular, the dyes described in paragraph Nos. 0072 to 0081 and the like), the Ru complex dyes described in WO2013/088898A (in particular, the dyes described in [0286] to [0293]), and the Ru complex dyes described in WO2013/47615A (in particular, the dyes described in [0078] to [0082]).

Examples of the squarylium cyanine dye include the squarylium cyanine dyes described in JP1999-214730A (JP-H11-214730A) (in particular, the dyes described in paragraph Nos. 0036 to 0047), the squarylium cyanine dyes described in JP2012-144688A (in particular, the dyes described in paragraph Nos. 0039 to 0046 and 0054 to 0060), and the squarylium cyanine dyes described in JP2012-84503A (in particular, the dyes described in paragraph Nos. 0066 to 0076 and the like).

Examples of the organic dye include the organic dyes described in JP2004-063274A (in particular, the dyes described in paragraph Nos. 0017 to 0021), the organic dyes described in JP2005-123033A (in particular, the dyes described in paragraph Nos. 0021 to 0028), the organic dyes described in JP2007-287694A (in particular, the dyes described in paragraph Nos. 0091 to 0096), the organic dyes described in JP2008-71648A (in particular, the dyes described in paragraph Nos. 0030 to 0034), and the organic dyes described in WO2007/1l9525A (in particular, the dyes described in paragraph No. [0024]).

Examples of the porphyrin dye include the porphyrin dyes described in Angew. Chem. Int. Ed., 49, pp. 1 to 5 (2010), or the like, and examples of the phthalocyanine dye include the phthalocyanine dyes described in Angew. Chem. Int. Ed., 46, p. 8358 (2007), or the like.

As the dye to be used in combination, Ru complex dyes, squarylium cyanine dyes, or organic dyes are preferable.

The overall amount of the dye to be used is preferably 0.01 to 100 millimoles, more preferably 0.1 to 50 millimoles, and particularly preferably 0.1 to 10 millimoles, per square meter of the surface area of the electrically conductive support 1 or 41. Further, the amount of the dye 21 to be adsorbed onto the semiconductor fine particles 22 is preferably 0.001 to 1 millimole, and more preferably 0.1 to 0.5 millimoles, with respect to 1 g of the semiconductor fine particles 22. By setting the amount of the dye to such a range, the sensitization effect on the semiconductor fine particles 22 is sufficiently obtained.

In a case where the metal complex dye represented by Formula (I) is used in combination with another dye, the ratio of the mass of the metal complex dye represented by Formula (I)/the mass of another dye is preferably 95/5 to 10/90, more preferably 95/5 to 50/50, still more preferably 95/5 to 60/40, particularly preferably 95/5 to 65/35, and most preferably 95/5 to 70/30.

After the dye is carried on the semiconductor fine particles 22, the surface of the semiconductor fine particles 22 may be treated using an amine compound. Preferred examples of the amine compound include pyridine compounds (for example, 4-t-butylpyridine and polyvinylpyridine). These may be used as they are in a case where they are liquids, or may be used in a state where they are dissolved in an organic solvent.

### - Co-Adsorbent -

In the present invention, it is preferable to use a co-adsorbent together with the metal complex dye represented by Formula (I) or with another dye to be used in combination, if necessary. Such a co-adsorbent which includes a co-adsorbent having at least one acidic group (preferably a carboxyl group or a salt thereof) is preferable, and examples thereof include a fatty acid and a compound having a steroid skeleton.

The fatty acid may be a saturated fatty acid or an unsaturated fatty acid, and examples thereof include a butanoic acid, a hexanoic acid, an octanoic acid, a decanoic acid, a hexadecanoic acid, a dodecanoic acid, a palmitic acid, a stearic acid, an oleic acid, a linoleic acid, and a linolenic acid.

Examples of the compound having a steroid skeleton include cholic acid, glycocholic acid, chenodeoxycholic acid, hyocholic acid, deoxycholic acid, lithocholic acid, and ursodeoxycholic acid, among which cholic acid, deoxycholic acid, and chenodeoxycholic acid are preferable; and chenodeoxycholic acid are more preferable.

A preferred co-adsorbent is a compound represented by the following Formula (CA).

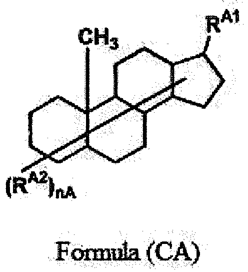

In the formula, R^{A1} represents a substituent having an acidic group. R^{A2} represents a substituent. nA represents an integer of 0 or more.

The acidic group has the same definitions as the acidic group in Formula (AL-1), and a preferred range thereof is also the same.

Among these, R^{A1} is preferably an alkyl group substituted with a carboxyl group, a sulfo group, or a salt thereof, and more preferably -CH(CH₃)CH₂CH₂CO₂H or -CH(CH₃)CH₂CH₂CONHCH₂CH₂SO₃H.

Examples of R^{A2} include groups selected from the substituent group T^{R}. Among those, an alkyl group, a hydroxyl group, an acyloxy group, an alkylaminocarbonyloxy group, or an arylaminocarbonyloxy group is preferable; and an alkyl group, a hydroxyl group, or an acyloxy group is more preferable.

nA is preferably 2 to 4.

By making the co-adsorbent adsorbed onto the semiconductor fine particles 22, the co-adsorbent exhibits an effect of suppressing the inefficient association of the metal complex dye and an effect of preventing reverse electron transfer from the surface of the semiconductor fine particles to the redox system in the electrolyte. The amount of the co-adsorbent to be used is not particularly limited, and from the viewpoint of exhibiting the above effects effectively, the amount is preferably 1 to 200 moles, more preferably 10 to 150 moles, and particularly preferably 20 to 50 moles, with respect to 1 mole of the metal complex dye.

### - Light-Scattering Layer -

In the present invention, the light-scattering layer is different from the semiconductor layer in that the light-scattering layer has a function of scattering incident light.

In the dye-sensitized solar cell 20, the light-scattering layer 46 preferably contains rod-shaped or plate-shaped metal oxide particles. Examples of the metal oxide particles to be used in the light-scattering layer 46 include particles of the chalcogenides (oxides) of the metals. In the case of providing the light-scattering layer 46, it is preferable that the thickness of the light-scattering layer is set to 10% to 50% of the thickness of the photoconductor layer 42.

The light-scattering layer 46 is preferably the light-scattering layer described in JP2002-289274A, and the description of JP2002-289274A is preferably herein incorporated by reference.

### <Charge Transfer Layer>

The charge transfer layers 3 and 47 used in the photoelectric conversion element of the present invention are layers having a function of complementing electrons for the oxidized forms of the dye 21, and are provided between the light-receiving electrode 5 or 40 and the counter electrode 4 or 48.

The charge transfer layers 3 and 47 include electrolytes. Here, the expression, "the charge transfer layer includes an electrolyte", is used to inclusion of both of an aspect in which the charge transfer layer consists of only electrolytes and an aspect in which the charge transfer layer consists of electrolytes and materials other than the electrolytes.

The charge transfer layers 3 and 47 may be any of a solid form, a liquid form, a gel form, or a mixture thereof.

### - Electrolyte -

Examples of the electrolyte include a liquid electrolyte having a redox pair dissolved in an organic solvent, and a so-called gel electrolyte in which a molten salt containing a redox pair and a liquid having a redox pair dissolved in an organic solvent are impregnated in a polymer matrix. Among those, from the viewpoint of photoelectric conversion efficiency, a liquid electrolyte is preferable.

Examples of the redox pair include a combination of iodine and an iodide (preferably an iodide salt or an iodide ionic liquid, and preferably lithium iodide, tetrabutylammonium iodide, tetrapropylammonium iodide, and methylpropylimidazolium iodide), a combination of an alkylviologen (for example, methylviologen chloride, hexylviologen bromide, and benzylviologen tetrafluoroborate) and a reductant thereof, a combination of a polyhydroxybenzene (for example, hydroquinone and naphthohydroquinone) and an oxidized form thereof, a combination of a divalent iron complex and a trivalent iron complex (for example, a combination of potassium ferricyanide and potassium ferrocyanide), and a combination of a divalent cobalt complex and a trivalent cobalt complex. Among these, a combination of iodine and an iodide, or a combination of a divalent cobalt complex and a trivalent cobalt complex is preferable, and a combination of iodine and an iodide is particularly preferable.

As the cobalt complex, the complex represented by Formula (CC) described in paragraph Nos. 0144 to 0156 of JP2014-82189A is preferable, and the description of paragraph Nos. 0144 to 0156 of JP2014-82189A is preferably incorporated in the present specification.

In a case where a combination of iodine and iodide is used as an electrolyte, it is preferable that a nitrogen-containing aromatic cation iodide salt as a 5- or 6-membered ring is additionally used.

The organic solvent which is used in a liquid electrolyte and a gel electrolyte is not particularly limited, but is preferably an aprotic polar solvent (for example, acetonitrile, propylene carbonate, ethylene carbonate, dimethylformamide, dimethylsulfoxide, sulfolane, 1,3-dimethylimidazolinone, and 3-methyloxazolidinone).

In particular, as the organic solvent which is used for a liquid electrolyte, a nitrile compound, an ether compound, an ester compound, or the like is preferable, a nitrile compound is more preferable, and acetonitrile or methoxypropionitrile is particularly preferable.

As a molten salt, an ionic liquid including an imidazolium or triazolium type cation, an ionic liquid including an oxazolium type cation, an ionic liquid including a pyridinium type cation, an ionic liquid including a guanidium type cation, and combinations of these are preferable. Further, these cations may be used in combination with specific anions. Additives may be added to these molten salts. The molten salt may have a substituent having liquid crystalline properties. In addition, a molten salt of the quaternary ammonium salt may also be used as the molten salt.

Other examples of the molten salts include a molten salt to which fluidity at room temperature has been provided by mixing lithium iodide and at least one kind of other lithium salt (for example, lithium acetate and lithium perchlorate) with polyethylene oxide. In this case, the amount of the polymer to be added is 1% to 50% by mass. Further, an electrolytic solution may contain γ-butyrolactone, and this γ-butyrolactone increases the diffusion efficiency of iodide ions, whereby the photoelectric conversion efficiency is enhanced.

Examples of the polymer (polymer matrix) to be used in a matrix of the gel electrolyte include polyacrylonitrile and polyvinylidene fluoride.

The electrolyte may be quasi-solidified by adding a gelling agent to an electrolytic solution formed of an electrolyte and a solvent, followed by gelling (the quasi-solidified electrolyte may also be hereinafter referred to as a "quasi-solidified electrolyte"). Examples of the gelling agent include an organic compound having a molecular weight of 1,000 or less, an Si-containing compound having a molecular weight in the range of 500 to 5,000, an organic salt generated from a specific acidic compound and a specific basic compound, a sorbitol derivative, and polyvinylpyridine.

Furthermore, a method of confining a polymer matrix, a crosslinkable polymer compound or monomer, a crosslinking agent, an electrolyte, and a solvent in a polymer may also be used.

Preferred examples of the polymer matrix include a polymer having a nitrogen-containing heterocycle in a repeating unit in the main chain or in the side chain, and a crosslinked structure formed by reacting the polymer with an electrophilic compound, a polymer having a triazine structure, a polymer having a ureide structure, a polymer containing a liquid crystalline compound, a polymer having an ether bond, polyvinylidene fluoride, a methacrylate, an acrylate, a thermosetting resin, crosslinked polysiloxane, polyvinyl alcohol (PVA), a clathrate compound of polyalkylene glycol with dextrin or the like, a system incorporated with an oxygen-containing or sulfur-containing polymer, and a naturally occurring polymer. An alkali-swellable polymer, a polymer having a compound capable of forming a charge transfer complex of iodine with a cation moiety within one polymer or the like may be added to the polymer matrix.

A system containing, as a polymer matrix, a crosslinked polymer formed by reacting a bifunctional or higher-functional isocyanate as one component with a functional group such as a hydroxyl group, an amino group or a carboxyl group, may also be used. Furthermore, a crosslinked polymer based on a hydrosilyl group and a double-bonded compound, a crosslinking method involving reacting polysulfonic acid, polycarboxylic acid, or the like with a divalent or higher-valent metal ion compound, and the like may also be used.

Examples of the solvent that can be preferably used in combination with the quasi-solid electrolyte described above include a specific phosphoric ester, a mixed solvent including ethylene carbonate, and a solvent having a specific relative permittivity. A liquid electrolyte solution may be retained in a solid electrolyte membrane or in pores, and preferred examples of the method for retaining the liquid electrolyte solution include a method using an electrically conductive polymer membrane, a fibrous solid, and a fabric-like solid such as a filter.

The electrolyte may contain aminopyridine compounds, benzimidazole compounds, aminotriazole compounds, aminothiazole compounds, imidazole compounds, aminotriazine compounds, urea compounds, amide compounds, pyrimidine compounds, and heterocycles not including nitrogen, in addition to pyridine compounds such as 4-t-butylpyridine, as an additive.

Moreover, a method of controlling the moisture content of the electrolytic solution may be employed in order to enhance the photoelectric conversion efficiency. Preferred examples of the method of controlling the moisture content include a method of controlling the concentration, and a method of adding a dehydrating agent. The moisture content of the electrolytic solution is preferably adjusted to 0% to 0.1% by mass.

Iodine can also be used as a clathrate compound of iodine with cyclodextrin. Furthermore, a cyclic amidine may be used, or an antioxidant, a hydrolysis inhibitor, a decomposition inhibitor, or zinc iodide may be added.

A solid-state charge transport layer such as a p-type semiconductor or a hole transport material, for example, CuI or CuNCS, may be used in place of the liquid electrolyte and the quasi-solid-state electrolyte as described above. Moreover, the electrolytes described in Nature, vol. 486, p. 487 (2012) and the like may also be used. For a solid-state charge transport layer, an organic hole transport material may be used. Preferred examples of the hole transport layer include electrically conductive polymers such as polythiophene, polyaniline, polypyrrole, and polysilane; a spiro compound in which two rings share a central element adopting a tetrahedral structure, such as C and Si; aromatic amine derivatives such as triarylamine; triphenylene derivatives; nitrogen-containing heterocyclic derivatives; and liquid crystalline cyano derivatives.

The redox pair serves as an electron carrier, and accordingly, it is preferably contained at a certain concentration. The concentration of the redox pair in total is preferably 0.01 mol/L or more, more preferably 0.1 mol/L or more, and particularly preferably 0.3 mol/L or more. In this case, the upper limit is not particularly limited, but is usually approximately 5 mol/L.

### <Counter Electrode>

The counter electrodes 4 and 48 preferably work as a positive electrode in a dye-sensitized solar cell. The counter electrodes 4 and 48 usually have the same configurations as the electrically conductive support 1 or 41, but in a configuration in which strength is sufficiently maintained, a substrate 44 is not necessarily required. A preferred structure of the counter electrodes 4 and 48 is a structure having a high charge collecting effect. At least one of the electrically conductive support 1 or 41 and the counter electrode 4 or 48 should be substantially transparent so that light may reach the photoconductor layers 2 and 42. In the dye-sensitized solar cell of the present invention, the electrically conductive support I or 41 is preferably transparent to allow sunlight to be incident from the side of the electrically conductive support 1 or 41. In this case, the counter electrodes 4 and 48 more preferably have light reflecting properties. As the counter electrodes 4 and 48 of the dye-sensitized solar cell, glass or plastic on which a metal or an electrically conductive oxide is deposited is preferable, and glass on which platinum is deposited is particularly preferable. In the dye-sensitized solar cell, a lateral side of the cell is preferably sealed with a polymer, an adhesive, or the like in order to prevent evaporation of components.

The present invention can be applied to the photoelectric conversion elements and the dye-sensitized solar cells described in, for example, JP4260494B, JP2004-146425A, JP2000-340269A, JP2002-289274A, JP2004-152613A, or JP1997-27352A (JP-H09-27352A). In addition, the present invention can be applied to the photoelectric conversion elements and the dye-sensitized solar cells described in, for example, JP2000-90989A, JP2003-217688A, JP2002-367686A, JP2003-323818A, JP2001-43907A, JP2005-85500A, JP2004-273272A, JP2000-323190A, JP2000-228234A, JP2001-266963A, JP2001-185244A, JP2001-525108A, JP2001-203377A, JP2000-100483A, JP2001-210390A, JP2002-280587A, JP2001-273937A, JP2000-285977A, or JP2001-320068A.

### [Method for Producing Photoelectric Conversion Element and Dye-Sensitized Solar Cell]

The photoelectric conversion element and the dye-sensitized solar cell of the present invention are preferably produced using a dye solution (the dye solution of the present invention) which contains the metal complex dye of the present invention and a solvent.

Such a dye solution is formed of the metal complex dye of the present invention dissolved in a solvent, and may also include a co-adsorbent and other components, if necessary.

Examples of the solvent to be used include the solvents described in JP2001-291534A, but are not particularly limited thereto. In the present invention, an organic solvent is preferable, and an alcohol solvent, an amide solvent, a nitrile solvent, a hydrocarbon solvent, and a mixed solvent of two or more kinds of these solvents are more preferable. As the mixed solvent, a mixed solvent of an alcohol solvent and a solvent selected from an amide solvent, a nitrile solvent, and a hydrocarbon solvent is preferable; a mixed solvent of an alcohol solvent and an amide solvent, a mixed solvent of an alcohol solvent and a hydrocarbon solvent, and a mixed solvent of an alcohol solvent and a nitrile solvent are more preferable; and a mixed solvent of an alcohol solvent and an amide solvent, and a mixed solvent of an alcohol solvent and a nitrile solvent are particularly preferable. Specifically, a mixed solvent of at least one kind of methanol, ethanol, propanol, butanol, and t-butanol, and at least one kind of dimethylformamide and dimethylacetamide, and a mixed solvent of at least one kind of methanol, ethanol, propanol, and t-butanol, and acetonitrile are preferable.

The dye solution preferably contains a co-adsorbent, and as the co-adsorbent, the aforementioned co-adsorbent is preferable. Among those, the compound represented by Formula (CA) is preferable.

Here, the dye solution of the present invention is preferably one in which the concentrations of the metal complex dye and the co-adsorbent have been adjusted so that the dye solution can be used as it is during production of the photoelectric conversion element or the dye-sensitized solar cell. In the present invention, the dye solution of the present invention preferably contains 0.001% to 0.1% by mass of the metal complex dye of the present invention. The amount of the co-adsorbent to be used is as described above.

For the dye solution, it is preferable to adjust the moisture content, and thus in the present invention, the water content is preferably adjusted to 0% to 0.1% by mass.

In the present invention, it is preferable to manufacture a photoconductor layer by making the metal complex dye represented by Formula (I) or a dye including the same on the surface of the semiconductor fine particles, using the dye solution. That is, the photoconductor layer is preferably formed by applying (including a dip method) the dye solution onto the semiconductor fine particles provided on the electrically conductive support, followed by drying and curing.

By further providing a charge transfer layer, a counter electrode, or the like for a light-receiving electrode including the photoconductor layer as manufactured above, the photoelectric conversion element or the dye-sensitized solar cell of the present invention can be obtained.

The dye-sensitized solar cell is produced by connecting an external circuit 6 with the electrically conductive support 1 and the counter electrode 4 of the photoelectric conversion element thus manufactured.

### EXAMPLES

The present invention will be described below in more detail, based on Examples, but the present invention is not limited thereto.

Hereinafter, a method for synthesizing the metal complex dye of the present invention will be described in detail, but the starting materials, the dye intermediates, and the synthesis routes are not limited thereto.

In the present invention, the room temperature means 25°C. Further, Me represents methyl, Et represents ethyl, and TBA represents tetrabutylammonium.

The metal complex dye and the synthesis intermediate synthesized in Example 1 were identified by mass spectrum (MS) measurement and ¹H-NMR measurement. The TBA salt of the synthesized metal complex dye becomes the same mass as the metal complex dye which is electrically neutral by protonization in the MS measurement, and therefore the results of the MS measurement are omitted for the TBA salt.

In addition, the synthesized terpyridine compound (an ethyl esterified product of the ligand LA) was identified by MS measurement and ¹H-NMR measurement.

### Example 1 (Synthesis of Metal Complex Dye)

Metal complex dyes D-1 to D-33 synthesized in the present Example are shown below.

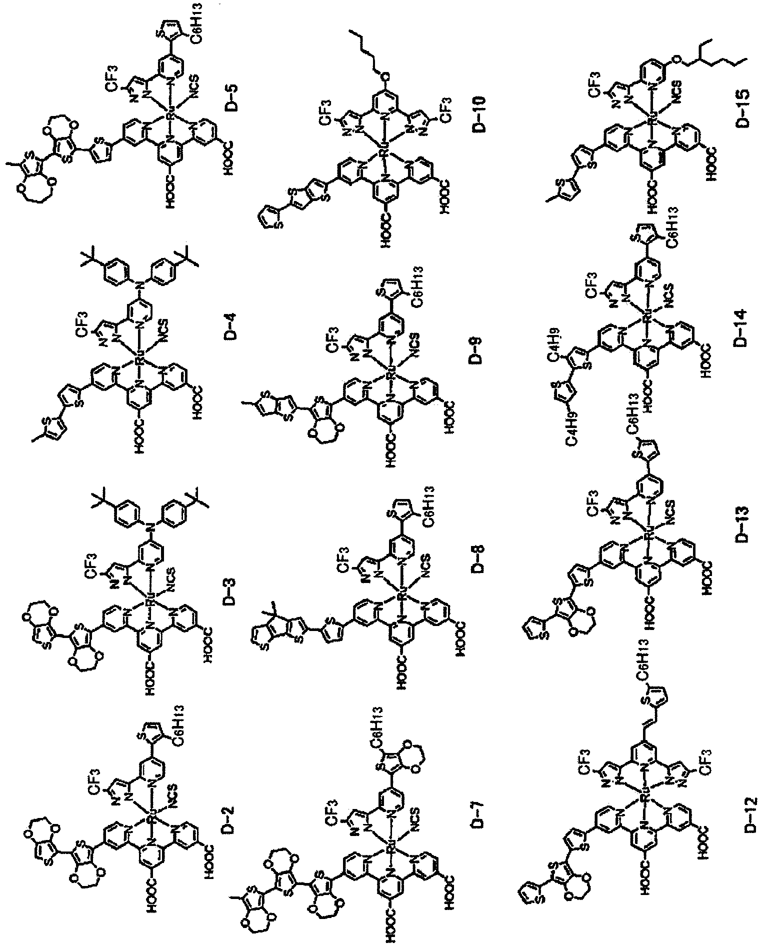

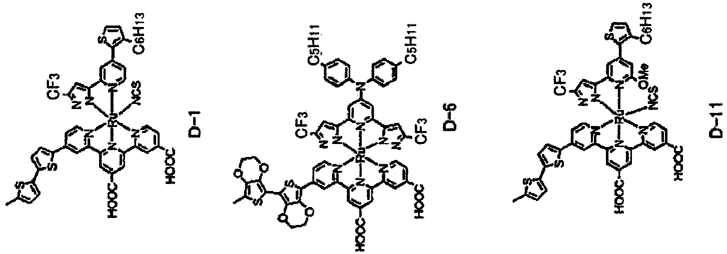

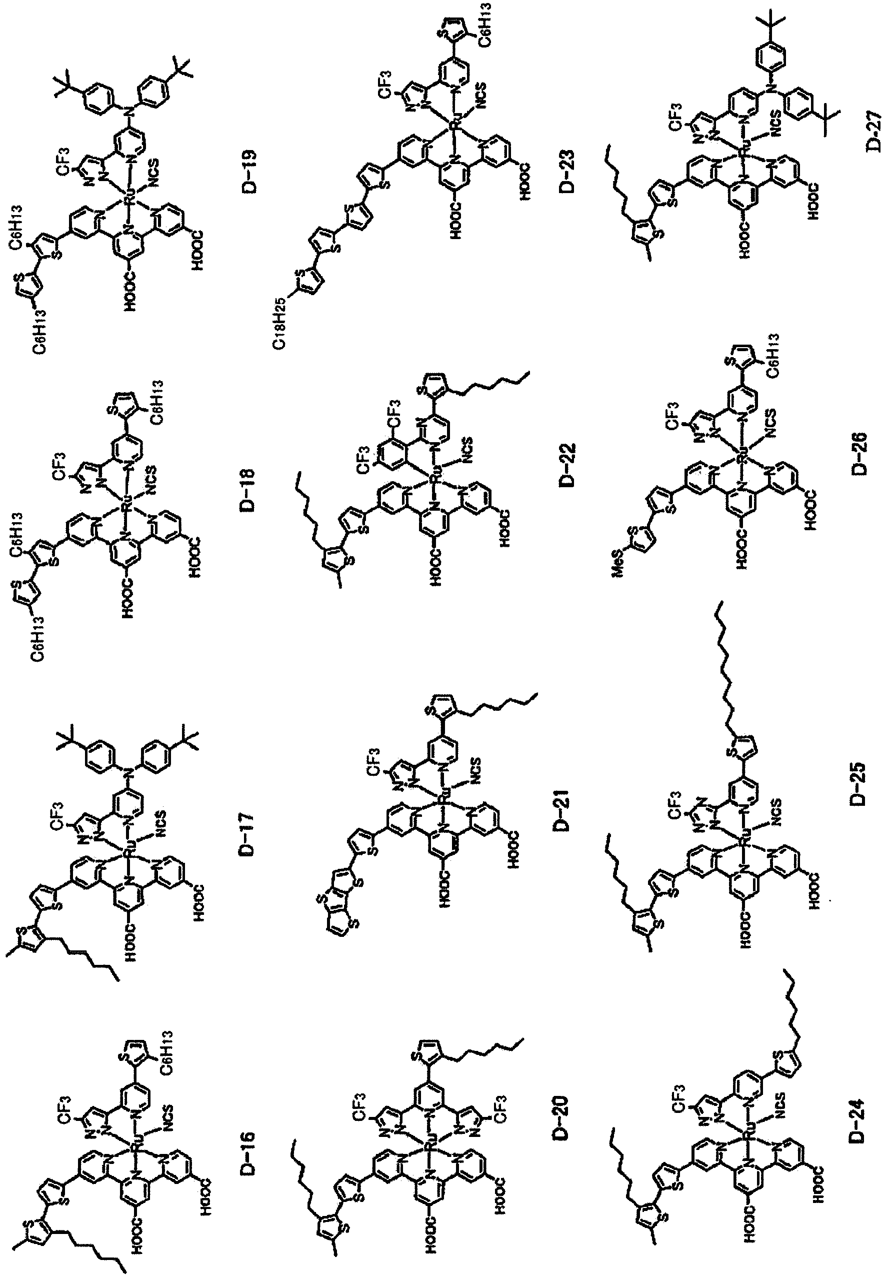

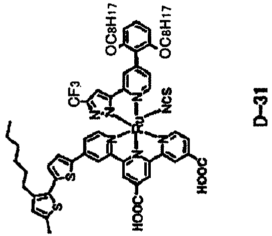

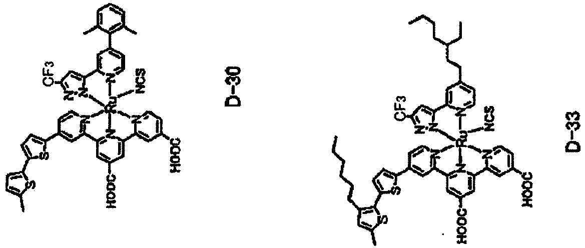

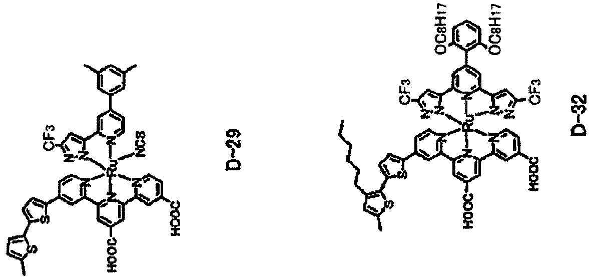

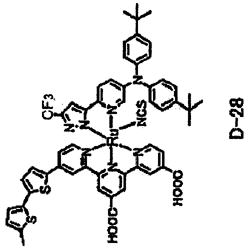

### (Synthesis of Metal Complex Dye (D-1) and Metal Complex Dye (D-ITBA))

According to the following scheme, a metal complex dye (D-1) and a metal complex dye (D-1TBA) were synthesized.

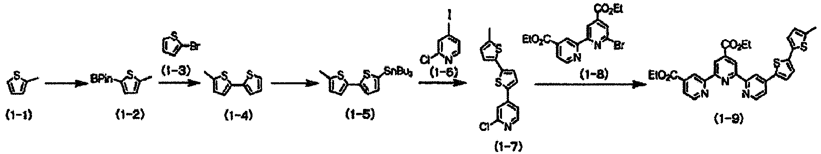

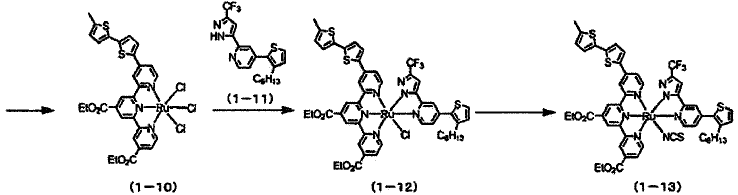

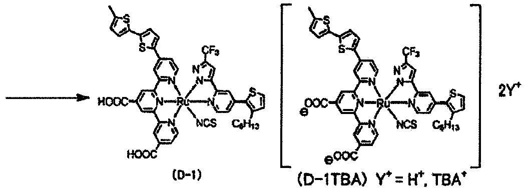

### (i) Synthesis of Compound (1-2)

Tetrahydrofuran (THF, 250 mL) and a compound (1-1) (10 g) were put into a 500 mL 3-neck flask, and the mixture was cooled to -78°C in a nitrogen atmosphere. n-BuLi (a 1.6 M hexane solution, 76 mL) was added thereto, and the mixed liquid was stirred at -78°C for 30 minutes. Thereafter, isopropoxy(pinacol)borane (iPrO-BPin, 45 mL) was added thereto and further stirred at room temperature. The obtained solution was neutralized with ammonium chloride and the reaction product was extracted with ethyl acetate. The organic phase was concentrated to obtain a compound (1-2).

### (ii) Synthesis of Compound (1-4)

THF (100 mL), the compound (1-2) (7.6 g), a compound (1-3) (5 g), 2-dicyclohexylphosphino-2',4',6'-triisopropyl-1,1'-biphenyl[(2-2'-amino-1,1'-biphenyl)]palla dium (II) methanesulfonate (XPhosPdG3, 30.78 g), and K₃PO₄ (48.5 g) were put into a 300 mL 3-neck flask, and the mixture was heated and refluxed in a nitrogen atmosphere. The obtained solution was returned to room temperature, and 20 mL of H₂O was put thereinto. The mixture was filtered through Celite and the reaction product was extracted with ethyl acetate. The organic phase was concentrated and the concentrated residue was purified by silica gel column chromatography to obtain 3 g of a compound (1-4).

### (iii) Synthesis of Compound (1-7)

The compound (1-4) (2 g) and THF (40 mL) were put into a 100 mL 3-neck flask, and the mixture was cooled to -78°C in a nitrogen atmosphere. n-BuLi (a 1.6 M hexane solution, 8 mL) was added thereto and the mixed liquid was stirred at -78°C for 30 minutes. Thereafter, Bu₃SnCl (4.17 g) was added to the mixed liquid and the mixture was stirred at room temperature. The obtained solution was neutralized with ammonium chloride and the reaction product was extracted with ethyl acetate. The organic phase was concentrated to obtain a compound (1-5).

The obtained compound (1-5), a compound (1-6) (1.95 g), Pd(PPh₃)₄ (0.673 g), and toluene (40 mL) were put into a 100 mL eggplant flask, and the mixture was stirred at 90°C in a nitrogen atmosphere. The obtained solution was returned to room temperature and concentrated, and the concentrated residue was purified by silica gel column chromatography to obtain 700 mg of a compound (1-7).

### (iv) Synthesis of Compound (1-9)

The compound (1-7) (600 mg), toluene (20 mL), Pd(PPh₃)₄ (0.5 g), and Me₃SnSnMe₃ (0.53 mL) were put into a 100 mL 3-neck flask, and the mixture was heated and refluxed for 3 hours in a nitrogen atmosphere. The obtained solution was returned to room temperature, 30 mL of H₂O was added thereto, and the mixture was filtered through Celite. The reaction product was extracted with toluene. The organic phase was concentrated and the concentrated residue was dried at 50°C. The obtained Sn product was put into a 100 mL 3-neck flask, toluene (20 mL), Pd(PPh₃)₄ (0.25 g), and a compound (1-8) (0.82 g) were put thereinto, and the mixture was heated and refluxed for 4 hours in a nitrogen atmosphere. The obtained solution was returned to room temperature and concentrated to obtain a crude product. This crude product was recrystallized from methylene chloride and isopropanol to obtain 600 mg of a compound (1-9) which is a diethyl esterified product of a terpyridine compound.

### The compound (1-9) was identified from the following data.

Chemical shift σ (ppm) by ¹H-NMR (400 MHz, solvent: CDCl₃, internal standard substance: tetramethylsilane (TMS)) = 9.20 (1H, m), 9.03 (2H, s), 8.91 (1H, m), 8.85 (1H, m), 8.72 (1H, m), 7.96 (1H, m), 7.59 (1H, m), 7.53 (1H, m), 7.15 (1H, m), 7.08 (1H, m), 6.72 (1H, m), 4.50 (4H, m), 2.52 (3H, s), 1.48 (6H, m)
MS (ESI⁺) m/z: 556.5 ([M+H]⁺)

### (v) Synthesis of Compound (1-10)

The compound (1-9) (0.5 g), ruthenium chloride (0.24 g), and ethanol (10 mL) were put into a 50 mL eggplant flask, and the mixture was heated and refluxed for 3 hours in a nitrogen atmosphere. The precipitate was collected by filtration and washed with ethanol to obtain 0.6 g of a compound (1-10).

### (vi) Synthesis of Compound (1-12)

The compound (1-10) (0.6 g), a compound (1-11) (0.32 g), N,N-dimethylformamide (DMF, 10 mL), and tributylamine (1 mL) were put into a 50 mL eggplant flask, and the mixture was heated at 140°C for 3 hours in a nitrogen atmosphere. The reaction mixture was returned to room temperature and then concentrated, and the concentrated residue was purified by silica gel column chromatography to obtain 0.6 g of a compound (1-12).

### (vii) Synthesis of Compound (1-13)

The compound (1-12) (0.6 g), ammonium thiocyanate (0.42 g), DMF (40 mL), and H₂O (4 mL) were put into a 50 mL eggplant flask, and the mixture was heated at 100°C for 3 hours. The reaction mixture was returned to room temperature and then concentrated, and the concentrated residue was purified by silica gel column chromatography to obtain 0.3 g of a compound (1-13).

### (viii) Synthesis of Metal Complex Dye (D-1)

The compound (1-13) (250 mg), DMF (50 mL), and a 1 N aqueous NaOH solution (1.7 mL) were put into a 100 mL eggplant flask, and the mixture was allowed to undergo a reaction at room temperature. Trifluoromethanesulfonic acid (TfOH) was added to the obtained solution to adjust the pH to 2.9. The precipitate was collected by filtration and washed with ultrapure water to obtain 200 mg of a metal complex dye (D-1).

The metal complex dye (D-1) was identified from the following data.
MS (ESI+) m/z: 1038.5 ([M+H]+)

### (ix) Synthesis of Metal Complex Dye (D-1TBA)

The metal complex dye (D-1) (100 mg) and a 10% tetrabutylammonium hydroxide (TBAOH) methanol solution (0.25 g) were introduced into a 10 mL eggplant flask, and the mixture was allowed to undergo a reaction at room temperature. The obtained solution was concentrated to obtain 100 mg of a metal complex dye (D-1TBA).

In the same manner as for the synthesis of the metal complex dye (D-1) and the metal complex dye (D-1TBA), the metal complex dyes (D-2) to (D-33) and metal complex dyes (D-2TBA) to (D-33TBA) were synthesized, respectively.

The synthesized metal complex dyes (D-2) to (D-33) were identified from the data in Table 1 below.

### [Table 1]

**Table 1**

| Diesterified product of terpyridine compound | MS (ESI⁺) |
|---|---|
| D-2 | MS (ESI⁺) m/z: 1140.2 ([M+H]⁺) |
| D-3 | MS (ESI⁺) m/z: 1253.5 ([M+H]⁺) |
| D-4 | MS (ESI⁺) m/z: 1151.4 ([M+H]⁺) |
| D-5 | MS (ESI⁺) m/z: 1250.3 ([M+H]⁺) |
| D-6 | MS (ESI⁺) m/z: 1370.6 ([M+H]⁺) |
| D-7 | MS (ESI⁺) m/z: 1352.4 ([M+H]⁺) |
| D-8 | MS (ESI⁺) m/z: 1146.3 ([M+H]⁺) |
| D-9 | MS (ESI⁺) m/z: 1152.6 ([M+H]⁺) |
| D-10 | MS (ESI⁺) m/z: 1075.3 ([M+H]⁺) |
| D-11 | MS (ESI⁺) m/z: 1068.3 ([M+H]⁺) |
| D-12 | MS (ESI⁺) m/z: 1265.5 ([M+H]⁺) |
| D-13 | MS (ESI⁺) m/z: 1164.3 ([M+H]⁺) |
| D-14 | MS (ESI⁺) m/z: 1136.4 ([M+H]⁺) |
| D-15 | MS (ESI⁺) m/z: 1000.6 ([M+H]⁺) |
| D-16 | MS (ESI⁺) m/z: 1122.6 ([M+H]⁺) |
| D-17 | MS (ESI⁺) m/z: 1235.5 ([M+H]⁺) |
| D-18 | MS (ESI⁺) m/z: 1192.4 ([M+H]⁺) |
| D-19 | MS (ESI⁺) m/z: 1305.8 ([M+H]⁺) |
| D-20 | MS (ESI⁺) m/z: 1197.4 ([M+H]⁺) |
| D-21 | MS (ESI⁺) m/z: 1136.2 ([M+H]⁺) |
| D-22 | MS (ESI⁺) m/z: 1201.6 ([M+H]⁺) |
| D-23 | MS (ESI⁺) m/z: 1428.9 ([M+H]⁺) |
| D-24 | MS (ESI⁺) m/z: 1122.5 ([M+H]⁺) |
| D-25 | MS (ESI⁺) m/z: 1123.3 ([M+H]⁺) |
| D-26 | MS (ESI⁺) m/z: 1070.8 ([M+H]⁺) |
| D-27 | MS (ESI⁺) m/z: 1235.6 ([M+H]⁺) |
| D-28 | MS (ESI⁺) m/z: 1151.4 ([M+H]⁺) |
| D-29 | MS (ESI⁺) m/z: 976.3 ([M+H]⁺) |
| D-30 | MS (ESI⁺) m/z: 976.1 ([M+H]⁺) |
| D-31 | MS (ESI⁺) m/z: 1288.8 ([M+H]⁺) |
| D-32 | MS (ESI⁺) m/z: 1363.5 ([M+H]⁺) |
| D-33 | MS (ESI⁺) m/z: 1082.3 ([M+H]⁺) |

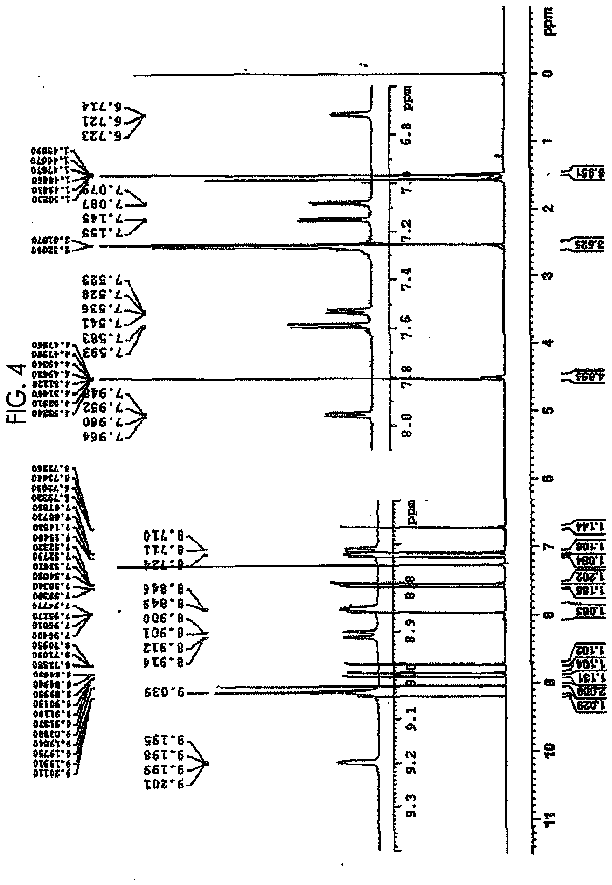

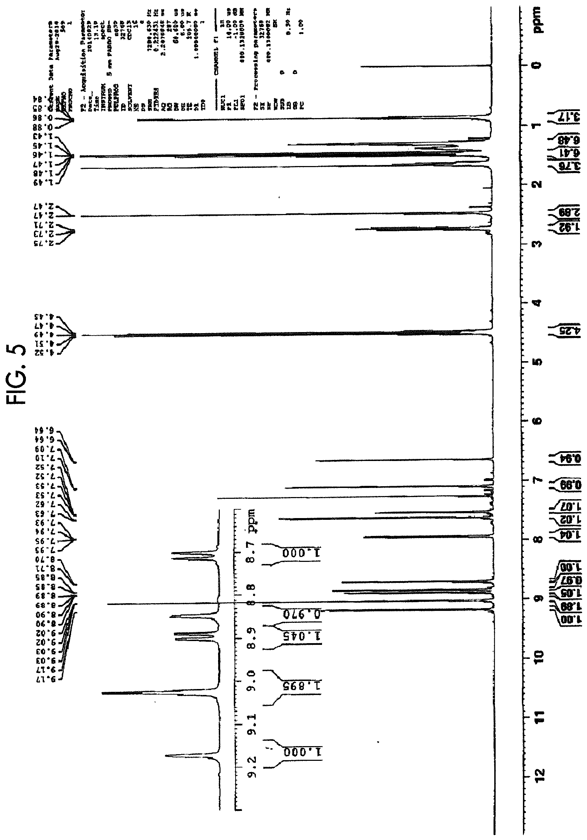

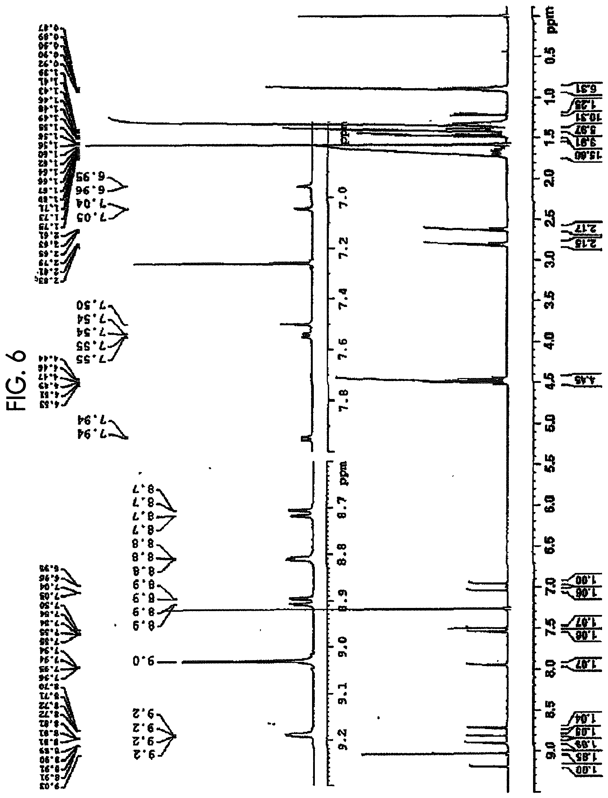

In the synthesis of the metal complex dyes, the synthesized AC-1 to AC-6 which are each a diethyl esterified product of a terpyridine compound were identified from the date in Table 2 below. Further, the terpyridine compounds AC-1, AC-5, and AC-6 were also each identified from the ¹H-NMR data shown in Figs. 4 to 6. The ¹H-NMR of AC-1 (Fig. 4), AC-5 (Fig. 5), and AC-6 (Fig. 6) which are each a diethyl esterified product of a terpyridine compound was measured with a proton resonance frequency of 400 MHz, each using a CDCl₃ solvent and tetramethylsilane (TMS) as an internal standard substance.

For example, AC-1 which is a diethyl esterified product of a terpyridine compound is the compound (1-9), and was used for the synthesis of the metal complex dye (D-1) and the like.

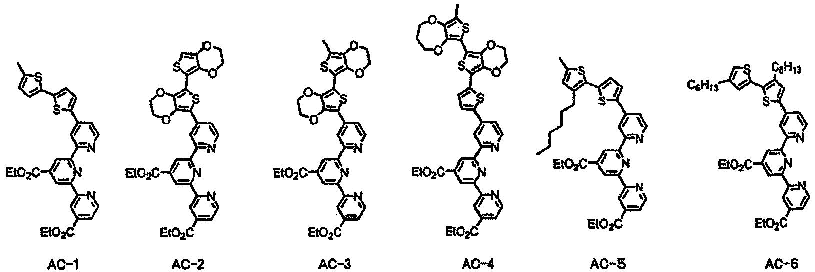

### [Table 2]

**Table 2**

| Diesterified product of terpyridine compound | MS (ESI⁺) |
|---|---|
| AC-1 | MS (ESI⁺) m/z: 556.8 ([M+H]⁺) |
| AC-2 | MS (ESI⁺) m/z: 658.9 ([M+H]⁺) |
| AC-3 | MS (ESI⁺) m/z: 672.7 ([M+H]⁺) |
| AC-4 | MS (ESI⁺) m/z: 768.9 ([M+H]⁺) |
| AC-5 | MS (ESI⁺) m/z: 641.1 ([M+H]⁺) |
| AC-6 | MS (ESI⁺) m/z: 710.9 ([M+H]⁺) |

### (Measurement of Visible Absorption Spectrum)

The visible absorption spectrum of the synthesized metal complex dye (D-1) was measured.

The metal complex dye (D-1) was dissolved in a TBAOH/methanol solution having a concentration of 340 mmol/L to prepare a TBAOH/methanol solution having a concentration of the metal complex dye (D-1) of 17 µmol/L. This solution was used to measure the light absorption spectrum of the metal complex dye (D-1). "UV-3600" (manufactured by Shimadzu Corporation) was used as a measurement device.

In addition, in the same manner, the visible absorption spectra of the metal complex dyes (D-16) to (D-19) and (D-24) were measured.

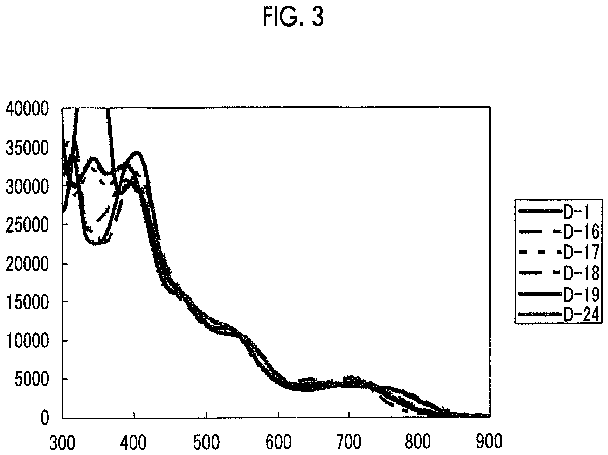

The obtained absorption spectra are shown in Fig. 3. In the absorption spectra of Fig. 3, the vertical axis indicates a molar light absorption coefficient ε (L/mol·cm).

As shown in Fig. 3, it could be confirmed that all of the metal complex dyes (D-1), (D-16) to (D-19), and (D-24) had wide bottoms of the absorption peaks to the long-wavelength region with more than a wavelength of 700 nm. As such, according to the obtained visible absorption spectrum, it could be seen that the metal complex dyes all showed increased absorbance with respect to Example 2 (A-2: Fig. 3) of US2012/0247561A.

### Example 2 (Production of Dye-Sensitized Solar Cell)

By using each of the metal complex dyes (D-1) to (D-33) and (D-1TBA) to (D-33TBA) synthesized in Example 1 or the following comparative compounds (c-1) to (c-4), the dye-sensitized solar cell 20 (in a scale of 5 mm × 5 mm) shown in Fig. 2 was produced. The production was carried out in accordance with the procedure shown below. The following performance of each of the produced dye-sensitized solar cells 20 was evaluated. The results are shown in Tables 3-1 and 3-2.

### (Manufacture of Light-Receiving Electrode Precursor [A])

An electrically conductive support 41 was manufactured in which a fluorine-doped SnO₂ electrically-conductive film (transparent electrically-conductive film 43, film thickness of 500 nm) was formed on a glass substrate (substrate 44, thickness of 4 mm). Further, a titania paste "18NR-T" (manufactured by DyeSol) was screen-printed on the SnO₂ electrically-conductive film, followed by drying at 120°C. Then, the dried titania paste "18NR-T" was screen-printed again, followed by drying at 120°C for 1 hour. Thereafter, the dried titania paste was calcined at 500°C in air to form a semiconductor layer 45 (layer thickness; 10 µm). Further, a titania paste "18NR-AO" (manufactured by DyeSol) was screen-printed on this semiconductor layer 45, followed by drying at 120°C for 1 hour. Then, the dried titania paste was calcined at 500°C to form a light-scattering layer 46 (layer thickness; 5 µm) on the semiconductor layer 45.

Thus, a photoconductor layer 42 (the area of the light-receiving surface; 5 mm × 5 mm, layer thickness; 15 µm, a metal complex dye being not carried) was formed on the SnO₂ electrically-conductive film, thereby manufacturing a light-receiving electrode precursor [A] not carrying a metal complex dye.

### (Manufacture of Light-Receiving Electrode Precursor [B])

An electrically conductive support 41 was manufactured in which a fluorine-doped SnO₂ electrically-conductive film (transparent electrically-conductive film 43, film thickness; 500 nm) was formed on a glass substrate (substrate 44, thickness of 4 mm). Further, a titania paste "18NR-T" (manufactured by DyeSol) was screen-printed on this SnO₂ electrically-conductive film, followed by drying at 120°C. Then, the dried titania paste was calcined at 500°C in air to form a semiconductor layer 45 (the area of the light-receiving surface; 5 mm × 5 mm, layer thickness; 6 µm).

Thus, a photoconductor layer 42 (the area of the light-receiving surface; 5 mm × 5 mm, layer thickness; 6 µm, a metal complex dye being not carried) not having the light-scattering layer 46 provided thereon was formed on the SnO₂ electrically-conductive film, thereby manufacturing a light-receiving electrode precursor [B] not carrying a metal complex dye.

### (Adsorption of Dye)

Next, each of the metal complex dyes ((D- 1) to (D-33) and (D-1TBA) to (D-33TBA)) that had been synthesized in Example 1 was carried on the photoconductor layer 42 not carrying a metal complex dye, in the following manner. First, each of the metal complex dyes was dissolved in a mixed solvent of t-butanol and acetonitrile at 1:1 (volume ratio) to have a concentration of 2 × 10⁻⁴ mol/L. Further, 30 mol of cholic acid as a co-adsorbent was added per mol of the metal complex dye to prepare each of dye solutions. Next, the light-receiving electrode precursor [A] was immersed in each of the dye solutions at 25°C for 20 hours, and dried after pulling out from the dye solution.

Thus, each of light-receiving electrodes 40 having the respective metal complex dyes carried on the light-receiving electrode precursor [A] was manufactured.

Each of the metal complex dyes was similarly carried on the light-receiving electrode precursor [B] to manufacture each of light-receiving electrodes 40 having the respective metal complex dyes carried on the light-receiving electrode precursor [B].

### (Assembly of Dye-Sensitized Solar Cell)

Then, a platinum electrode (thickness of a thin film with Pt; 100 nm) having the same shape and size as those of the electrically conductive support 41 was manufactured as a counter electrode 48. Further, 0.1 M (mol/L) of iodine, 0.1 M of lithium iodide, 0.5 M of 4-t-butylpyridine, and 0.6 M of 1,2-dimethyl-3-propylimidazolium iodide were dissolved in acetonitrile to prepare a liquid electrolyte as an electrolytic solution. Further, a Spacer-S "SURLYN" (trade name, manufactured by DuPont), which has a shape matching to the size of the photoconductor layer 42, was prepared.

Each of the light-receiving electrodes 40 manufactured as above and the counter electrode 48 were arranged to face each other through the spacer-S and thermally compressed, and then the liquid electrolyte was filled from the inlet for the electrolytic solution between the photoconductor layer 42 and the counter electrode 48, thereby forming a charge transfer layer 47. The outer periphery and the inlet for the electrolytic solution of thus manufactured cell were sealed and cured using RESIN XNR-5516 (manufactured by Nagase ChemteX Corporation), thereby producing each of dye-sensitized solar cells (Sample Nos. 1 to 33).

The dye-sensitized solar cells with the respective Sample Nos. encompass two kinds of dye-sensitized solar cells, with ones using electrically neutral metal complex dyes (D-1 to D-33) and the others using metal complex dyes (D-1TBA to D-33TBA) of TBA salts.

Furthermore, in the dye-sensitized solar cells with the respective Sample Nos., the dye-sensitized solar cells using the electrically neutral metal complex dyes each encompass two kinds of the dye-sensitized solar cells produced using the light-receiving electrode precursor [A] ("A" is attached to the Sample No.) and the dye-sensitized solar cells produced using the light-receiving electrode precursor [B] ("B" is attached to the Sample No.). Similarly, the dye-sensitized solar cell using the metal complex dye of the TBA salt encompasses two kinds of the dye-sensitized solar cell produced using the light-receiving electrode precursor [A] and the dye-sensitized solar cell produced using the light-receiving electrode precursor [B].

For comparison, dye-sensitized solar cells (Sample Nos. c1 to c4) were produced in the same manner as for the production of the dye-sensitized solar cells, except that each of the following metal complex dyes (c-1) to (c-4) was used instead of the metal complex dye synthesized in Example 1 in the production of the dye-sensitized solar cell.

The metal complex dye (c-1) is the compound "Dye-604" described in JP2013-67773A. The metal complex dye (c-2) is the compound described in [0042] of JP2012-36237A. The metal complex dye (c-3) is the compound "D-9" described in JP2013-229285A. The metal complex dye (c-4) is the compound "Example 2 (A-2)" described in US2012/0247561A.

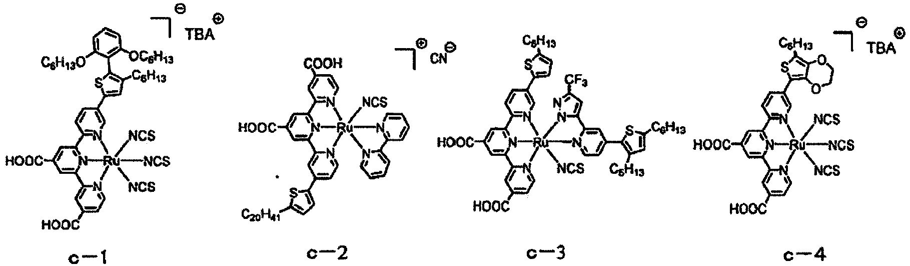

### <Test of Photoelectric Conversion Efficiency>

Each of the produced dye-sensitized solar cells was used to carry out a cell characteristic test. The cell characteristic test was carried out by irradiating artificial sunlight of 1,000 W/m² from a xenon lamp through an AM1.5 filter, using a solar simulator (WXS-85H manufactured by WACOM). The current-voltage characteristics were measured using an I-V tester to determine the photoelectric conversion efficiency.

### (Conversion Efficiency (A))

For each of the dye-sensitized solar cells (Sample Nos. 1A to 33A and c1A to c4A) produced using the light-receiving electrode precursors [A] in the dye-sensitized solar cells with the respective Sample Nos., the photoelectric conversion efficiency (referred to as conversion efficiency (A)) was measured as described above. The measured conversion efficiency (A) was evaluated. For the evaluation, the conversion efficiency (S_{A}) of the dye-sensitized solar cell (Sample Nos. c1A) produced by using the light-receiving electrode precursor [A] was used as a standard.

In the evaluation criteria of the conversion efficiency (A), "A" and "B" are the acceptable levels in the present test, with "A" being preferable.

### (Evaluation Criteria for Conversion Efficiency (A))

The conversion efficiency (A) was evaluated as follows in comparison with that of the conversion efficiency (S_{A}).
A: More than 1.2 times
B: More than 1.1 times and 1.2 times or less
C: More than 1.0 time and 1.1 times or less
D: 1.0 time or less

### (Conversion Efficiency (B))

For each of the dye-sensitized solar cells (Sample Nos. 1B to 33B and c1B to c4B) produced using the light-receiving electrode precursors [B] in the dye-sensitized solar cells with the respective Sample Nos., the photoelectric conversion efficiency (referred to as conversion efficiency (B)) was as described above. The measured conversion efficiency (B) was evaluated. For the evaluation, the conversion efficiency (S_{A}) of the dye-sensitized solar cell (Sample Nos. c1A) produced by using the light-receiving electrode precursor [A] was used as a standard.

In the evaluation criteria of the conversion efficiency (B), "A" and "B" are the acceptable levels in the present test, with "A" being preferable.

### (Evaluation Criteria for Conversion Efficiency (B))

The conversion efficiency (B) was evaluated as follows in comparison with that of the conversion efficiency (S_{A}).
A: More than 1.1 times
B: More than 1.0 time and 1.1 times or less
C: More than 0.9 times and 1.0 time or less
D: 0.9 times or less

### <Evaluation of Durability>

The heat cycle test was carried out for evaluation on durability (thermal deterioration), using each of the dye-sensitized solar cells (Sample Nos. 1A to 33A and c1A to c4A) produced using the light-receiving electrode precursors [A] in the dye-sensitized solar cells with the respective Sample Nos.

Each of the dye-sensitized solar cells was alternately introduced into a freezer at -10°C and a constant-temperature tank at 50°C every 12 hours so as to repeat cooling and heating (heat cycle test). The current was measured for the dye-sensitized solar cells before the heat cycle test and the dye-sensitized solar cell at 72 hours after the heat cycle test, respectively. The value determined by dividing the current value (short-circuit current density) determined from the current-voltage characteristic measurement in the dye-sensitized solar cell at 72 hours after the heat cycle test by the current value (short-circuit current density) measured in the dye-sensitized solar cells before the heat cycle test was taken as a current holding ratio. By the current holding ratios thus obtained, the durability was evaluated according to the following criteria.

In the evaluation criteria for the durability, "A" and "B" are the acceptable levels in the present test, with "A" being preferable.

### (Evaluation Criteria for Durability)

A: 0.9 times or more
B: Less than 0.9 times and 0.8 times or more
C: Less than 0.8 times and 0.7 times or more
D: Less than 0.7 times

### [Table 3]

**Table 3-1**

| Sample Nos. | Metal complex dye | Conversion efficiency (A) | Conversion efficiency (B) | Durability | Note |
|---|---|---|---|---|---|
| 1 | D-1 | A | A | A | The present invention |
| | D-1TBA | A | A | B | The present invention |
| 2 | D-2 | A | A | A | The present invention |
| | D-2TBA | A | A | B | The present invention |
| 3 | D-3 | A | A | A | The present invention |
| | D-3TBA | A | A | B | The present invention |
| 4 | D-4 | A | A | A | The present invention |
| | D-4TBA | A | A | B | The present invention |
| 5 | D-5 | A | A | A | The present invention |
| | D-5TBA | A | A | B | The present invention |
| 6 | D-6 | A | A | A | The present invention |
| | D-6TBA | A | A | B | The present invention |
| 7 | D-7 | A | A | A | The present invention |
| | D-7TBA | A | A | B | The present invention |
| 8 | D-8 | A | A | A | The present invention |
| | D-8TBA | A | A | B | The present invention |
| 9 | D-9 | A | A | A | The present invention |
| | D-9TBA | A | A | B | The present invention |
| 10 | D-10 | A | A | A | The present invention |
| | D-10TBA | A | A | B | The present invention |
| 11 | D-11 | A | A | A | The present invention |
| | D-11TBA | A | A | B | The present invention |
| 12 | D-12 | A | A | A | The present invention |
| | D-12TBA | A | A | B | The present invention |
| 13 | D-13 | A | A | A | The present invention |
| | D-13TBA | A | A | B | The present invention |
| 14 | D-14 | A | A | A | The present invention |
| | D-14TBA | A | A | B | The present invention |
| 15 | D-15 | A | A | A | The present invention |
| | D-15TBA | A | A | B | The present invention |
| 16 | D-16 | A | A | A | The present invention |
| | D-16TBA | A | A | B | The present invention |
| 17 | D-17 | A | A | A | The present invention |
| | D-17TBA | A | A | B | The present invention |
| 18 | D-18 | A | A | A | The present invention |
| | D-18TBA | A | A | B | The present invention |
| 19 | D-19 | A | A | A | The present invention |
| | D-19TBA | A | A | B | The present invention |
| 20 | D-20 | A | A | A | The present invention |
| | D-20TBA | A | A | B | The present invention |

### [Table 4]

**Table 3-2**

| Sample Nos. | Metal complex dye | Conversion efficiency (A) | Conversion efficiency (B) | Durability | Note |
|---|---|---|---|---|---|
| 21 | D-21 | A | A | A | The present invention |
| | D-21TBA | A | A | B | The present invention |
| 22 | D-22 | A | A | A | The present invention |
| | D-22TBA | A | A | B | The present invention |
| 23 | D-23 | A | B | A | The present invention |
| | D-23TBA | A | B | B | The present invention |
| 24 | D-24 | A | A | A | The present invention |
| | D-24TBA | A | A | B | The present invention |
| 25 | D-25 | A | A | A | The present invention |
| | D-25TBA | A | A | B | The present invention |
| 26 | D-26 | A | A | A | The present invention |
| | D-26TBA | A | A | B | The present invention |
| 27 | D-27 | A | A | A | The present invention |
| | D-27TBA | A | A | B | The present invention |
| 28 | D-28 | A | A | A | The present invention |
| | D-28TBA | A | A | B | The present invention |
| 29 | D-29 | A | A | A | The present invention |
| | D-29TBA | A | A | B | The present invention |
| 30 | D-30 | A | A | A | The present invention |
| | D-30TBA | A | A | B | The present invention |
| 31 | D-31 | A | A | A | The present invention |
| | D-31TBA | A | A | B | The present invention |
| 32 | D-32 | A | A | A | The present invention |
| | D-32TBA | A | A | B | The present invention |
| 33 | D-33 | A | A | A | The present invention |
| | D-33TBA | A | A | B | The present invention |
| c1 | c-1 | D | C | C | Comparative Example |
| c2 | c-2 | C | C | C | Comparative Example |
| c3 | c-3 | C | C | B | Comparative Example |
| c4 | c-4 | C | C | C | Comparative Example |

From the results of Tables 3-1 and 3-2, it can be seen as follows.

All of Sample Nos. 1 to 33 (the present invention) used metal complex dyes (D-1 to D-33) having a combination of the ligand LA represented by Formula (AL-1) and a bidentate or tridentate ligand LD coordinating to the metal ion M through one nitrogen atom and an anion of at least one atom as a ligand. In all of the photoelectric conversion elements and the dye-sensitized solar cells of the present invention (Sample Nos. 1 to 33) in which these metal complex dyes (D-1 to D-33) were carried on semiconductor fine particles, the conversion efficiency (A) and the conversion efficiency (B) were both high, and the current holding ratio was also high. In particular, it could be seen that when n in Formula (AL-1) is 2 or 3, excellent conversion efficiency (B) is obtained. Further, the metal complex dye of the present invention could provide the same results whether it was electrically neutral or in the form of a TBA salt.

Moreover, the metal complex dye of the present invention could be suitably used as a sensitizing dye of the photoelectric conversion element and the dye-sensitized solar cell of the present invention. The dye solution of the present invention, containing the metal complex dye of the present invention and a solvent, could be suitably used for the preparation of semiconductor fine particles carrying the metal complex dye of the present invention. In addition, the terpyridine compound of the present invention was suitable as a ligand of the metal complex dye of the present invention, and in particular, an esterified product thereof was suitable as a ligand precursor of the metal complex dye of the present invention.

To the contrary, in a case of using the metal complex dyes not having the ligand LA represented by Formula (AL-1), the conversion efficiency and the durability were not sufficient.

That is, the photoelectric conversion elements and the dye-sensitized solar cells of Sample Nos. c1 and c4 used the metal complex dyes having neither the ligand LA represented by Formula (AL-1) nor the ligand LD. Further, the photoelectric conversion elements and the dye-sensitized solar cells of Sample No. c2 used the metal complex dyes having a ligand with one thiophene ring group and bipyridine. In addition, the photoelectric conversion element and the dye-sensitized solar cell of Sample Nos. c3 used the metal complex dye having a ligand in which one thiophene ring group is bonded at the 3-position with respect to a coordinating atom. However, none of the conversion efficiency and the current holding ratio reached the acceptable level.

Although the present invention has been described with reference to embodiments, it is not intended that the present invention is not limited by any of the details of the description unless otherwise specified, but should rather be construed broadly within the scope of the present invention as set out in the accompanying claims.

The present application claims the priorities based on JP2014-140079 filed on July 7, 2014, and JP2015-039390 filed on February 27, 2015.

### Explanation of References

1, 41: ELECTRICALLY CONDUCTIVE SUPPORT
2, 42: PHOTOCONDUCTOR LAYER
21: DYE
22: SEMICONDUCTOR FINE PARTICLES
3, 47: CHARGE TRANSFER LAYER
4, 48: COUNTER ELECTRODE
5, 40: LIGHT-RECEIVING ELECTRODE
6: EXTERNAL CIRCUIT
10: PHOTOELECTRIC CONVERSION ELEMENT
100: SYSTEM IN WHICH PHOTOELECTRIC CONVERSION ELEMENT IS APPLIED TO CELL USES
M: OPERATING MEANS (FOR EXAMPLE, ELECTRIC MOTOR)
20: DYE-SENSITIZED SOLAR CELL
43: TRANSPARENT ELECTRICALLY-CONDUCTIVE FILM
44: SUBSTRATE
45: SEMICONDUCTOR LAYER
46: LIGHT-SCATTERING LAYER
S: SPACER

## Claims

1. A photoelectric conversion element comprising:
an electrically conductive support;
a photoconductor layer including an electrolyte;
a charge transfer layer including an electrolyte; and
a counter electrode,
wherein the photoconductor layer has semiconductor fine particles carrying a metal complex dye represented by the following Formula (I),
Formula (I) M(LA)(LD)(LX)_{mX}·(CI)_{mY}
in the formula,
M represents a metal ion,
LA represents a tridentate ligand represented by the following Formula (AL-1),
LD represents a bidentate ligand, or a tridentate ligand different from LA, and at least one of coordinating atoms in LD bonded to the metal ion M is a nitrogen atom, and at least one of the coordinating atoms is an anion,
LX represents a monodentate ligand, and when LD is a bidentate ligand, mX represents 1, and when LD is a tridentate ligand, mX represents 0,
CI represents a counterion necessary for neutralizing the charge of the metal complex dye, and
mY represents an integer of 0 to 3; 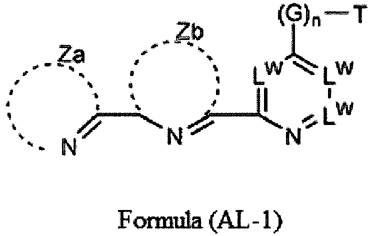 in the formula,
Za and Zb each independently represent a non-metal atomic group necessary for forming a 5- or 6-membered ring, in which at least one of rings formed by Za and Zb, respectively, has one or more acidic groups, L^{W}'s each independently represent a nitrogen atom or CR^{W}, and R^{W} represents a hydrogen atom or a substituent,
G represents a ring group represented by any one of the following Formulae (X-1) to (X-3),
n represents an integer of 2 to 7,
T represents a hydrogen atom or a substituent, and
a -(G)n-T group does not have the acidic group and an amino group; 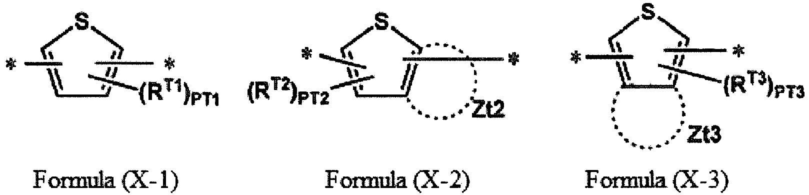 in the formulae,
Zt2 and Zt3 each represent a non-metal atomic group necessary for forming a fused ring with a thiophene ring in Formula (X-2) or (X-3),
R^{T1}, R^{T2}, and R^{T3} each independently represent a substituent,
PT1 represents an integer of 0 to 2,
PT2 and PT3 each independently represent an integer of 0 or more, and is no more than the number of hydrogen atoms when the group represented by Formula (X-2) or (X-3) is unsubstituted, and
* represents a binding position to the ring including L^{W}, or another G or T.

2. The photoelectric conversion element according to claim 1, wherein the ring group represented by Formula (X-1) is a ring group represented by any one of the following Formulae (X-1a) to (X-1c), 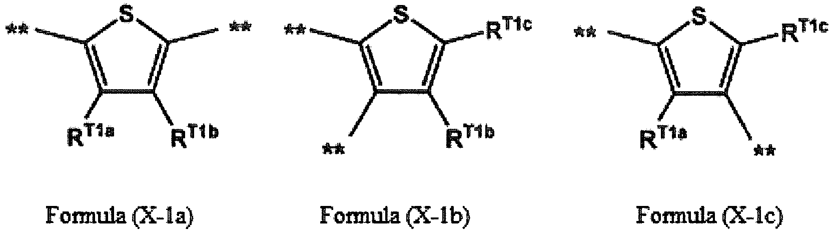 in the formulae, R^{T1a} to R^{T1c} each independently represent a hydrogen atom or a substituent, and ** represents a binding position to the ring including L^{W}, or another G or T.

3. The photoelectric conversion element according to claim 1, wherein the ring group represented by Formula (X-2) is a ring group represented by any one of the following Formulae (X-2a) to (X-2e), 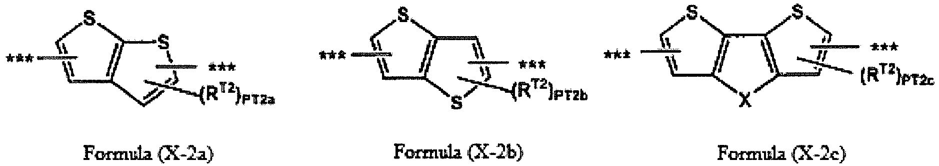 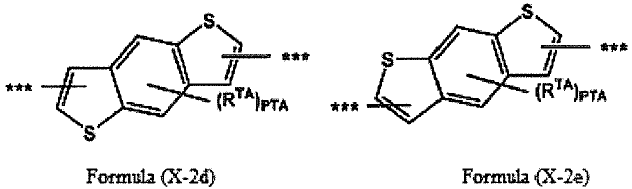 in the formulae,
X represents -O-, -S-, -NR^{X2c}-, -C(R^{X2c})₂-, -(R^{X2c})C=C(R^{X2c})-, or -Si(R^{X2c})₂-,
R^{X2c} represents a hydrogen atom or a substituent,
R^{T2} and R^{TA} each independently represent a substituent,
PT2a to PT2c each independently represent an integer of 0 to 2,
PTA's each represent an integer of 0 to 4, and
*** represents a binding position to the ring including L^{W}, or another G or T.

4. The photoelectric conversion element according to claim 1, wherein the ring group represented by Formula (X-3) is a ring group represented by the following Formula (X-3a) or (X-3b), 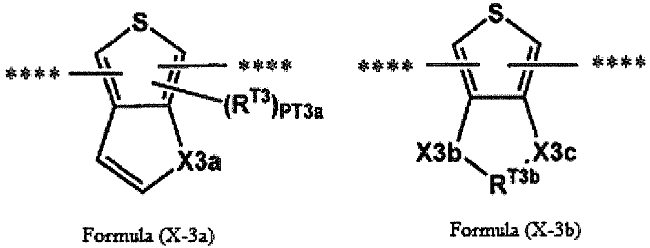 in the formula,
X3a to X3c each independently represent -O- or -S-,
R^{T3b} represents an alkylene group,
R^{T3} represents a substituent,
PT3a represents an integer of 0 to 2, and
**** represents a binding position to the ring including L^{W}, or another G or T.

5. The photoelectric conversion element according to any one of claims 1 to 4, wherein LA is a tridentate ligand represented by the following Formula (AL-2), 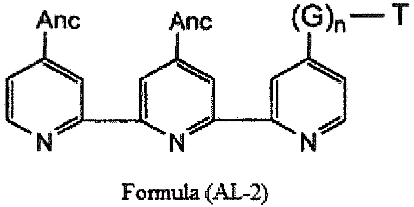 in the formula,
Anc represents an acidic group, and
G, T, and n have the same definitions as G, T, and n, respectively, in Formula (AL-1).

6. The photoelectric conversion element according to any one of claims 1 to 5, wherein LD is a bidentate ligand represented by any one of the following Formulae (2L-1) to (2L-4), 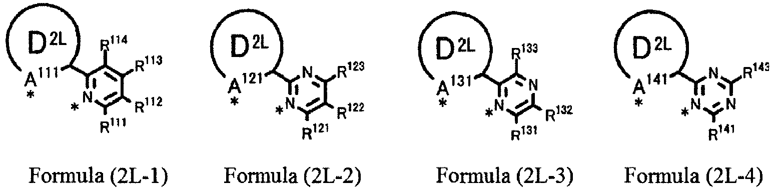 in the formulae,
the ring D^{2L} represents an aromatic ring,
A¹¹¹ to A¹⁴¹ each independently represent an anion of a nitrogen atom or an anion of a carbon atom,
R¹¹¹ to R¹⁴³ each independently represent a hydrogen atom or a substituent not having an acidic group, and
* represents a coordinating position to the metal ion M.

7. The photoelectric conversion element according to any one of claims 1 to 5, wherein LD is a tridentate ligand represented by any one of the following Formulae (3L-1) to (3L-4), 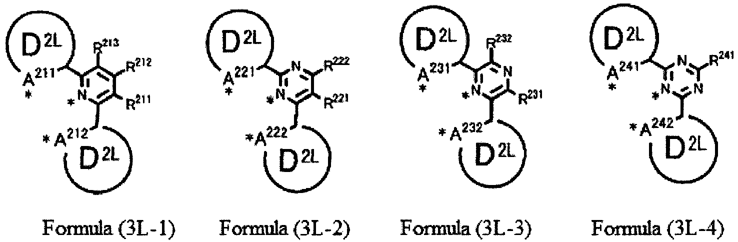 in the formulae,
the ring D^{2L} represents an aromatic ring,
A²¹¹ to A²⁴² each independently represent a nitrogen atom or a carbon atom, in which at least one of each of A²¹¹ and A²¹², A²²¹ and A²²², A²³¹ and A²³², and A²⁴¹ and A²⁴² is an anion,
R²¹¹ to R²⁴¹ each independently represent a hydrogen atom or a substituent not having an acidic group, and
* represents a coordinating position to the metal ion M.

8. The photoelectric conversion element according to any one of claims 1 to 7, wherein the metal complex dye represented by Formula (I) is represented by the following Formula (I-1) or (I-2), 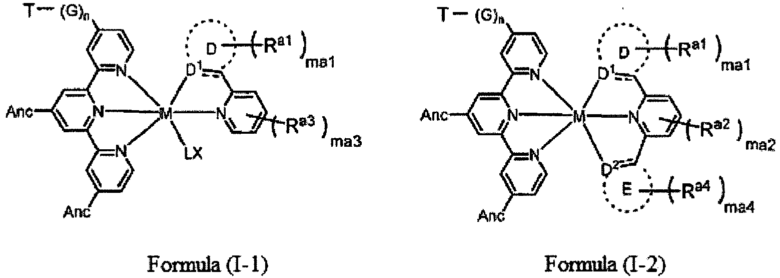 in the formulae,
M and LX have the same definitions as M and LX, respectively, in Formula (I),
G, T, and n have the same definitions as G, T, and n, respectively, in Formula (AL-1), Anc represents an acidic group,
the ring D and the ring E each independently represent a 5- or 6-membered aromatic ring,
D¹ and D² each independently represent an anion of a carbon atom or an anion of a nitrogen atom, in which a bond between D¹ and D² in the ring D and the ring E, and a carbon atom bonded to a pyridine ring is a single bond or a double bond,
R^{a1} to R^{a4} each independently represent a substituent, and
ma1, ma2, and ma4 each independently represent an integer of 0 to 3, ma3 represents an integer of 0 to 4, and when ma1 to ma4 each represent an integer of 2 or more, a plurality of R^{a1}'s to R^{a4}'s may each be bonded to each other to form a ring.

9. The photoelectric conversion element according to claim 8, wherein the ring D and the ring E are each independently a pyrazole ring, a triazole ring, or a benzene ring.

10. A dye-sensitized solar cell comprising the photoelectric conversion element according to any one of claims 1 to 9.

11. A metal complex dye represented by the following Formula (I),
Formula (I) M(LA)(LD)(LX)_{mX}·(CI)_{mY}
in the formula,
M represents a metal ion,
LA represents a tridentate ligand represented by the following Formula (AL-1),
LD represents a bidentate ligand, or a tridentate ligand different from LA, and at least one of coordinating atoms in LD bonded to the metal ion M is a nitrogen atom, and at least one of the coordinating atoms is an anion,
LX represents a monodentate ligand, and when LD is a bidentate ligand, mX represents 1, and when LD is a tridentate ligand, mX represents 0,
CI represents a counterion necessary for neutralizing the charge of the metal complex dye, and
mY represents an integer of 0 to 3; 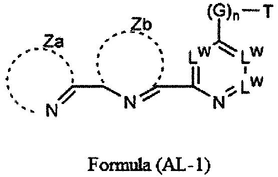 in the formula,
Za and Zb each independently represent a non-metal atomic group necessary for forming a 5- or 6-membered ring, in which at least one of rings formed by Za and Zb, respectively, has one or more acidic groups,
L^{W}'s each independently represent a nitrogen atom or CR^{W}, and R^{W} represents a hydrogen atom or a substituent,
G represents a ring group represented by any one of the following Formulae (X-1) to (X-3),
n represents an integer of 2 to 7,
T represents a hydrogen atom or a substituent, and
a -(G)n-T group does not have the acidic group and an amino group; 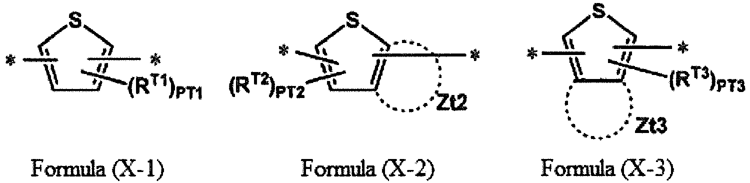 in the formulae,
Zt2 and Zt3 each represent a non-metal atomic group necessary for forming a fused ring with a thiophene ring in Formula (X-2) or (X-3),
R^{T1}, R^{T2}, and R^{T3} each independently represent a substituent,
PT1 represents an integer of 0 to 2,
PT2 and PT3 each independently represent an integer of 0 or more, and is no more than the number of hydrogen atoms when the group represented by Formula (X-2) or (X-3) is unsubstituted, and
* represents a binding position to the ring including L^{W}, or another G or T.

12. A dye solution comprising the metal complex dye according to claim 11 and a solvent.

13. A terpyridine compound or an esterified product thereof represented by the following Formula (AL-2), 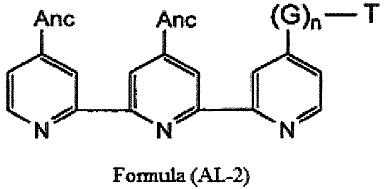 in the formula,
Anc represents an acidic group,
G represents a ring group represented by any one of the following Formulae (X-1) to (X-3),
n represents 2 or 3,
T represents a hydrogen atom or a substituent, and
a -(G)n-T group does not have the acidic group and an amino group; 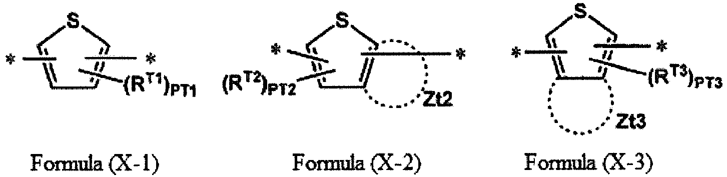 in the formulae,
Zt2 and Zt3 each represent a non-metal atomic group necessary for forming a fused ring with a thiophene ring in Formula (X-2) or (X-3),
R^{T1}, R^{T2}, and R^{T3} each independently represent a substituent,
PT1 represents an integer of 0 to 2,
PT2 and PT3 each independently represent an integer of 0 or more, and is no more than the number of hydrogen atoms when the group represented by Formula (X-2) or (X-3) is unsubstituted, and
* represents a binding position to the ring including L^{W}, or another G or T.

14. The terpyridine compound or an esterified product thereof according to claim 13, wherein the -(G)n- group in the Formula (AL-2) is a group of a combination of two ring groups represented by the following Formula (X-1a), 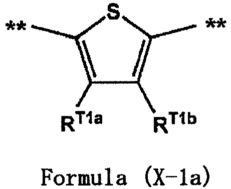 in the formula,
R^{T1a} and R^{T1b} each independently represent a hydrogen atom or a substituent, and
** represents a binding position to the pyridine ring, or another ring group or T.

15. The terpyridine compound or an esterified product thereof according to claim 13 or 14, wherein the -(G)n-T group in the Formula (AL-2) is a group represented by the following Formula (TF-1), 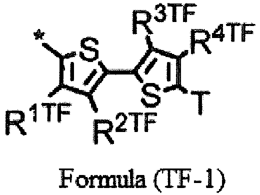 in the formula,
R^{1TF} to R^{4TF} each independently represent a hydrogen atom or an alkyl group,
T represents a hydrogen atom or a substituent, and
* represents a binding portion to the pyridine ring.

## Patentansprüche

1. Fotoelektrisches Umwandlungselement, enthaltend:
einen elektrisch leitenden Träger,
eine Fotoleiterschicht, enthaltend einen Elektrolyten,
eine Ladungstransferschicht, enthaltend einen Elektrolyten und
eine Gegenelektrode,
worin die Fotoleiterschicht feine Halbleiterteilchen aufweist, die einen Metallkomplexfarbstoff mit der folgenden Formel (I) tragen
Formel (I) M(LA)(LD)(LX)_{mX}·(CI)_{mY}
worin in der Formel
M ein Metallion,
LA ein Tridentat-Ligand mit der folgenden Formel (AL-1) ist,
LD ein Bidentat-Ligand oder ein Tridentat-Ligand ist, der von LA verschieden ist, und worin zumindest eines der koordinierenden Atome in LD, das an das Metallion M gebunden ist, ein Stickstoffatom ist und zumindest eines der koordinierenden Atome ein Anion ist,
LX ein Monodentat-Ligand ist und wenn LD ein Bidentat-Ligand ist, mX 1 ist und wenn LD ein Tridentat-Ligand ist, mX 0 ist,
CI ein Gegenion ist, das zum Neutralisieren der Ladung des Metallkomplexfarbstoffes notwendig ist, und
mY eine ganze Zahl von 0 bis 3 ist; 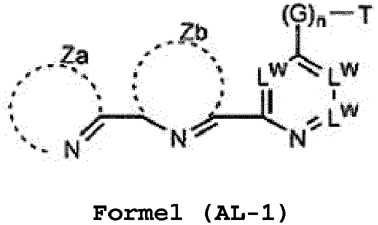 worin in der Formel
Za und Zb jeweils unabhängig eine Nicht-Metallatomgruppe sind, die zur Bildung eines 5- oder 6-gliedrigen Rings notwendig ist, worin zumindest einer der Ringe, gebildet durch Za und Zb, eine oder mehrere saure Gruppen hat, die Gruppen L^{W} jeweils unabhängig ein Stickstoffatom oder CR^{W} sind und R^{W} ein Wasserstoffatom oder ein Substituent ist,
G eine Ringgruppe ist, dargestellt durch eine der folgenden Formeln (X-1) bis (X-3),
n eine ganze Zahl von 2 bis 7 ist,
T ein Wasserstoffatom oder ein Substituent ist und
eine -(G)n-T-Gruppe keine saure Gruppe und eine Aminogruppe hat, 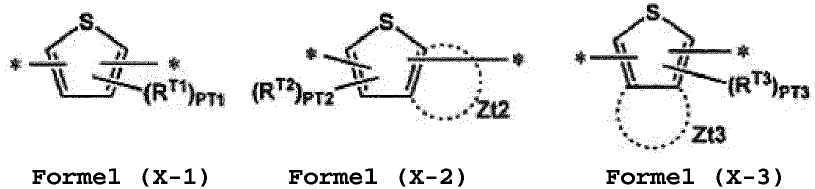 worin in den Formeln
Zt2 und Zt3 eine Nicht-Metallatomgruppe sind, die notwendig ist zur Bildung eines verschmolzenen Rings mit einem Thiophenring in der Formel (X-2) oder (X-3),
R^{T1}, R^{T2} und R^{T3} jeweils unabhängig ein Substituent sind,
PT1 eine ganze Zahl von 0 bis 2 ist,
PT2 und PT3 jeweils unabhängig eine ganze Zahl von 0 oder mehr sind und nicht mehr als die Zahl der Wasserstoffatome sind, wenn die Gruppe, dargestellt durch die Formel (X-2) oder (X-3), unsubstituiert ist, und
* eine Bindeposition an dem Ring ist, einschließlich L^{W} oder anderes G oder T.

2. Fotoelektrisches Umwandlungselement gemäß Anspruch 1, worin die Ringgruppe, dargestellt durch die Formel (X-1), eine Ringgruppe mit einer der folgenden Formeln (X-1a) bis (X-1c) ist 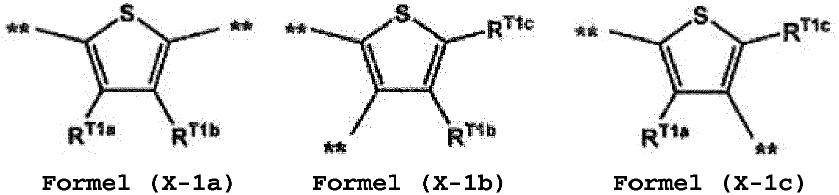 worin in den Formeln R^{T1a} bis R^{T1c} jeweils unabhängig ein Wasserstoffatom oder ein Substituent sind und ** eine Bindeposition an dem Ring, einschließlich L^{W}, oder ein anderes G oder T ist.

3. Fotoelektrisches Umwandlungselement gemäß Anspruch 1, worin die Ringgruppe, dargestellt durch die Formel (X-2), eine Ringgruppe ist, dargestellt eine der folgenden Formeln (X-2a) bis (X-2e) 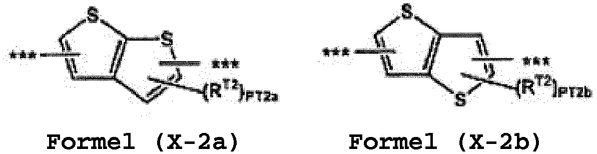 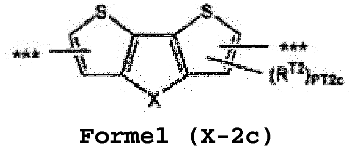 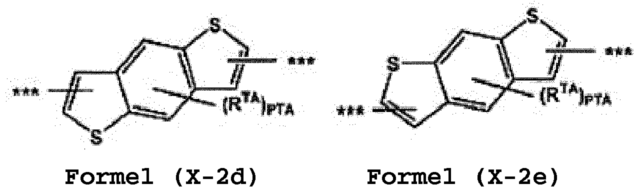 worin in den Formeln
X -O-, -S-, -NR^{X2c}-, -C(R^{X2c})₂-, -(R^{X2c})C=C(R^{X2c})- oder -Si(R^{X2c})₂- ist,
R^{X2c} ein Wasserstoffatom oder ein Substituent ist,
R^{T2} und R^{TA} jeweils unabhängig ein Substituent sind,
PT2a bis PT2c jeweils unabhängig eine ganze Zahl von 0 bis 2 sind,
PTA jeweils eine ganze Zahl von 0 bis 4 sind und
*** eine Bindeposition an dem Ring, einschließlich L^{W} oder ein anderes G oder T ist.

4. Fotoelektrisches Umwandlungselement gemäß Anspruch 1, worin die Ringgruppe, dargestellt durch die Formel (X-3), eine Ringgruppe ist, dargestellt die folgende Formel (X-3a) oder (X-3b) 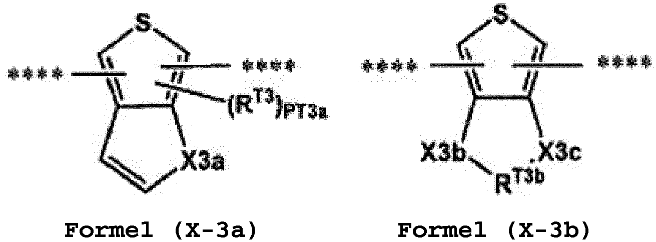 worin in der Formel
X3a bis X3c jeweils unabhängig -O- oder -S- sind,
R^{T3b} eine Alkylengruppe ist,
R^{T3} ein Substituent ist,
PT3a eine ganze Zahl von 0 bis 2 ist und
**** eine Bindeposition an dem Ring, einschließlich L^{W} oder ein anderes G oder T ist.

5. Fotoelektrisches Umwandlungselement gemäß einem der Ansprüche 1 bis 4, worin LA ein Tridentat-Ligand mit der folgenden Formel (AL-2) ist 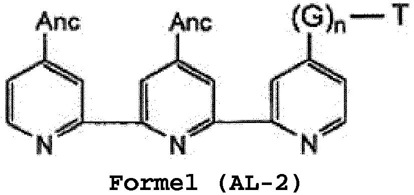 worin in der Formel
Anc eine saure Gruppe ist und
G, T und n die gleichen Bedeutungen wie G, T bzw. n in der Formel (AL-1) haben.

6. Fotoelektrisches Umwandlungselement gemäß einem der Ansprüche 1 bis 5, worin LD ein Bidentat-Ligand mit einer der folgenden Formeln (2L-1) bis (2L-4) ist 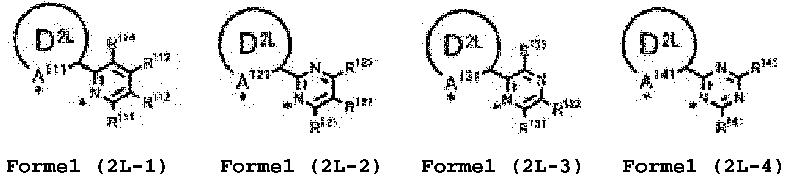 worin in den Formeln
der Ring D^{2L} ein aromatischer Ring ist,
A¹¹¹ bis A¹⁴¹ jeweils unabhängig ein Anion eines Stickstoffatoms oder ein Anion eines Kohlenstoffatoms sind,
R¹¹¹ bis R¹⁴³ jeweils unabhängig ein Wasserstoffatom oder ein Substituent ohne saurer Gruppe sind, und
* eine koordinierende Position an das Metallion M ist.

7. Fotoelektrisches Umwandlungselement gemäß einem der Ansprüche 1 bis 5, worin LD ein Tridentat-Ligand ist, dargestellt durch eine der folgenden Formeln (3L-1) bis (3L-4) ist 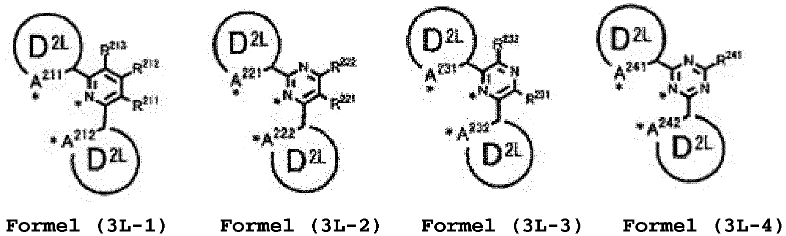 worin in den Formeln
der Ring D^{2L} ein aromatischer Ring ist,
A²¹¹ bis A²⁴² jeweils unabhängig ein Stickstoffatom oder Kohlenstoffatom sind, worin zumindest eines von jeden von A²¹¹ und A²¹², A²²¹ und A²²², A²³¹ und A²³², A²⁴¹ und A²⁴² ein Anion ist,
R²¹¹ bis R²⁴¹ jeweils unabhängig ein Wasserstoffatom oder ein Substituent sind, der keine saure Gruppe hat, und
* eine Koordinationsposition an das Metallion M ist.

8. Fotoelektrisches Umwandlungselement gemäß einem der Ansprüche 1 bis 7, worin Metallkomplexfarbstoff mit der Formel (I) durch die folgende Formel (I-1) oder (I-2) dargestellt ist, 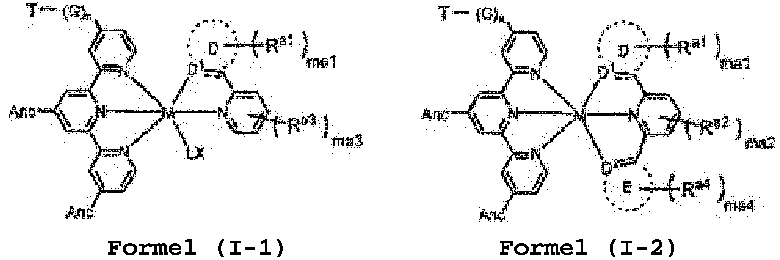 worin in den Formeln
M und LX die gleichen Bedeutungen wie M und LX in der Formel (I) haben,
G, T und n die gleichen Definitionen wie G, T und n in der Formel (AL-1) haben,
Anc eine saure Gruppe ist,
der Ring D und der Ring E jeweils unabhängig ein 5- oder 6-gliedriger, aromatischer Ring sind,
D¹ und D² jeweils unabhängig ein Anion eines Kohlenstoffatoms oder ein Anion eines Stickstoffatoms sind, worin eine Bindung zwischen D¹ und D² in dem Ring D und dem Ring E und ein Kohlenstoffatom, das an einen Pyridinring gebunden ist, eine Einfachbindung oder eine Doppelbindung ist,
R^{a1} bis R^{a4} jeweils unabhängig ein Substituent sind, und
ma1, ma3 und ma4 jeweils unabhängig eine ganze Zahl von 0 bis 3 sind, ma3 eine ganze Zahl von 0 bis 4 ist, und wenn ma1 bis ma4 jeweils eine ganze Zahl von 2 oder mehr sind, eine Vielzahl von R^{a1} bis R^{a4} jeweils aneinander zur Bildung eines Rings gebunden sein können.

9. Fotoelektrisches Umwandlungselement gemäß Anspruch 8, worin der Ring D und der Ring E jeweils unabhängig ein Pyrazolring, Triazolring oder Benzolring sind.

10. Farbstoff-sensibilisierte Solarzelle, enthaltend das fotoelektrische Umwandlungselement gemäß einem der Ansprüche 1 bis 9.

11. Metallkomplexfarbstoff, dargestellt durch die folgende Formel (I)
Formel (I) M(LA)(LD)(LX)_{mX}·(CI)_{mY}
worin in der Formel
M ein Metallion ist,
LA ein Tridentat-Ligand ist, dargestellt durch die folgende Formel (AL-1),
LD ein Bidentat-Ligand oder ein Tridentat-Ligand ist, der verschieden ist von LA und zumindest eines der koordinierenden Atome in LD, gebunden an das Metallion M, ein Stickstoffatom ist und zumindest eines der koordinierenden Atome ein Anion ist,
LX ein Monodentat-Ligand ist und wenn LD ein Bidentat-Ligand ist, bedeutet mX 1 und wenn LD ein Tridentat-Ligand, bedeutet mX 0,
CI ein Gegenion ist, das zum Neutralisieren der Ladung des Metallkomplexfarbstoffes notwendig ist, und
mY eine ganze Zahl von 0 bis 3 ist; 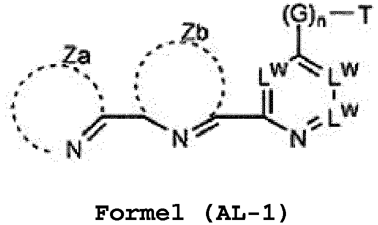 worin in der Formel
Za und Zb jeweils unabhängig eine Nicht-Metallatomgruppe sind, die zur Bildung eines 5- oder 6-gliedrigen Rings notwendig ist, worin zumindest einer der Ringe, gebildet durch Za und Zb, eine oder mehrere saure Gruppen hat,
L^{W} jeweils unabhängig ein Stickstoffatom oder CR^{W} sind und R^{W} ein Wasserstoffatom oder ein Substituent ist,
G eine Ringgruppe ist, dargestellt durch eine der folgenden Formeln (X-1) bis (X-3),
n eine ganze Zahl von 2 bis 7 ist,
T ein Wasserstoffatom oder ein Substituent ist und
eine -(G)n-T-Gruppe keine saure Gruppe und eine Aminogruppe hat, 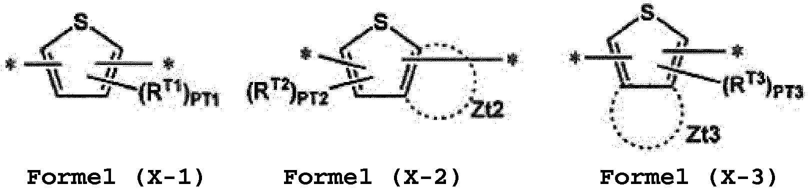 worin in den Formeln
Zt2 und Zt3 eine Nicht-Metallatomgruppe sind, die notwendig ist zur Bildung eines verschmolzenen Rings mit einem Thiophenring in der Formel (X-2) oder (X-3),
R^{T1}, R^{T2} und R^{T3} jeweils unabhängig ein Substituent sind,
PT1 eine ganze Zahl von 0 bis 2 ist,
PT2 und PT3 jeweils unabhängig eine ganze Zahl von 0 oder mehr sind und nicht mehr als die Zahl von Wasserstoffatomen sind, wenn die Gruppe, dargestellt durch die Formel (X-2) oder (X-3) unsubstituiert ist, und
* eine Bindeposition an dem Ring, einschließlich L^{W} oder anderes G oder T ist.

12. Farbstofflösung, enthaltend den Metallkomplexfarbstoff gemäß Anspruch 11 und ein Lösungsmittel.

13. Terpyridenverbindung oder ein verestertes Produkt davon, dargestellt durch die folgende Formel (AL-2) 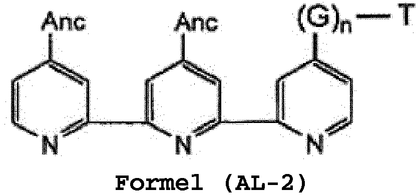 worin in der Formel
Anc eine saure Gruppe ist,
G eine Ringgruppe ist, dargestellt durch eine der folgenden Formeln (X-1) bis (X-3),
n eine ganze Zahl von 2 oder 3 ist,
T ein Wasserstoffatom oder ein Substituent ist und
eine -(G)n-T-Gruppe keine saure Gruppe und eine Aminogruppe hat, 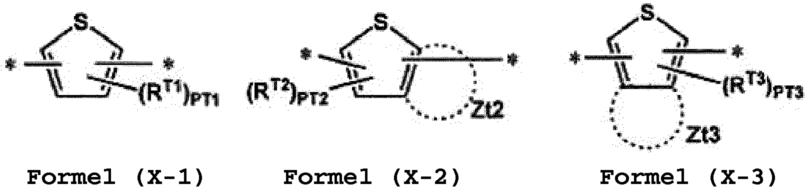 worin in den Formeln
Zt2 und Zt3 eine Nicht-Metallatomgruppe sind, die notwendig ist zur Bildung eines verschmolzenen Rings mit einem Thiophenring in der Formel (X-2) oder (X-3),
R^{T1}, R^{T2} und R^{T3} jeweils unabhängig ein Substituent sind,
PT1 eine ganze Zahl von 0 bis 2 ist,
PT2 und PT3 jeweils unabhängig eine ganze Zahl von 0 oder mehr sind und nicht mehr als die Zahl der Wasserstoffatome sind, wenn die Gruppe, dargestellt durch die Formel (X-2) oder (X-3) unsubstituiert ist, und
* eine Bindeposition an dem Ring ist, einschließlich L^{W} oder anderes G oder T.

14. Terpyridinverbindung oder ein verestertes Produkt davon gemäß Anspruch 13, worin die -(G)n-Gruppe in der Formel (AL-2) eine Gruppe einer Kombination von zwei Ringgruppen mit der folgenden Formel (X-1a) ist 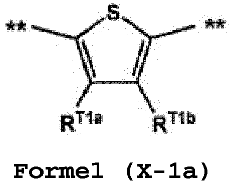 worin in der Formel
R^{T1a} und R^{T1b} jeweils unabhängig ein Wasserstoffatom oder ein Substituent sind und
** eine Bindeposition an dem Pyridinring ist oder eine andere Ringgruppe oder T ist.

15. Terpyridinverbindung oder ein verestertes Produkt davon gemäß Anspruch 13 oder 14, worin die -(G)n-T-Gruppe in der Formel (AL-2) eine Gruppe mit der folgenden Formel (TF-1) ist 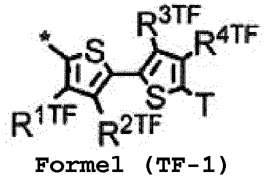 worin in der Formel
R^{1TF} bis R^{4TF} jeweils unabhängig ein Wasserstoffatom oder eine Alkylgruppe sind,
T ein Wasserstoffatom oder ein Substituent ist und
* eine Bindeposition an dem Pyridinring ist.

## Revendications

1. Élément de conversion photoélectrique comprenant :
un support électroconducteur ;
une couche photoconductrice incluant un électrolyte ;
une couche de transfert de charge incluant un électrolyte ; et
une contre-électrode,
dans lequel la couche photoconductrice a de fines particules semi-conductrices portant un colorant à complexe métallique représenté par la formule (I) suivante,
Formule (I) M(LA)(LD)(LX)_{mX}·(CI)_{mY}
dans la formule,
M représente un ion métallique,
LA représente un ligand tridentelé représenté par la formule (AL-1) suivante,
LD représente un ligand bidentelé, ou un ligand tridentelé différent de LA, et au moins l'un des atomes de coordination dans LD lié à l'ion métallique M est un atome d'azote, et au moins l'un des atomes de coordination est un anion,
LX représente un ligand monodentelé, et lorsque LD est un ligand bidentelé, mX représente 1, et lorsque LD est un ligand tridentelé, mX représente 0,
CI représente un contre-ion nécessaire pour neutraliser la charge du colorant à complexe métallique, et
mY représente un nombre entier de 0 à 3 ; 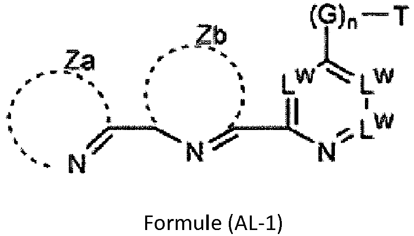 dans la formule,
Za et Zb représentent chacun indépendamment un groupe atomique non métallique nécessaire pour former un cycle à 5 ou 6 chaînons, dans lequel au moins l'un des cycles formés par Za et Zb, respectivement, a un ou plusieurs groupes acides, chaque L^{W} représente indépendamment un atome d'azote ou CR^{W}, et R^{W} représente un atome d'hydrogène ou un substituant,
G représente un groupe cyclique représenté par l'une quelconque des formules (X-1) à (X-3) suivantes,
n représente un nombre entier de 2 à 7,
T représente un atome d'hydrogène ou un substituant, et
un groupe -(G)n-T n'a pas le groupe acide et un groupe aminé ; 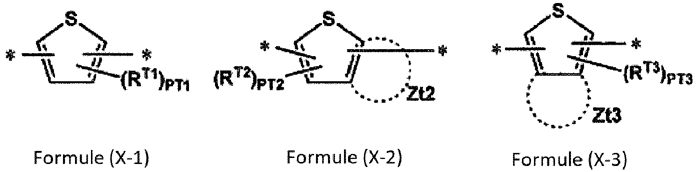 dans les formules,
Zt2 et Zt3 représentent chacun un groupe atomique non métallique nécessaire pour former un cycle fusionné avec un cycle thiophène dans la formule (X-2) ou (X3),
R^{T1}, R^{T2}, et R^{T3} représentent chacun indépendamment un substituant,
PT1 représente un nombre entier de 0 à 2,
PT2 et PT3 représentent chacun indépendamment un nombre entier de 0 ou plus, et n'est pas supérieur au nombre d'atomes d'hydrogène lorsque le groupe représenté par la formule (X-2) ou (X-3) n'est pas substitué, et
* représente une position de liaison au cycle incluant L^{W}, ou un autre G ou T.

2. Élément de conversion photoélectrique selon la revendication 1, dans lequel le groupe cyclique représenté par la formule (X-1) est un groupe cyclique représenté par l'une quelconque des formules (X-1a) à (X-1c) suivantes, 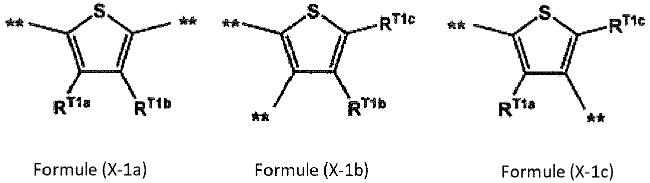 dans les formules, R^{T1a} à R^{T1c} représentent chacun indépendamment un atome d'hydrogène ou un substituant, et ** représente une position de liaison au cycle incluant L^{W}, ou un autre G ou T.

3. Élément de conversion photoélectrique selon la revendication 1, dans lequel le groupe cyclique représenté par la formule (X-2) est un groupe cyclique représenté par l'une quelconque des formules (X-2a) à (X-2e) suivantes, 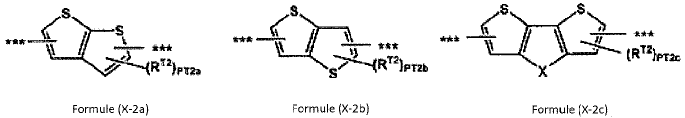 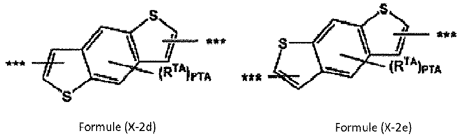 dans les formules,
X représente -O-, -S-, -NR^{X2c}-, -C(R^{X2c})₂-, -(R^{X2c})C=C(R^{X2c})-, ou -Si(R^{X2c})₂-,
R^{X2c} représente un atome d'hydrogène ou un substituant,
R^{T2} et R^{TA} représentent chacun indépendamment un substituant,
PT2a à PT2c représentent chacun indépendamment un nombre entier de 0 à 2, chaque PTA représente un nombre entier de 0 à 4, et
*** représente une position de liaison au cycle incluant L^{W}, ou un autre G ou T.

4. Élément de conversion photoélectrique selon la revendication 1, dans lequel le groupe cyclique représenté par la formule (X-3) est un groupe cyclique représenté par la formule (X-3a) ou (X-3b) suivante, 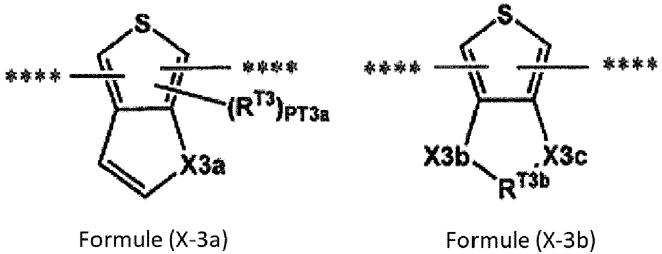 dans la formule,
X3a à X3c représentent chacun indépendamment -O- ou -S-,
R^{T3b} représente un groupe alkylène,
R^{T3} représente un substituant,
P^{T3a} représente un nombre entier de 0 à 2, et
**** représente une position de liaison au cycle incluant L^{W}, ou un autre G ou T.

5. Élément de conversion photoélectrique selon l'une quelconque des revendications 1 à 4, dans lequel LA représente un ligand tridentelé représenté par la formule (AL-2) suivante, 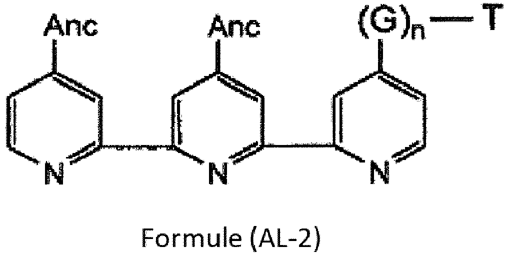 dans la formule,
Anc représente un groupe acide, et
G, T, et n ont les mêmes définitions que G, T, et n, respectivement, dans la formule (AL-1).

6. Élément de conversion photoélectrique selon l'une quelconque des revendications 1 à 5, dans lequel LD est un ligand bidentelé représenté par l'une quelconque des formules (2L-1) à (2L-4) suivantes, 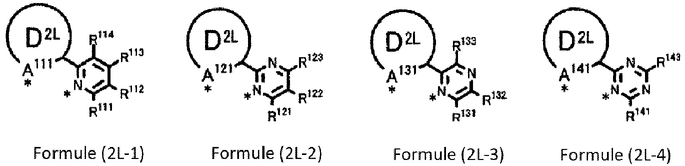 dans les formules,
le cycle D^{2L} représente un cycle aromatique,
A¹¹¹ à A¹⁴¹ représentent chacun indépendamment un anion d'un atome d'azote ou un anion d'un atome de carbone,
R¹¹¹ à R¹⁴³ représentent chacun indépendamment un atome d'hydrogène ou un substituant n'ayant pas de groupe acide, et
* représente une position de coordination à l'ion métallique M.

7. Élément de conversion photoélectrique selon l'une quelconque des revendications 1 à 5, dans lequel LD est un ligand tridentelé représenté par l'une quelconque des formules (3L-1) à (3L-4) suivantes, 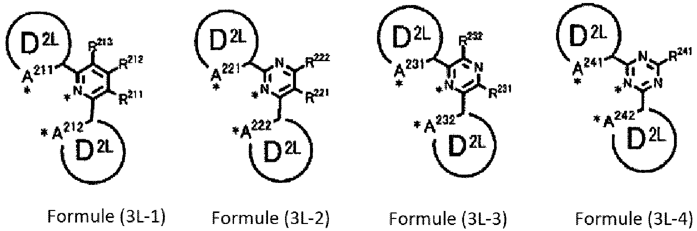 dans les formules,
le cycle D^{2L} représente un cycle aromatique,
A²¹¹ à A²⁴² représentent chacun indépendamment un atome d'azote ou un atome de carbone, dans lequel au moins l'un de chacun d'A²¹¹ et A²¹², A²²¹ et A²²², A²³¹ et A²³², et A²⁴¹ et A²⁴² est un anion,
R²¹¹ à R²⁴¹ représentent chacun indépendamment un atome d'hydrogène ou un substituant n'ayant pas de groupe acide, et
* représente une position de coordination à l'ion métallique M.

8. Élément de conversion photoélectrique selon l'une quelconque des revendications 1 à 7, dans lequel le colorant à complexe métallique représenté par la formule (I) est représenté par la formule (I-1) ou (I-2) suivante, 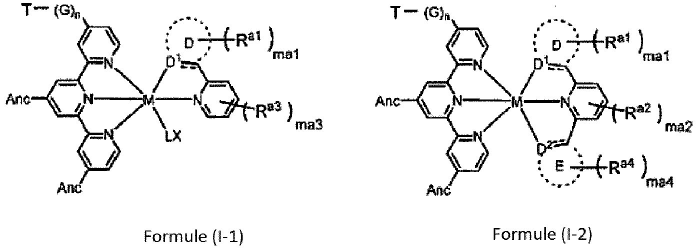 dans les formules,
M et LX ont les mêmes définitions que M et LX, respectivement, dans la formule (I),
G, T, et n ont les mêmes définitions que G, T, et n, respectivement, dans la formule (AL-1),
Anc représente un groupe acide,
le cycle D et le cycle E représentent chacun indépendamment un cycle aromatique à 5 ou 6 chaînons,
D¹ et D² représentent chacun indépendamment un anion d'un atome de carbone ou un anion d'un atome de d'azote, dans lequel une liaison entre D¹ et D² dans le cycle D et dans le cycle E, et un atome de carbone lié à un cycle pyridine est une liaison simple ou une liaison double,
R^{a1} à R^{a4} représentent chacun indépendamment un substituant, et
ma1, ma2, et ma4 représentent chacun indépendamment un nombre entier de 0 à 3, ma3 représente un nombre entier de 0 à 4, et lorsque ma1 à ma4 représentent chacun un nombre entier de 2 ou plus, une pluralité de R^{a1} à R^{a4} peuvent être chacun liés les uns aux autres pour former un cycle.

9. Élément de conversion photoélectrique selon la revendication 8, dans lequel le cycle D et le cycle E sont chacun indépendamment un cycle pyrazole, un cycle triazole, ou un cycle benzène.

10. Cellule solaire à pigment photosensible comprenant l'élément de conversion photoélectrique selon l'une quelconque des revendications 1 à 9.

11. Colorant à complexe métallique représenté par la formule (I) suivante,
Formule (I) M(LA)(LD)(LX)_{mX}·(CI)_{mY}
dans la formule,
M représente un ion métallique,
LA représente un ligand tridentelé représenté par la formule (AL-1) suivante,
LD représente un ligand bidentelé, ou un ligand tridentelé différent de LA, et au moins l'un des atomes de coordination dans LD lié à l'ion métallique M est un atome d'azote, et au moins l'un des atomes de coordination est un anion,
LX représente un ligand monodentelé, et lorsque LD est un ligand bidentelé, mX représente 1, et lorsque LD est un ligand tridentelé, mX représente 0,
CI représente un contre-ion nécessaire pour neutraliser la charge du colorant à complexe métallique, et
mY représente un nombre entier de 0 à 3 ; 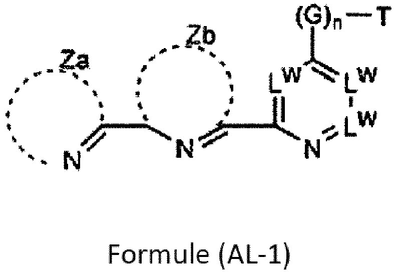 dans la formule,
Za et Zb représentent chacun indépendamment un groupe atomique non métallique nécessaire pour former un cycle à 5 ou 6 chaînons, dans lequel au moins l'un des cycles formés par Za et Zb, respectivement, a un ou plusieurs groupes acides,
chaque L^{W} représente indépendamment un atome d'azote ou CR^{W}, et R^{W} représente un atome d'hydrogène ou un substituant,
G représente un groupe cyclique représenté par l'une quelconque des formules (X-1) à (X-3) suivantes,
n représente un nombre entier de 2 à 7,
T représente un atome d'hydrogène ou un substituant, et
un groupe -(G)n-T n'a pas le groupe acide et un groupe aminé ; 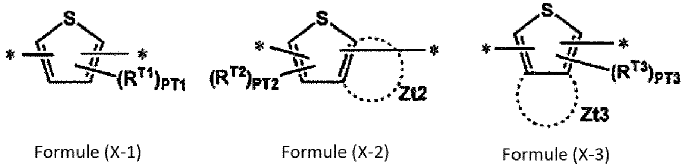 dans les formules,
Zt2 et Zt3 représentent chacun un groupe atomique non métallique nécessaire pour former un cycle fusionné avec un cycle thiophène dans la formule (X-2) ou (X3),
R^{T1}, R^{T2}, et R^{T3} représentent chacun indépendamment un substituant,
PT1 représente un nombre entier de 0 à 2,
PT2 et PT3 représentent chacun indépendamment un nombre entier de 0 ou plus, et n'est pas supérieur au nombre d'atomes d'hydrogène lorsque le groupe représenté par la formule (X-2) ou (X-3) n'est pas substitué, et
* représente une position de liaison au cycle incluant LW, ou un autre G ou T.

12. Solution de colorant comprenant le colorant à complexe métallique selon la revendication 11 et un solvant.

13. Composé terpyridine ou un produit estérifié de celui-ci représenté par la formule (AL-2) suivante, 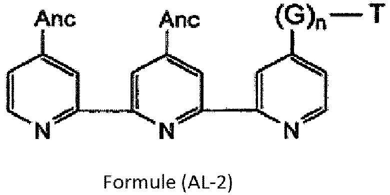 dans la formule,
Anc représente un groupe acide,
G représente un groupe cyclique représenté par l'une quelconque des formules (X-1) à (X-3) suivantes,
n représente 2 ou 3,
T représente un atome d'hydrogène ou un substituant, et
un groupe -(G)n-T n'a pas le groupe acide et un groupe aminé ; 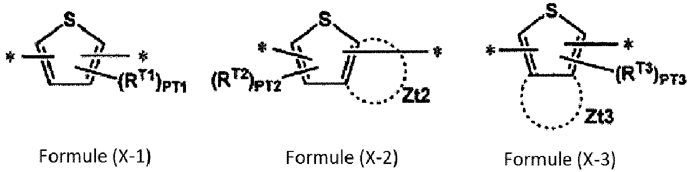 dans les formules,
Zt2 et Zt3 représentent chacun un groupe atomique non métallique nécessaire pour former un cycle fusionné avec un cycle thiophène dans la formule (X-2) ou (X3),
R^{T1}, R^{T2}, et R^{T3} représentent chacun indépendamment un substituant,
PT1 représente un nombre entier de 0 à 2,
PT2 et PT3 représentent chacun indépendamment un nombre entier de 0 ou plus, et n'est pas supérieur au nombre d'atomes d'hydrogène lorsque le groupe représenté par la formule (X-2) ou (X-3) n'est pas substitué, et
* représente une position de liaison au cycle incluant LW, ou un autre G ou T.

14. Composé terpyridine ou un produit estérifié de celui-ci selon la revendication 13, dans lequel le groupe -(G)n dans la formule (AL-2) est un groupe d'une combinaison de deux groupes cycliques représenté par la formule (X-1a) suivante, 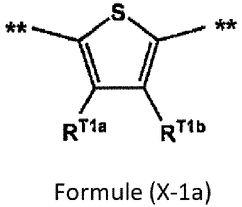 dans la formule,
R^{T1a} à R^{T1b} représentent chacun indépendamment un atome d'hydrogène ou un substituant, et
** représente une position de liaison au cycle pyridine, ou un autre groupe cyclique ou T.

15. Composé terpyridine ou un produit estérifié de celui-ci selon la revendication 13 ou 14, dans lequel le groupe -(G)n-T dans la formule (AL-2) est un groupe représenté par la formule (TF-1) suivante, 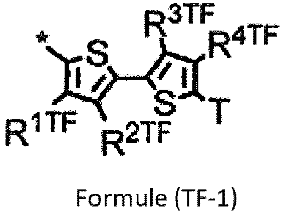 dans la formule,
R^{1TF} à R^{4TF} représentent chacun indépendamment un atome d'hydrogène ou un groupe alkyle,
T représente un atome d'hydrogène ou un substituant, et * représente une partie de liaison au cycle pyridine.
